# EUROPÄISCHE PATENTSCHRIFT

(11) **EP 1 844 333 B1**
(45) Veröffentlichungstag und Bekanntmachung des Hinweises auf die Patenterteilung: **08.12.2010**
(21) Anmeldenummer: 06705836.2
(22) Anmeldetag: 23.01.2006
(51) Int. Cl.: G01N 33/569, G01N 33/92

(54) **VERFAHREN ZUM NACHWEIS UND ZUR ENTFERNUNG VON ENDOTOXIN**
METHOD FOR DETECTING AND REMOVING ENDOTOXIN
PROCEDES POUR DETECTER ET ELIMINER DES ENDOTOXINES

(30) Priorität: 21.01.2005 DE 102005002969
(43) Veröffentlichungstag der Anmeldung: 17.10.2007
(73) Patentinhaber: Hyglos Invest GmbH, 82347 Bernried (DE)
(72) Erfinder: MILLER, Stefan, 93053 Regensburg (DE); MEYER, Roman, 92287 Schmidmühlen (DE); GRASSL, Renate, 93049 Regensburg (DE); BIEBL, Manfred, 93047 Regensburg (DE); GRALLERT, Holger, 69198 Schriesheim (DE)
(74) Vertreter: Dehmel, Albrecht
(86) Internationale Anmeldenummer: PCT/DE2006/000098
(87) Internationale Veröffentlichungsnummer: WO 2006/076905

(56) Entgegenhaltungen:
- WO-A-2004/001418
- US-A- 5 506 121
- US-A1- 2004 248 298
- DATABASE EMBL Juli 2003 (2003-07), "Gp12 short tail fiber" XP002387509 Database accession no. AAQ17871
- MERINO S ET AL: "IDENTIFICATION OF THE CELL SURFACE RECEPTOR FOR BACTERIOPHAGE 18 FROM AEROMONAS-HYDROPHILA" RESEARCH IN MICROBIOLOGY, Bd. 141, Nr. 2, 1990, Seiten 173-180, XP002387493 ISSN: 0923-2508

## Beschreibung

Die vorliegende Erfindung betrifft Bakteriophagenschwanzproteine, die sowohl in Anwesenheit als auch in Abwesenheit von zweiwertigen positiven Ionen, insbesondere Ca²⁺ oder Mg²⁺, Endotoxine binden können. Die vorliegende Erfindung betrifft ferner Verfahren zur Abreicherung von Endotoxinen aus Lösungen und Proben mittels der erfindungsgemäßen Bakteriophagenschwanzproteine sowie ein Nachweisverfahren für Endotoxine.

Bakteriophagen erkennen Strukturen (Membranproteine, Zuckermoleküle etc.) auf der Oberfläche ihrer Wirtsbakterien über entsprechende Proteine, die die Bakteriophagen auf ihrer Oberfläche tragen. Manche Bakteriophagen besitzen nur einen Typ von Erkennungsprotein wie z.B. der Salmonella-Phage P22, andere mindestens deren zwei oder mehr. Die Erkennungsproteine können enzymatische Aktivität besitzen, wie beim Phagen P22 (Seckler, J. Struct. Biol. 1998; 122(1-2):216-222), oder auch nicht enzymatisch aktiv sein. Enzymatische Aktivität bedeutet, dass diese Proteine wie z.B. das P22 Tailspike Protein, das Rezeptormolekül, das sie erkennen, d.h. bei P22 das Salmonella O-Antigen, hydrolysieren können. Der bekannteste Bakteriophage, der zwei Erkennungsproteine besitzt, ist der für E.coli spezifische Phage T4. Dieser Phage besitzt lange und kurze Schwanzfasem. Die langen Schwanzfasern dienen der spezifischen Erkennung seines Wirtsbakteriums E.coli über das Membranprotein OmpC oder über Lipopolysaccharid bei E.coli B. Während die langen Schwanzfasern der Phagen T4, T2 und K3 spezifisch an OmpC bzw. Lipopolysaccharid von E.coli B (T4), OmpF (T2; Hantke K. , Mol Gen Genet. 1978; 164 (2):131-135) und OmpA (K3; Hancock RE, Reeves P , J Bacteriol. 1975 ;121(3):983-993; Riede I, Eschbach ML, Henning U., Mol Gen Genet. 1984;195(1-2):144-152) binden, liegen die kurzen Schwanzfasern an der Unterseite der Basisplatte des Phagen und sind nicht an der Wirtsspezifität beteiligt, sondern austauschbar zwischen T4, T2 und K3 Phagen (Riede, Mol Gen Genet. 1987;206(1):110-115). Erst nachdem mindestens drei lange Schwanzfasern gebunden haben, werden die kurzen Schwanzfasern aus der Basisplatte ausgeklappt und sind für die irreversible Bindung des T4-Phagen an die E.coli-Wirtsbakterien verantwortlich (Leiman et al., Cell Mol Life Sci. 2003;60(11):2356-2370). Diese kurzen Schwanzfaserproteine binden, wie für die des Phagen T4 gezeigt (WO 2004/001418), in der Herzregion des Lipopolysaccharids und eignen sich daher zur Erkennung und Bindung von Endotoxin.

Endotoxin (ET) bezeichnet eine Familie von Lipopolysacchariden, die zusammen mit Proteinen und Phospholipiden die äußere Zellmembran Gram-negativer Bakterien bilden. Endotoxine kommen ausschließlich in dieser Bakteriengruppe vor und spielen eine wichtige Rolle in der Organisation, Stabilität und Barrierefunktion der äußeren Membran. Zahlreiche Bakteriophagen nutzen Endotoxin bzw. allgemein Lipopolysaccharid zur spezifischen Erkennung ihrer Wirtsbakterien.

Endotoxine sind Biomoleküle, die ohne entsprechende Vorsichtsmaßnahmen in praktisch allen wässrigen Lösungen vorzufinden sind. Endotoxine wirken bei Mensch und Tier stark pyrogen, rufen also Fieberreaktionen hervor und können zu Sepsis, einer .starken Fehlreaktion des Immunsystems führen, die mit einer hohen Mortalitätsrate einhergeht. Daher sind z.B. bei der Herstellung von Proteinen für die medizinische oder pharmazeutische Verwendung Verunreinigungen mit Endotoxin exakt nachzuweisen und in der Folge komplett zu entfernen. Endotoxin stellt ein Problem bei gentechnisch hergestellten Arzneimitteln, Gentherapeutika oder Substanzen dar, die in Mensch oder Tier (z.B. Tiermedizinische Behandlung oder bei Tierversuchen) injiziert werden. Doch nicht nur bei medizinischen, sondern auch bei Forschungsanwendungen, wie bei Transfektionsexperimenten von Säugerzellen kann eine Hemmung bzw. ein Senken der Transfektionseffizienz durch Endotoxin beobachtet werden.

Alle Endotoxinvarianten bestehen aus einem Heteropolysaccharid, das kovalent an Lipid A; gebunden ist (Holst, O., 1999, In: Endotoxin in health and disease (Brade, H.et al; eds.), Marcel Dekker Inc. New York)). Lipid A verankert Endotoxin in der äußeren Bakterienmembran. Das Heteropolysaccharid, das aus einem Herzoligosaccharid und dem O-Antigen besteht, zeigt in die umgebende Lösung und bestimmt die serologische Identität des Bakteriums. Das O-Antigen besteht aus repetitiven Oligosaccharideinheiten, deren Zusammensetzung stammspezifisch ist (siehe hierzu Holst et al., supra). Charakteristische Bausteine des Herzoligosaccharids sind 2-Keto-3-desoxyoctonsäure (KDO) und L-Glycero-D-manno-heptose (Hep).

Der konservativste Teil von Endotoxin verschiedener Gattungen ist das Lipid A. Ähnlich konserviert wie Lipid A ist die innere Herzregion, während die äußere Herzregion bereits eine höhere Variation aufweist. Die innere Herzregion, KDO und Lipid A selbst tragen mehrere Phosphatgruppen als Substituenten und sind so für die negative Ladung von Endotoxin verantwortlich. Darüber hinaus können die Phosphatgruppen am Lipid A und der Herzregion variabel mit Arabinose, Ethanolamin und Phosphat substituiert sein. Einzelne Saccharidbausteine des O-Antigens sind acetyliert, sialysiert oder glycosyliert. Das O-Antigen variiert außerdem bezüglich der Anzahl repetitiver Einheiten, weshalb die Endotoxin-Population jedes Bakteriums eine gewisse Heterogenität aufweist (Palva E.T. und Makela P.H., Eur J Biochem. 1980;107(1):137-43; Goldman R.C. und Leive L., Eur J Biochem. 1980;107(1):145-53).

Um Proteine im Rahmen von klinischen Studien einsetzen zu können, verlangen die europäische und die amerikanische Pharmacopoeia, dass die Proteine bestimmte Grenzwerte an Endotoxinbelastung unterschreiten (z.B. Immunserum Globulin ≤0,91 EU/ml, dies entspricht ≤ 5 EU/kg Körpergewicht & Stunde (Dosis = EU/kg * h); EU = Endotoxin Unit; FDA (Food and Drug Administration): Guideline on Validation of LAL as End Product). Falls ein Medikament bzw. darin enthaltene Proteine eine zu hohe Endotoxinbelastung aufweisen, kann dies zum Tod des Patienten führen. Die fehlgeleitete Immunabwehr schädigt durch eine Überreaktion den Patienten. Dies kann zu Gewebeentzündungen, Blutdruckabfall, Herarasen, Thrombose bis hin zu septischem Schock mit multiplem Organversagen führen. Bereits eine länger anhaltende Endotoxin-Exposition in Picogramm-Mengen kann zu chronischen Nebenwirkungen wie z.B. Immunschwächen, septischen Symptomen etc. führten. Im Rahmen der Substanzherstellung wird daher, insbesondere bei Prozessen unter "Good Manufacturing Practice" (GMP) Bedingungen, versucht, Endotoxin soweit wie möglich abzweichern bzw. zu entfernen. Allerdings ist die Endotoxin-Entfernung bei Proteinen, Polysacchariden und DNA problematisch. Gerade bei Proteinen gibt es große Probleme durch deren intrinsische Eigenschaften wie Ladungszustand oder Hydrophobizität, die eine Endotoxinentfemung nahezu verhindern bzw. zu großen Produktverlusten bei der Entfernungsprozedur führen können.

Außerdem wird der Endotoxin Nachweis sowie die Entfernung durch die Umgebung beeinflusst, da Faktoren wie z.B. die Ionenzusammensetzung, der pH, die Temperatur oder die Anwesenheit von anderen Substanzen, die Wechselwirkung eines Liganden mit Endotoxin entscheidend verändern können. Dabei gilt es zu beachten, dass die Interaktion von Liganden mit unterschiedlichen Strukturteilen der Endotoxine, wie dem hydrophoben Lipid A oder dem hydrophilen Polysaccharidteil, erfolgen kann. Diese Wechselwirkungen beruhen dementsprechend in der Regel auf ionischen oder hydrophoben Kräften, die durch die Zusammensetzung der Lösung unterschiedlich beeinflusst werden.

Die Polysaccharidstruktur der Endotoxine wird durch positiv geladene zweiwertige Ionen, wie Calcium oder Magnesium, stabilisiert (Galanos C. und Lüderitz O., Eur. J. Biochem. 1975; 54: 603-610). Diese Ionen können auch die Wechselwirkung mit Liganden vermitteln ("bridgingeffect").

Zur Endotoxin-Abreicherung bzw. Entfernung aus biologischen Lösungen allgemein gibt es eine Reihe von Verfahren. Insbesondere bei Proteinen gibt es allerdings bislang keine allgemein anwendbaren Standardverfahren. Die jeweils verwendeten Verfahren sind angepasst an die spezifischen Eigenschaften des jeweiligen Proteins und auf den entsprechenden Produktionsprozess des Proteins. Es gibt verschiedene Möglichkeiten zur Endotoxin-Abreicherung, wobei jedes dieser Verfahren spezifische Vor- und Nachteile aufweist.

Die Ultrafiltration (Petsch, D. & Anspach, F.B., 2000, J. Biotechnol. 76, 97-119 und Referenzen darin) wird für Endotoxin-Abreicherungen aus Wasser und Lösungen mit niedermolekularen Bestandteilen wie Salze, Zucker und Antibiotika verwendet, ist jedoch nicht für hochmolekulare Proteine oder DNA geeignet.

Die 2-Phasen-Extraktion (z.B. WO 01/66718, Merck) soll wasserlösliche Proteine und DNA von Endotoxin trennen, bedingt jedoch Detergenzreste im gereinigten Produkt. Das Verfahren ist außerdem durch mehrmaliges Wiederholen der Reinigungsprozedur zeitaufwändig.

Ebenfalls wird für die Endotoxinabreicherung aus DNA und sauren Proteinen ein Anionenaustauscher (DEAE)-Verfahren verwendet (z.B. US 5,990,301, Qiagen; WO 94/14837, Enzon; EP 0592989 A2, Braun Melsungen), das jedoch eine niedrige Ionenstärke (<50 mM NaCl) voraussetzt und zu einer Protein Co-Adsorption bei sauren Proteinen führt. Für basische Proteine werden Kationenaustauscher eingesetzt, die z.T. mit Detergenzien kombiniert werden (z. B. US 2002/0147315 A1).

Kationische Peptide zur Endotoxin-Entfernung werden in EP 1232754 B1 (Commonwealth Biotechnologies) verwendet.

Es werden auch hydrophile Matrices eingesetzt wie eine Kombination aus Dextran und N',N'-Methylen-bis-acrylamid (US 5,917,022).

Hydrophobe Chromatographieverfahren werden in WO 94/14837 (Enzon) eingesetzt.

Ein weiteres Verfahren zur Endotoxinabreicherung aus DNA und Proteinen (z.B. BSA, Myoglobin, gamma-Globulin, Cytochrom C) ist die Affinitäts-Adsorption (z. B. Polymyxin B, Histamine, Histidin, Poly-L-lysin, Polyethylenimin) z.B. GB 2,192,633 (Hammersmith Hospital), US 2002/0130082 A1 (Tokodoro), US 5,510,242 oder WO 95/025117 (GMBF), die jedoch im Fall von Polymyxin B toxisch ist und bei niedrigen Ionenstärken zur Co-Adsorption von Proteinen führen kann.

Folgende Verfahren beschreiben eine Entfernung von Endotoxin mit Hilfe der Metallaffinitätschromatographie (US 6,365,147; US 6,942,802; WO 02/083710, American Cyanide).

LPS-Bindeprotein oder Peptide oder Derivate davon werden ebenfalls zur spezifischen Bindung von Endotoxin eingesetzt (US 6,376,462, Xoma Corp.; US 6,384,188, Dana Faber Cancer Institute; WO 95/005393, Morphosys; WO 95/008560, Centocor; WO 95/025117, Scripps).

Weiterhin wird die Immun-Affinitäts-Chromatographie eingesetzt, wobei die Spezifität für bestimmte Endotoxine nur über teure Antikörper (US 5,179,018, Centocor; WO 00/08463, Bioserv; EP 0074240 A2, Gaffin) gegen Hera-Oligosaccharid erreicht werden kann.

Ferner wird das S3delta-Peptid (WO 01/27289) des Faktors C (eines Bestandteils des LAL-Tests) (WO 99/15676 beide: National University of Singapur) bei Proteinen (z.B. BSA, Chymotrypsinogen) verwendet, wobei jedoch dieses Verfahren eine geringe Effizienz bei hohen Ionenstärken besitzt und die hohen Herstellungskosten (Produktion in Insekten-Zellkultur) hinzukommen.

Außerdem wird das Endotoxin Neutralizing Protein (ENP) aus *Limulus polyphemus,* das ebenfalls spezifisch an Endotoxin bindet, zur Endotoxin-Abreicherung verwendet (z. B. US 5,747,455; US 5,627,266) oder das LPS Bindeprotein des Pfeilschwanzkrebses (US 5,760,177). Die Gewinnung dieses Proteins aus dem Pfeilschwanzkrebs oder rekombinant aus *Saccharomyces* ist ebenfalls zeitaufwändig und kostenintensiv.

Ein weiteres Verfahren zur Entfernung von Endotoxinen aus einer Probe wird in der WO 2004/001418 beschrieben. Die Endotoxine werden dabei durch auf einem Träger immobilisierte Bakteriophagenschwanzproteine gebunden und so von der Probe getrennt. Für eine effiziente Trennung sind jedoch zweiwertige Ionen nötig, wodurch das Verfahren mit industriell relevanten Puffern, wie z.B. Phosphat- oder Citrat-Puffern, oder auch in Gegenwart von Chelatoren wie EDTA, oder EGTA nicht verwendet werden kann.

In der Anwendung in der pharmazeutischen Industrie befinden sich für Proteinlösungen, angepasst an die Eigenschaften der Zielproteine, im Wesentlichen drei Verfahren:
- Anionenaustauscherchromatographie
- Reversed-Phase Chromatographie; Diese hat den Nachteil, dass sie nicht für alle Proteine gleichermaßen geeignet, insbesondere bei hydrophoben Proteinen problematisch ist. Darüber hinaus ist dieses Verfahren sehr zeitintensiv und Proteine werden normalerweise unter Bedingungen der Reversed-Phase Chromatographie denaturiert, so dass sie nachher aufwändig und oft mit hohem Materialverlust verbunden, renaturiert werden müsssen.
- RemTox (Fa. Millipore): Dieses Verfahren hat den Nachteil, dass neben einer sehr langen Inkubationsdauer, der unspezifische Bindungsanteil hoch ist, und die Proteinwiederfindung oftmals nicht ausreichend ist.

Eine grobe Endotoxin-Abreicherung von Proteinen auf einen Wert bis zu 10 EU/ml ist mit den bestehenden Verfahren in vielen Fällen möglich. Die verbleibende Konzentration an Endotoxin wirkt jedoch immer noch toxisch. Eine weitere Abreicherung (d.h. Feinreinigung) ist daher geboten bzw. abhängig von der Dosis des Proteins in der medizinischen Anwendung. Die Europäische Pharmacopoeia, die USP (United States Pharmacopeial Convention) und die FDA (Food and Drug Administration) schreiben die Grenzwerte für medizinische Anwendungen verbindlich vor (z.B. 5 EU/kg Körpergewicht und Stunde in intravenösen Anwendungen). Allerdings ist diese Feinreinigung mit vorhandenen Methoden oft nicht zufriedenstellend gewährleistet. Die marktgängigen Verfahren weisen hier erhebliche Nachteile auf und sind bei bestimmten Proteinen oft nicht, oder nur unter erheblichen Verlusten des Zielproteins, anwendbar.

Im Hinblick auf industrielle Anwendungen ist ferner zu beachten, dass aus Kostengründen, nur möglichst billige Puffersubstanzen, wie z.B. Phosphat, Citrat, Borat, Carbonat oder Acetat, zum Einsatz kommen. Die Wechselwirkung von Liganden mit Endotoxinen sollte deshalb möglichst durch diese Puffer nicht gestört werden. Für Bindungsreaktionen, die Calcium benötigen, sind insbesondere Puffer oder Zusätze problematisch, die Calcium komplexieren, wie EDTA, EGTA oder Citrat. Darüber hinaus sind Puffer problematisch, deren Salze mit Calcium unlösliche oder schwerlösliche Niederschläge bilden. Calciumphosphat zum Beispiel präzipitiert, weshalb in Phosphatpuffern nur geringe Konzentrationen von freiem Calcium vorhanden sind.

Neben der Endotoxin-Abreicherung bzw. Entfernung spielt der Endotoxin-Nachweis in Proben, Lösungen oder pharmazeutischen Präparationen eine wesentliche Rolle. Derzeit sind 6 Verfahren zum Endotoxin-Nachweis in biologischen Lösungen beschrieben, wobei nur die beiden ersten Verfahren von der FDA zugelassen sind. Der EAA ist von der TPD (Therapeutic Product Directorate of Canada) und unter bestimmten Bedingungen (hohes Risiko für Sepsis bei Intensivpatienten) auch von der FDA zugelassen. 1. "Rabbit Pyrogen Testing": Ein Verfahren, bei dem einem lebenden Kaninchen eine Endotoxin-Lösung injiziert und damit eine Immunreaktion ausgelöst wird. Diese Endotoxin-verursachte Immunantwort wird über die Entwicklung von Fieber nachgewiesen. 2. Deutlich besser standardisierbar ist der "Limulus Amoebocyte Lysate (LAL)" - Test, der derzeit am häufigsten verwendete Test (Cambrex-BioWhittacker, Inc., Charles River, Inc., Associates of Cape Cod, Inc., alle USA). Bei diesem Verfahren wird nach Endotoxinkontakt eine Enzymkaskade im Blut des Pfeilschwanzkrebses (Limulus polyphemus) ausgelöst. Das Vorhandensein von Endotoxin, kann dann mit vier verschiedenen Methoden gemessen werden (gel-clot, turbidimetric, colorimetric, and chromogenic assay). 3. Der In Vitro Pyrogen Test basiert auf dem Nachweis von Interleukin-1β in menschlichem Blut, das an der Fieberinduktion beteiligt ist. Der Test besteht aus einem Inkubationsschritt von menschlichem Blut mit der zu untersuchenden Lösung und der anschließenden Detektion des Interleukins über Antikörper. 4. Ein ähnliches Verfahren ist der Nachweis der Induktion von Prostaglandin (PGE2) in Kaninchenblut nach Endotoxinkontakt (Ochiai et al., Microbiol. Immunol., 2003, 47, 585-590). 5. Eine weitere Möglichkeit ist der Einsatz eines speziellen Zellkultursystems (Sterogene Inc., USA), mit dem die Aktivierung von Monozyten über die Entstehung bestimmter Zytokine verfolgt wird. 6. Der EAA (endotoxin activity assay) der Firma Spectral Diagnostics, Inc., Canada ist ebenfalls ein Bluttest. Endotoxin reagiert mit Antikörpern, wobei das Signal nach Komplementaktivierung in den patienteneigenen Neutrophilen mit Hilfe eines Zymosans verstärkt und als Chemilumineszenz detektiert wird. Die beiden erstgenannten Verfahren sind jedoch sehr teuer und durch den großen Bedarf an Versuchstieren bzw. an Blut des sehr seltenen Pfeilschwanzkrebses nicht zuletzt aus Tierschutzgründen bedenklich. Der LAL-Test kann zwar miniaturisiert und automatisiert werden, hat aber massive Nachteile in der Anwendung. Er ist arbeitsintensiv, erfordert speziell geschultes Personal, relativ lange Inkubationszeiten, relativ große Probenvolumina und teuere Reagezien. Eine einmal geöffnete LAL-Lösung muss direkt weiterverarbeitet und aufgebraucht werden, da die Komponenten aufgrund geringer Stabilität innerhalb weniger Stunden aggregieren. In der Testdurchführung müssen ebenfalls zweiwertige Ionen anwesend sein, der pH-Bereich ist relativ eingeschränkt (pH 6 - 7.5) und häufig anwesende Glucane stören den Test. Endotoxin ist oft maskiert, d.h. es wird z.B. nicht erkannt, wenn es an Proteine gebunden vorliegt. Der In Vitro Pyrogen Test benötigt möglichst frisches menschliches Blut und ist relativ zeitaufwendig, da die Produktion des Interleukins 10-24 Stunden benötigt. Der Hauptvorteil dieser Methode liegt darin, dass neben Endotoxinen auch andere Pyrogene erkannt werden. Dieser Test ist in erster ' Linie als Ersatz für den "Rabbit Pyrogen Test" gedacht. Für alle Testverfahren ist geschultes Personal nötig und die Verfahren sind sehr störanfällig, weil z.B. das Immunsystem von Kaninchen auf die gleiche Endotoxindosis durchaus unterschiedlich reagieren kann. Das Zellkultur-Verfahren der Firma Sterogene ist, wie alle Zellkulturverfahren, ebenfalls sehr aufwändig und weist Probleme bei der Standardisierung auf. Vergleicht man die unterschiedlichen Methoden zum Endotoxinnachweis, so stimmen die Ergebnisse oft nicht. überein, d.h. verschiedene Endotoxine werden von den unterschiedlichen Testkomponenten nicht in gleicher Weise erkannt. Insgesamt kann festgestellt werden, dass es kein einfach handhabbares kostengünstiges Verfahren zum Endotoxin-Nachweis gibt und die derzeit eingesetzten Methoden eine Reihe von Nachteilen aufweisen.

Daher liegt der Erfindung die Aufgabe zu Grunde, Mittel und Verfahren bereitzustellen, mit denen Endotoxine aus und in solchen Lösungen unabhängig vom Gehalt an zweiwertigen positiven Ionen entfernt und nachgewiesen werden können.

Die Aufgabe wird durch den in den Patentansprüchen definierten Gegenstand gelöst.

Die nachfolgenden Figuren erläutern die Erfindung.

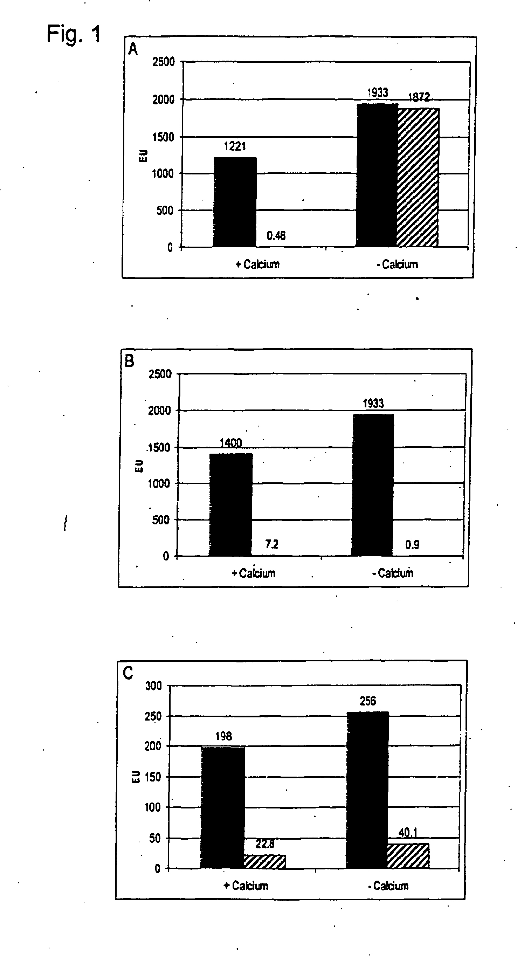

Figur 1 zeigt das Ergebnis der Endotoxin Entfernung mit Hilfe von T4p 12-Sepharose (A), N-Strep-Miro2p12-Sepharose (B) und N-Strep-Aehlp12-Sepharose (C) aus "Calcium-haltigen" (mit Calcium) und "Calcium-freien" (ohne Calcium) Puffern. Die schwarzen Balken zeigen jeweils die aufgetragene Endotoxinmenge und die gestrichelten Balken die Endotoxinmenge nach dem Lauf der Lösung über die entsprechende Sepharose. Die Endotoxinmengen wurden mit dem LAL-Test (kinetisch-chromogener LAL-Test, Cambrex) gemessen und sind in Endotoxin Einheiten (EU) angegeben. Die Bakteriophagenschwanzproteine wurden zunächst auf NHS-aktivierte Sepharose (Amersham Biosciences) immobilisiert und anschließend wurde mit diesem gekoppelten Sepharosematerial Chromatographiesäulen gegossen. Es wurden Säulen mit Volumina von 1ml (T4p12, N-Strep-Miro2p12) und 200 µl (N-Strep-Aehlp12) gegossen. Als Säulenkörper wurden 1 ml Polypropylen Säulenkörper (Qiagen) und Handee Mini-Spin Säulen (200 µl, Pierce) verwendet. Die Säulen wurden zunächst mit dem Laufpuffer equilibriert (6-faches Säulenvolumen), die Probe aufgetragen und anschließend mit 3-fachem Säulenvolumen gespült. Die Endotoxinmengen im Auftrag (schwarze Balken) und im Durchlauf (gestrichelte Balken) sind jeweils nebeneinander dargestellt. Die Versuche wurden mit "Calcium-haltigen" und "Calcium-freien" Puffer durchgeführt. Der "Calcium-haltige" Puffer bestand aus 20 mM Hepes, 150 mM NaCl, 0.1 mM CaCl₂, pH 7.5 und der "Calcium-freie" Puffer aus 20 mM Hepes, 150 mM NaCl, 2 mM EDTA, pH 7.5. Mit T4p12-Sepharose konnte nur in dem "Calcium-haltigen" Puffer Endotoxin aus der Lösung entfernt werden, während mit N-Strep-Miro2p12-und N-Strep-Aehlp12-Sepharose Calcium keinen Einfluss auf die Endotoxin Entfernung hatte.

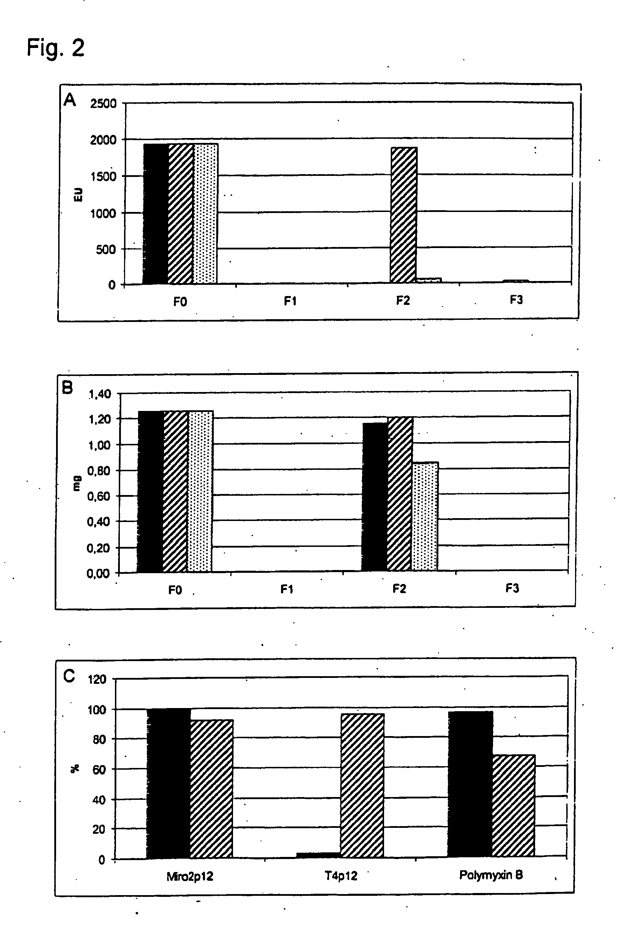

Figur 2 A-C zeigt das Ergebnis der Endotoxin Entfernung aus einer BSA Lösung über Chromatographiesäulen mit immobilisiertem Miro2p12 im Vergleich mit Polymyxin- und T4p12-Säulen. Der Laufpuffer (20 mM Hepes, 150 mM NaCl, 2 mM EDTA, pH 7.5) enthielt 2 mM EDTA und damit nur eine sehr geringe Konzentration an freiem Calcium (< 1µM; Donald Bers, Chris Patton, Richard Nuccitelli, Methods in Cell Biology, Vol. 40; A practical guide to the study of Ca in living cells; chapter1 A practical guide to the preparation of Ca Buffers, Academic Press, 1/94.). Die Chromatographiesäulen enthielten jeweils 2 ml Säulenmaterial. Vor dem Auftrag der Probe (Fraktion F0) wurde 1 ml Durchlauf des Laufpuffers gesammelt (F1). Anschließend wurde jeweils 1 ml einer BSA-Lösung (1.2 mg/ml), die mit etwa 2000 EU/ml gespickt war, auf jede Säulen aufgetragen und zwei weitere Fraktionen mit 4 ml, und 3 ml aufgefangen (F2 und F3). In Fig. 2(A) sind die Endotoxin Mengen und in Fig. 2(B) die BSA Mengen im Auftrag und den Fraktionen F1-F3 der Polymyxin B-Säule (gepunktete Balken), der T4p12 Säule (gestrichelte Balken) und der Miro2p12 Säule (gefüllte Balken) dargestellt. In Fig.2(C) ist die prozentuale Endotoxin Entfernung (gefüllte Balken) und Proteinwiederfindung (gestrichelte Balken), berechnet für die zweite Fraktion, gezeigt: Mit T4p12 konnte unter diesen Pufferbedingungen kein Endotoxin entfernt werden, während mit der Polymyxin B Säule 96% und mit der Miro2p12 Säule 99.6% des Endotoxins entfernt werden konnten. Die Proteinwiederfindung betrug für Polymyxin B 67% und für Miro2p12 92%. Die prozentuale Endotoxin Entfernung zeigt, wieviel des in der Probe zu Beginn des Experiments vorliegenden Endotoxins durch die Behandlung mit Miro2p12 oder anderen Proteinen entfernt wurde. Die Proteinwiederfindung gibt Auskunft darüber, wie viel des eingesetzten Proteins nach der Endotoxin Entfernung noch in der Probe vorliegen, bzw. durch die Endotoxin Entfernung unspezifisch mit entfernt wurde.

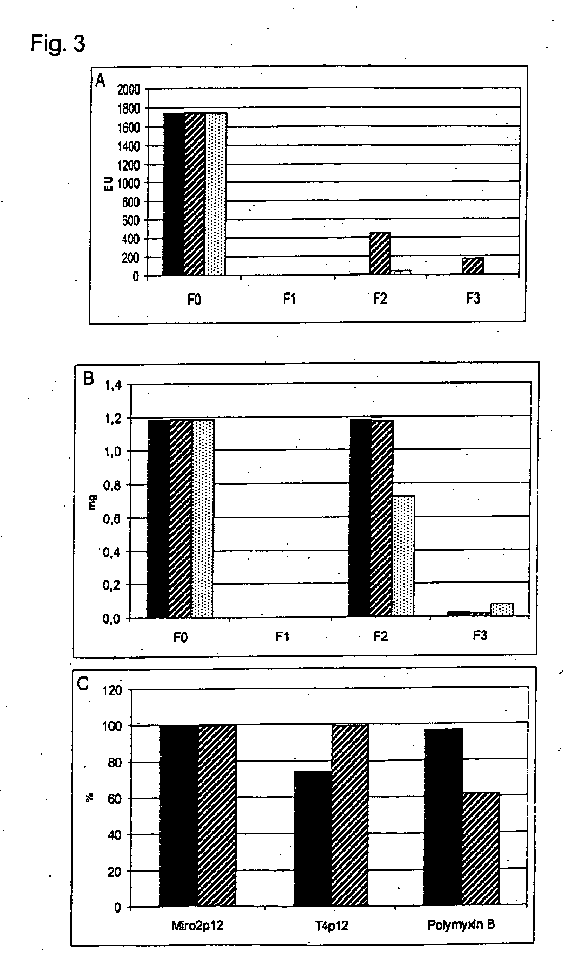

Figur 3 A-C zeigt das Ergebnis der Endotoxin Entfernung aus einer BSA Lösung über Chromatographiesäulen mit immobilisiertem Miro2p12 im Vergleich mit Polymyxin B- und T4p12-Säulen. Als Laufpuffer wurde in diesem Experiment der physiologisch wichtige PBS-Puffer (10 mM Na₂HPO₄, 1.8 mM KH₂PO₄, 137 mM NaCl, 2.7 mM KCl, pH 7.4) verwendet. Die Chromatographiesäulen enthielten jeweils 2 ml Säulenmaterial. Vor dem Auftrag der Probe (Fraktion F0) wurde 1 ml Durchlauf des Laufpuffers gesammelt (F1). Anschließend wurde jeweils 1 ml einer BSA-Lösung (1.2 mg/ml), die mit etwa 1800 EU/ml gespickt war, auf jede Säulen aufgetragen und zwei weitere Fraktionen mit 4 ml und 3 ml aufgefangen (F2 und F3). In Fig. 3(**A**) sind die Endotoxin Mengen und in Fig. 3(**B**) die BSA Mengen im Auftrag und den Fraktionen F1-F3 der Polymyxin B-Säule (gepunktete Balken), der T4p12 Säule (gestrichelte Balken) und der Miro2p12 Säule (gefüllte Balken) dargestellt In Fig. 3(**C**) ist die prozentuale Endotoxin Entfernung (gefüllte Balken) und Proteinwiedertindung (gestrichelte Balken), berechnet für die zweite Fraktion, gezeigt. Mit Polymyxin B konnten 97%, mit T4p12 74% und mit Miro2p12 99.7% Endotoxin entfernt werden. Die Proteinwiederfindung betrug für Polymyxin B 61%, für T4p12 99.4% und für Miro2p12 99.8%.

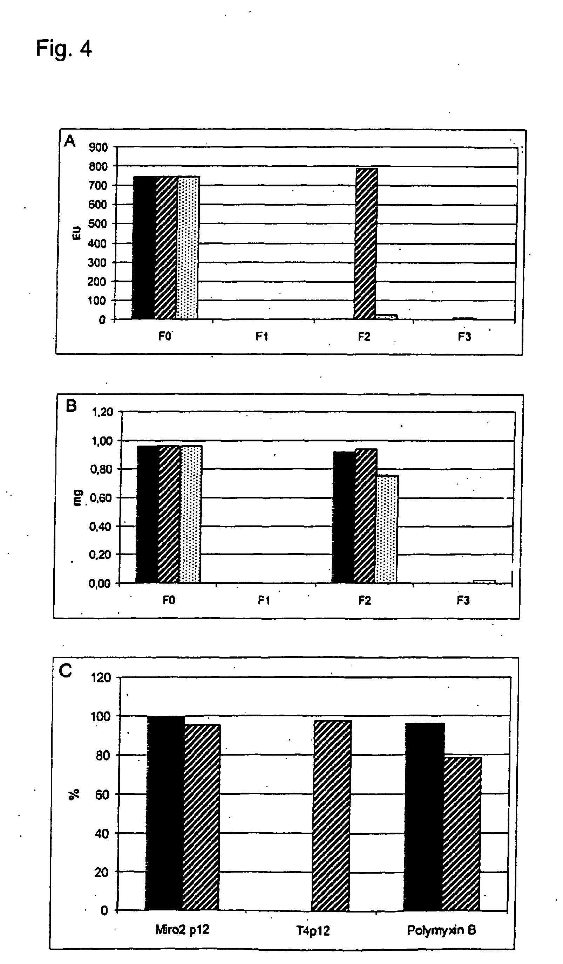

Figur 4 A-C zeigt das Ergebnis der Endotoxin Entfernung aus einer BSA Lösung über Chromatographiesäulen mit immobilisiertem Mim2p12 im Vergleich mit Polymyxin B- und T4p12-Säulen. Es wurde ein Citratpuffer (20 mM Citrat, 150 mM NaCl, pH 7.0) verwendet, der Calcium Ionen binden kann. Die Chromatographiesäulen enthielten jeweils 1.5 ml Säulenmaterial. Vor dem Auftrag der Probe (Fraktion F0) wurde 1 ml Durchlauf des Laufpuffers gesammelt (F1). Anschließend wurde jeweils 1 ml einer BSA-Lösung (0.96 mg/ml), die mit etwa 750 EU/ml gespickt war, auf jede Säulen aufgetragen und zwei weitere Fraktionen mit 4 ml und 3 ml aufgefangen (F2 und F3). In Fig. 4(**A**) sind die Endotoxin Mengen und in Fig. 4(**B**) die BSA Mengen im Auftrag und den Fraktionen F1-F3 der Polymyxin B-Säule (gepunktete Balken), der T4p12 Säule (gestrichelte Balken) und der Miro2p12 Säule(gefüllte Balken) dargestellt. In Fig. 4(**C**) ist die prozentuale Endotoxin Entfernung (gefüllte Balken) und Proteinwiederfindung (gestrichelte Balken), berechnet für die zweite Fraktion, gezeigt. Mit Polymyxin B konnten 96.2%, mit T4p12 0% und mit Miro2p12 99.5% Endotoxin entfernt werden. Die Proteinwiederfindung betrug für Polymyxin B 78.3%, für T4p12 97.8% und für Miro2p12 95.4%.

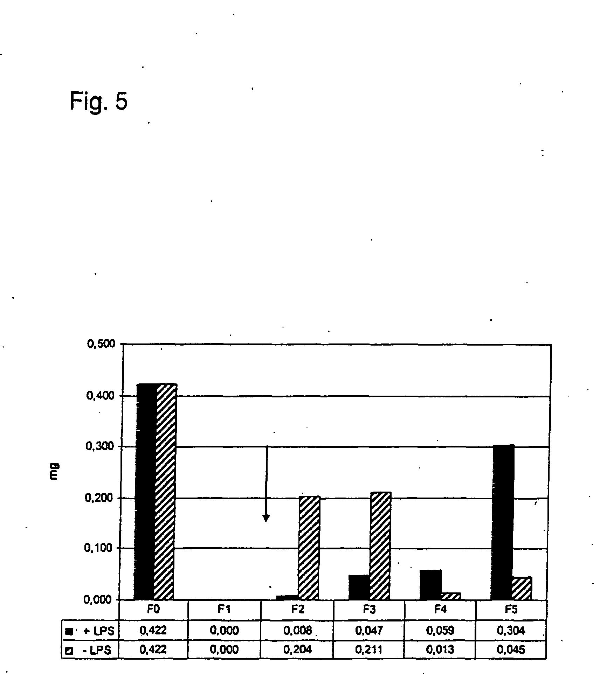

Figur 5 zeigt das Ergebnis des Nachweises von Endotoxin über die Bindung von T4p12 an immobilisiertes Endotoxin (Miro2p12-Endotoxin-T4p12 Sandwich). An das Säulenmaterial (0.5 ml) war das Bakteriophagenschwanzprotein Miro2p12 kovalent immobilisiert (3.4 mg Protein/ml Säulenmaterial). Eine Probe, enthaltend Endotoxin von *E*. *coli* 055:B5 (10⁶ EU/ml) wurde über die Säule gegeben und wurde von Miro2p12 gebunden (+LPS, schwarze Balken). Eine Kontrollsäule wurde mit einer Probe ohne Endotoxin äquilibriert (- LPS, gestreifte Balken). Die Menge des Bakteriophagenschwanzproteins p 12 wurde gegen die Fraktionen des Chromatographielaufs aufgetragen. Jeder Balken zeigt die Mittelwerte, die aus zwei parallelen Chromatographieläufen ermittelt wurden. Das erste Balkenpaar (F0) zeigt die aufgetragene T4p12 Menge und das zweite die Fraktion 1 (F1), eine Kontrollfraktion vor dem Auftrag von T4p12 auf die Säule. Der Pfeil markiert den Auftrag von p12 auf die Säule. Die Fraktionen F2 - F5 wurden nach dem Auftrag gesammelt. Die Menge an p12 (angegeben in mg) wurde durch Absorptionsmessung bei 280 nm ermittelt. Das Fraktionsvolumen war für die Fraktionen F1-F4 1ml und für die Fraktion F5 2 ml. Die Ablösung des gebundenen T4p12 Proteins in Fraktion F5 erfolgte durch Zugabe von 2 mM EDTA zum Laufpuffer (20 mM Herpes, 150 mM NaCl, 0.1 mM CaCl₂, pH 7.5). Das Bakteriophagenschwanzprotein T4p12 wurde auf den Säulen, die vorher mit Endotoxin beladen worden waren, zurückgehalten, während es durch die Säulen, die kein Endotoxin enthielten, ohne Verzögerung eluierte.

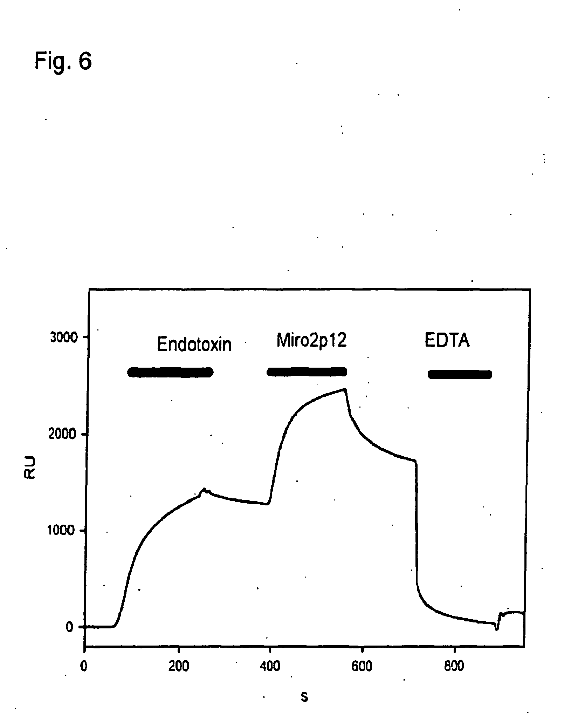

Figur 6 zeigt das Ergebnis der Bindung von Miro2p12 über Endotoxin vermittelt an T4p12. Es wurde ein CM-5 Chip der Firma Biacore mit kovalent immobilisiertem T4p12 benutzt (EDC/NHS-Kopplung). Zuerst wurde Endotoxin (von *E*. *coli* 055:B5; 1 mg/ml) injiziert, das wie die Zunahme des Resonanzsignals zeigt, an T4p12 bindet. Anschließend wurde Miro2p12 (0.02 mg(ml) injiziert, das ebenfalls bindet, wie die Zunahme des Resonanzsignals zeigt. Durch Zugabe von EDTA wurde zum Abschluss des Experiments die Endotoxin-T4p12 Bindung wieder gelöst. Die Injektionsphasen sind durch Balken gekennzeichnet. Als Laufpuffer wurde 20 mM Hepes, 150 mM NaCl, 0.1 mM CaCl₂ verwendet. Die zweite, unbeladene Zelle des Chips diente als Referenzzelle. Die Kurve zeigt die Differenz von Mess- und Referenzzelle. RU bedeutet resonance units; s bedeutet Sekunden.

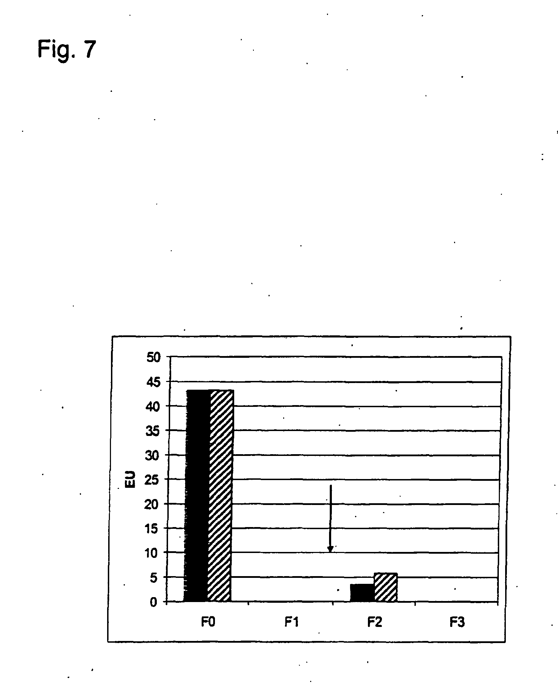

Figur 7 zeigt das Ergebnis der Endotoxin Entfernung aus menschlichem Serum mit Hilfe von N-Strep-Miro2p12 immobilisiert auf Sepharose. Auf zwei N-Strep-Miro2p12-Sepharose Säulen (Säulenvolumen: 1 ml, Säule1 = schwarze Balken, Säule2 = gestreifte Balken) wurde jeweils 1 ml menschliches Serum gespickt mit Endotoxin aus E. coli 055:B5 aufgetragen (F0) und nach der Säule Fraktionen von 4 bzw. 3 ml (F2, F3) gesammelt. Der Pfeil markiert den Auftrag der Probe. Vor dem Auftrag wurde die Säule mit 1 ml Laufpuffer gewaschen (F1), um sicherzustellen, dass die Säule nicht mit Endotoxin verunreinigt ist. Der Laufpuffer war wie folgt zusammengesetzt: 20 mM Hepes, 150 mM NaCl, 2 mM EDTA, pH 7.5. Es konnten 90 % des aufgetragenen Endotoxins aus dem Serum entfernt, d. h. auf der Säule zurückgehalten werden.

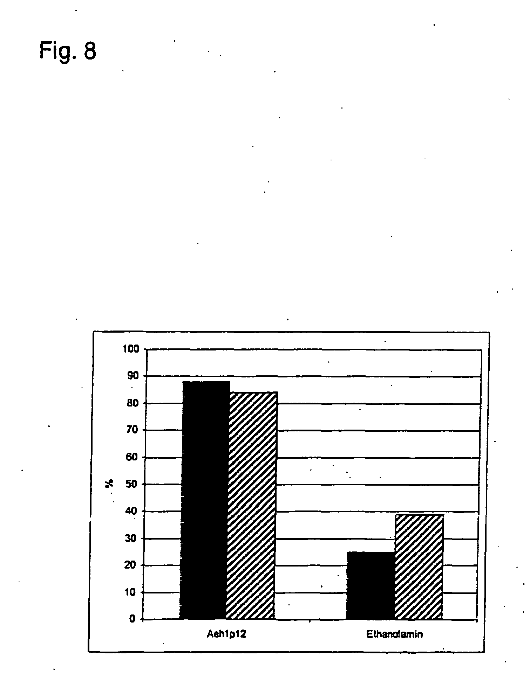

Figur 8 zeigt das Ergebnis der Endotoxin Entfernung mit Hilfe von Aehlp12, das auf Sepharose immobilisiert worden war (2.5 mg Aeh1p12 pro 1 ml Sepharose), im Vergleich mit Sepharose, die an Stelle von Aeh1p12 mit Ethanolamin beladen worden war, um die reaktiven Gruppen der aktivierten Sepharose abzusättigen. Der Versuch wurde mit und ohne Calcium im Laufpuffer durchgeführt, um den Einfluss von Calcium auf die Endotoxin Entfernung zu untersuchen. Die Laufpuffer waren wie folgt zusammengesetzt: Schwarze Balken (+ Ca): 20 mM Hepes, 150 mM NaCl, 0.1 mM CaCl₂, pH 7.5. Gestreifte Balken (- Ca): 20 mM Hepes, 150 mM NaCl, 2 mM
EDTA, pH 7.5. Es wurden jeweils 0.2 ml Säulen gegossen und diese vor dem Versuch mit 2 ml 0.5% Natriumdeoxycholat, 15 ml Wasser und 2 ml Laufpuffer gewaschen. Anschließend wurden 0.2 ml Lipopolysaccharid von E. coli 055:B5 (etwa 1000 EU/ml), das im jeweiligen Laufpuffer gelöst war, aufgetragen. Diese Probe wurde 30 min bei Raumtemperatur inkubiert, ablaufen lassen und mit 0.6 ml und 1 ml Laufpuffer gewaschen. Diese Fraktionen wurden mittels LAL-Test auf ihren Endotoxingehalt untersucht und anhand der Endotoxinmengen vor und nach der Säule die prozentuale Endotoxin Entfernung errechnet. Mit Aeh1p12 (88% und 84%) konnte deutlich mehr Endotoxin aus der Lösung entfernt werden als mit der Kontrollsäule (25% und 39 %), die kein Protein enthielt. Freies Calcium ist für die Bindung von Endotoxin an Aeh1p12 nicht notwendig, da die Endotoxin Entfernung mit Calcium (88 %) und ohne Calcium (84%) im Puffer ähnlich war.

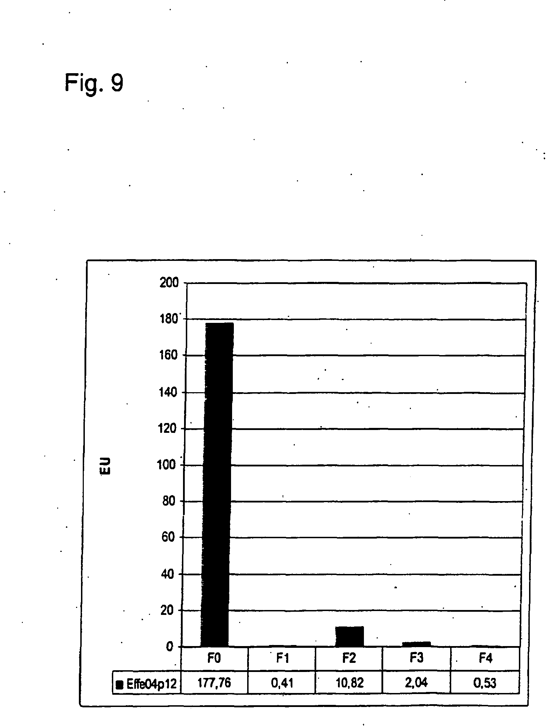

Figur 9 zeigt das Ergebnis der Endotoxin Entfernung mit Hilfe von Effe04p12. Das Protein wurde dazu kovalent an Sepharose gekoppelt und anschließend diese Sepharose mit einer Endotoxinlösung inkubiert, die durch Zentrifugation wieder von der Sepharose abgetrennt wurde. Die Endotoxin Entfernung wurde in einem Calcium-freien Puffer durchgeführt (20 mM Hepes, 150 mM NaCl, 2 mM EDTA, pH 7.5). Zunächst wurden 100 µl Effe04p12-Sepharose mit 1 ml endotoxinfreiem Puffer gewaschen (F1) und anschließend 100 µl Endotoxinlösung (Lipopolysaccharid von *E*. *coli* O55:B5) auf die Effe04p12-Sepharose aufgebracht. Die Sepharose wurde 30 min mit der Endotoxinlösung inkubiert und dabei mehrmals durch schütteln gemischt. Anschließend wurde die Lösung über eine Mini-Spin-Säule von der Sepharose getrennt und mit 200 µl Puffer gewaschen (F2). Zum Abschluß wurde noch zweimal mit jeweils 200 µl gewaschen (F3 und F4). Der Endotoxingehalt im Auftrag (F0) und den Fraktionen wurde mittels eines LAL-Tests bestimmt und daraus eine prozentuale Endotoxin Entfernung von 92.5% berechnet.

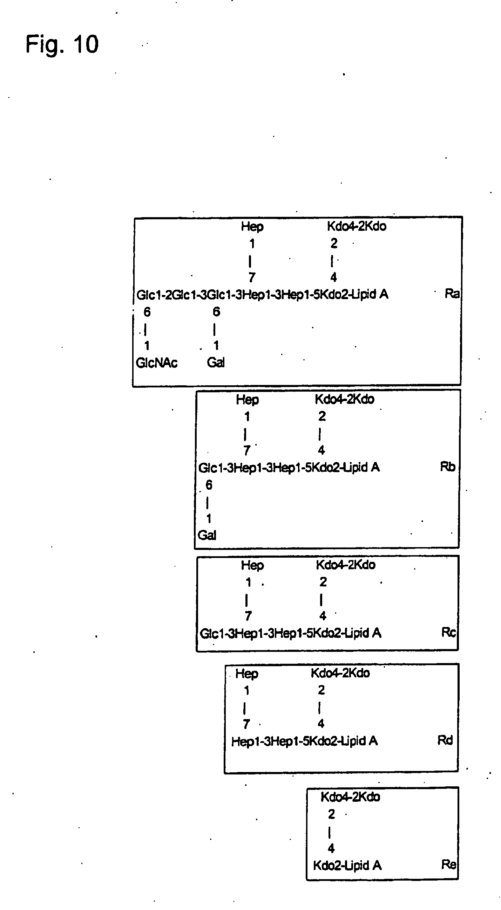

Figur 10 zeigt die Core-Zucker Stukturen der E. coli Mutanten, die für die Bindungsuntersuchungen mit N-Strep-Miro2p12 verwendet wurden. Hep = Heptose, Kdo = 2-Keto-3-deoxy-octonat, Glc = Glucose, Gal = Galactose, GlcNAc = N-Acetylglucosamin.

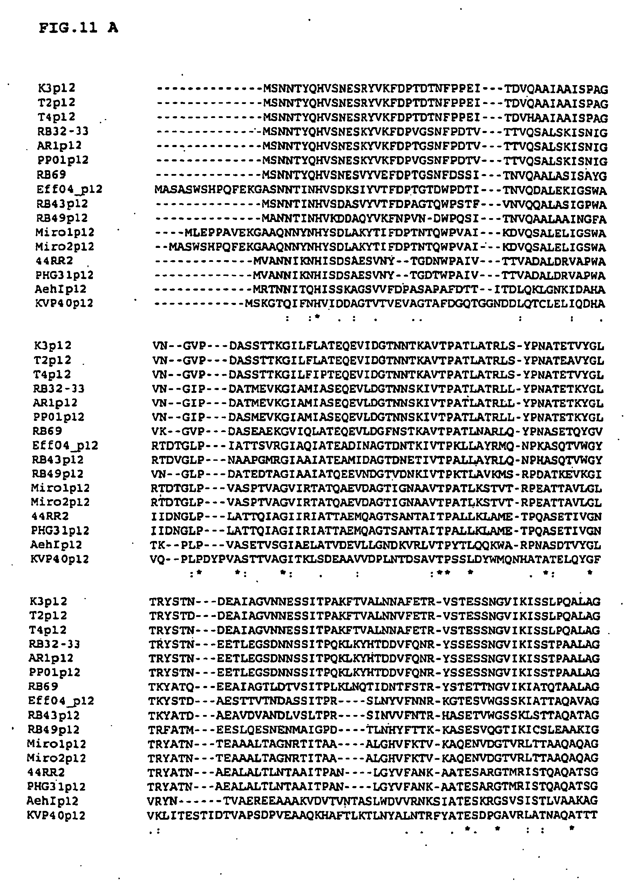

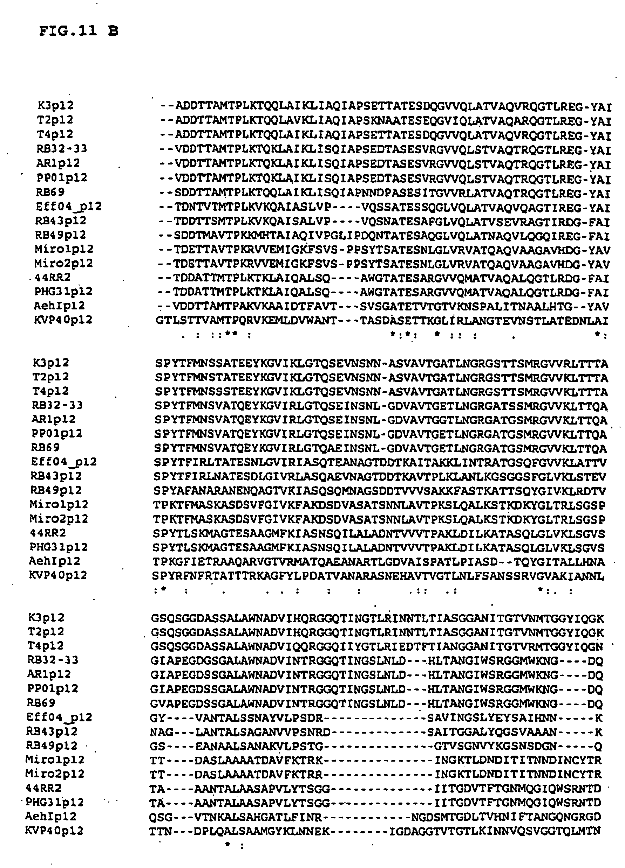

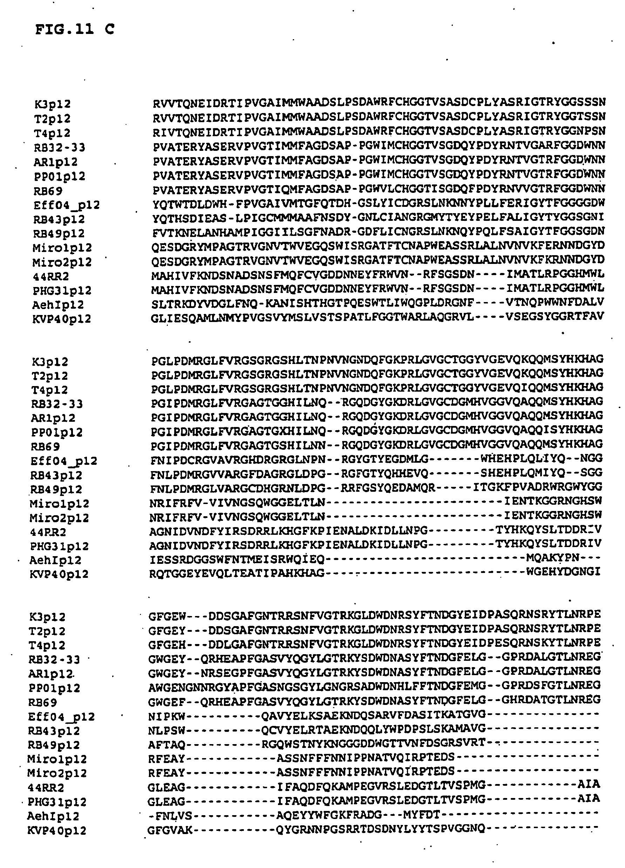

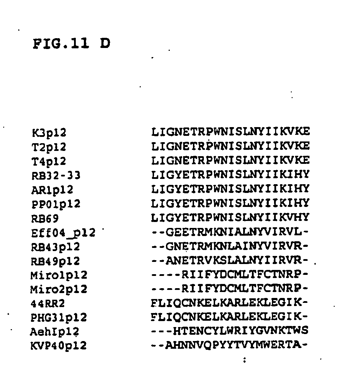

Figur 11 zeigt einen Sequenzvergleich auf Aminosäureebene zwischen verschiedenen Bakteriophagenschwanzproteinen. Der Sequenzvergleich wurde mit Clustal V (1.81) unter folgenden Einstellungen durchgeführt, wobei die Sequenzen von T4p12, T2p12 und K3p12 zuerst mit dem "Multiple Alignment Mode" aligned wurden: Alignment Parameter:
Pairwise Parameters:
Pairwise Alingmentsd: Slow-Accurate
Gap Opening [0-100]: 10
Gap Extension [0-100]: 0.1
Protein Weight Matrix: Gonnet 250

Multiple Parameters
Gap Opening [0-100]: 10
Gap Extension [0-100] 0.2
Delay Divergent Sequences (%): 30
Protein Weight Matrix: Gonnet Series

Protein Gap Parameters
Residue-specific Penalties: ON
Hydrophilic Penalties: ON
Hydrophilic Residues: GPSNDQEKR
Gap Separation Distance [0-100]: 4
End Gap Separation: OFF
Quality - Column Score Parameters:
Score Plot Scale: 5
Residue Exception Cutoff: 5
Protein Weight Matrix: Gonnet PAM 250

Die Phagensequenzen für das Alignment lassen sich in der Protein Sequence Database des NCBI finden, bzw. sind neue von den Erfindern isolierte Proteine und deren Sequenzen. 44RR2.8t, Acc.No: AAQ81466; RB49, Acc.No: AAQ15392; T2, Acc.No: CAA39905; T4, Acc.No: AAD42417; PP01, Acc.No: BAD20635; RB69, Acc.No: AAP76072; Aeh1, Acc.No: AAQ17871; KVP40, Acc.No: AAQ64417; AR1, Acc.No: AAN03609. Die entsprechenden Proteine der Phagen PHG31 und RB43 sind unter http://phage.bioc.tulane.edu/ zu finden; das Protein des Phagen K3 ist beschrieben in Burda M.R., Hindenach I., Miller S., Biol. Chem. (2000) 381, 225-258.

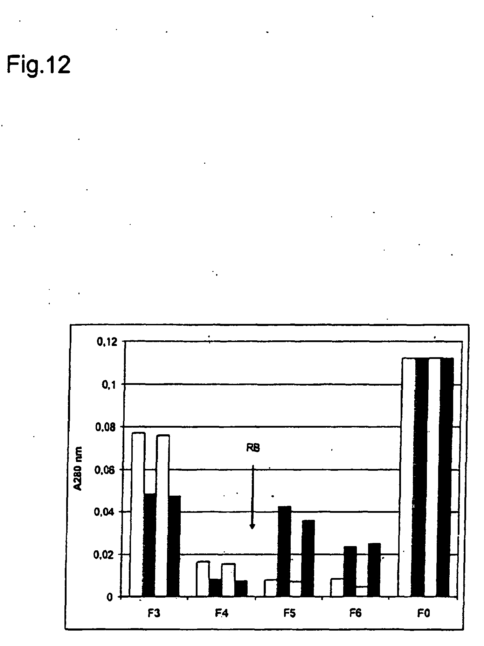

Figur 12 zeigt das Ergebnis des Nachweises von Endotoxin über die Bildung eines Miro2p12-Endotoxin-Miro2p12-Sandwiches in einem Säulenchromatographieverfahren. Es wurden 4 Säulen verwendet (Volumen 1 ml), bei denen Miro2p12 kovalent an Sepharose gekoppelt wurde (5 mg Protein/ml Säulenmaterial), wie in Beispiel 5 beschrieben. 2 davon wurden mit LPS von *E. coli* O55:B5 (10⁶ EU in 1ml PLS-Puffer, 10 mM Natriumphosphat, 70 mM NaCl, pH 7.4) beladen (+ ET, schwarze Balken), 2 dienten als Kontrollen (- ET, weiße Balken). Als Laufpuffer diente 10 mM Natriumphosphat, 80 mM NaCl, pH 7.4 Auf alle Säulen wurde Miro2p12 aufgetragen (je 600 µl einer Lösung mit 0.1 mg/ml Protein). Die Menge des aufgetragenen bzw. eluierten Miro2p12 wurde über Absorption bei 280 nm bestimmt. Die Menge des Bakteriophagenschwanzproteins Miro2p12 wurde gegen die Fraktionen des Chromatographielaufs aufgetragen. Fraktion 3 (F3) zeigt den Durchlauf von Miro2p12 nach dem Auftrag (F0), also alles Bakteriophagenschwanzprotein, das nicht auf der Säule zurückgehalten wurde, Fraktion 4 (F4) ist eine Waschfraktion. Nach dem Waschen wurde Regenerierungspuffer RB (10 mM Natriumphosphat, 500 mM NaCl, pH 7.4) zugegeben (siehe Pfeilmarkierung), oder an Endotoxin gebundenes Miro2p12 wieder von der Säule löst. Im Anschluss wurden die Fraktionen 5 und 6 gesammelt. Fraktion. F3 hat ein Volumen von 0.6 ml, alle weiteren Fraktionen haben ein Volumen von 1ml. Als Kontrolle ist der Auftrag auf die Säule (F0) mit der Gesamtmenge an Miro2p12 dargestellt. Man sieht, dass in den Säulen, auf denen zuvor Endotoxin immobilisiert wurde, Miro2p12 zurückgehalten wurde, während auf den Kontrollsäulen ohne Endotoxin nur eine geringe Menge Miro2p12 unspezifisch gebunden wurde.

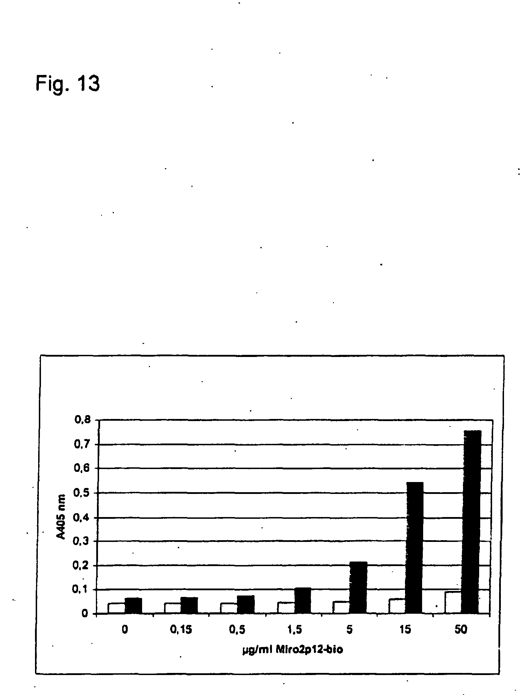

Figur 13 zeigt das Ergebnis des direkten Nachweise von immobilisiertem Endotoxin mit Hilfe von Biotin-markiertem Miro2p12. LPS von *E. coli* O55:B5 (3 µg/ml) wurde an PolySorp-Platten (Nunc) über Adsorption immobilisiert (18h bei Raumtemperatur in PBS-Puffer, 10 mM Na₂HPO₄, 1.8 mM KH₂PO₄, 137 mM NaCl, 2.7 mM KCl, pH 7.4). Anschliessend wurden die Mikrotiterplatten mit Casein (0.05 % in PBS, 1.5 h bei RT) blockiert und einmal mit PBS-Puffer gewaschen. Kontrollplatten wurden nicht mit Endotoxin inkubiert, sondern nur mit Casein blockiert. Jeweils 200 µl biotinmarkiertes Miro2p12 (Miro2p12-bio) in 50mM Tris, pH 8, 0.05% Casein, 0.05% Tween20 wurde in steigender Konzentration zugegeben (weiße Balken: Platten ohne ET, schwarze Balken: Platten mit ET, Protein-Konzentrationen wie angegeben). Der Nachweis von Endotoxin gebundenem biotin-markiertem Miro2p12 erfolgte über Absorptionsmessung bei 405 nm nach Bindung eines Konjugates aus Streptavidin mit alkalischer Phosphatase (Amersham Biosciences) unter Zugabe von pNPP (para-Nitrophenylphosphat) in einer Konzentration von 0.8 mg/ml als chromogenem Substrat. Biotinmarkiertes Miro2p12 bindet in konzentrationsabhängiger Form an die Mikrotiterplatten, die vorher mit Endotoxin beschichtet wurden.

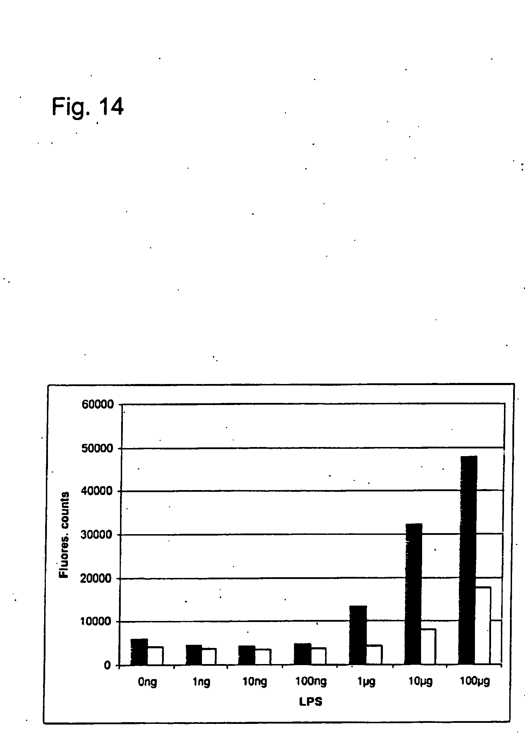

Figur 14 zeigt das Ergebnis der Bindung von FITC-markiertem Endotoxin auf eine Oberfläche, die vorher mit Miro2p12 beschichtet worden ist. Der Nachweis von an Bakteriophagenschwanzprotein gebundenem Endotoxin erfolgt in einem FITC-spezifischen. ELISA. Miro2p12 (je 200 µl mit 5 µg/ml Protein) wurde an eine MaxiSorp-Platte (Nunc) adsorbiert (16 h bei RT in PBS, 10 mM Na₂HPO₄, 1.8 mM KH₂PO₄, 137 mM NaCl, 2.7 mM KCl, pH 7.4). Anschliessend wurden die Mikrotiterplatten mit Casein (0.05 % Casein in PBS, 1.5h bei RT, je 200µl pro Vertiefung) blockiert und einmal mit PBS-Puffer gewaschen. Kontrollplatten wurden nicht mit Miro2p12 inkubiert, sondern nur mit Casein blockiert. Jeweils 100 µl FITC-markiertes LPS von *E. coli* O55:B5 (Sigma) in PBS wurde in steigender Konzentration zugegeben (weiße Balken: Platten ohne Mirc2p12, schwarze Balken: Platten mit Miro2p12). Der Nachweis des an Miro2p12 gebundenen fluoreszenzmarkierten LPS erfolgte über die Bindung eines FITC-spezifischen Antikörper (0.5 µg/ml, Zymed) in einem ersten Schritt und einen sekundären Antikörper, der mit alkalischer Phosphatase konjugiert war (1 µg/ml, Pierce). Die Quantifizierung erfolgte über Fluoreszenzmessung der Reaktionsprodukte eines fluoreszierenden alkalische Phosphatase Substrates (Methylumbelliferylphosphat; Sigma) mit 0.1 mg/ml Methylumbelliferylphosphat in 50 mM Tris, pH 8 (Anregung bei 375nm; Emission bei 465nm). Fluoreszenzmarkiertes Endotoxin bindet in konzentrationsabhängiger Form an die Mikrotiterplatten, die vorher mit Miro2p12 beschichtet wurden.

Der Begriff "Endotoxin-Abreicherung" oder "Endotoxin-Entfernung" wie hier verwendet bedeutet vollständige oder teilweise Entfernung von Endotoxin aus Probenmaterial.

Der Begriff "Probenmaterial" oder "Probe" wie hier verwendet umfasst sämtliche Lösungen, in denen Endotoxine nachgewiesen werden sollen oder aus denen Endotoxine entfernt werden sollen. Beispielhaft für Proben ist die folgende Aufzählung: Wasser, wässrige Lösungen und Gemische aus Wasser und organischen Lösungsmitteln, Blut, Blutprodukte, Plasma, Serum, Urin, Medien, Proteinlösungen, Wasser-Ethanol Gemische, Lebensmittel. Umfasst sind ferner auch Lösungen, in denen nicht wässrige feste zu untersuchende oder zu isolierende Substanzen gelöst wurden, beispielsweise Protein, DNA, RNA, Zucker, Salze, Arzneimittel, Impfstoffe, Lebensmittel, Organische und Anorganische Chemikalien (z.B. NaCl, MgCl₂, Purine, Pyrimidine, usw.).

Der Begriff "Endotoxin" wie hier verwendet bezeichnet bakterielles Lipopolysaccharid (LPS), das Bestandteil der äußeren Membran gram-negativer Bakterien ist.

Der Begriff "Calcium-unabhängige Bakteriophagenschwanzprotein" wie hier verwendet bezeichnet solche Proteine, die in Bakteriophagen vorkommen und Endotoxine unabhängig von der Gegenwart von zweiwertigen positiven Ionen wie Ca²⁺ oder Mg²⁺ binden können. Üblicherweise sind diese Proteine im Bakteriophagenschwanz lokalisiert, können jedoch auch auf dem Bakteriophagenkopf oder bei Bakteriophagen ohne Schwanz auf der normalen Bakteriophagenhülle lokalisiert sein. Der Begriff Bakteriophagenschwanzprotein umfasst sowohl kurze als auch lange Bakteriophagenschwanzproteine. So können Bakteriophagen mit einer Basisplatte (z.B. Myoviridae wie T4-ähnliche Phagen) unterschiedliche Bakteriophagenschwanzproteine, so genannte lange oder kurze Bakteriophagenschwanzproteine aufweisen, die auch unterschiedliche Spezifität für Strukturen von Bakterienmembranen besitzen. Somit sind vom Begriff "Bakteriophagenschwanzprotein" nicht nur die Endotoxin bindenden Bakteriophagenschwanzproteine sondern auch die Endotoxin bindenden Bakteriophagenkopfproteine und Bakteriophagenhüllproteine umfasst. Demzufolge sind Calcium-abhängige Bakteriophagenschwanzproteine solche, die nur in Gegenwart von zweiwertigen positiven Ionen wie Ca²⁺ oder Mg²⁺ Endotoxine binden können.

Der Begriff "unspezifische Immobilisierung" oder "ungerichtete Immobilisierung" wie hier verwendet bedeutet, dass die Kopplung eines Proteins an eine Matrix über Proteinreste (z.B. primäre Amine) erfolgt, die über die gesamte Proteinoberfläche verteilt sind. Die Auswahl der für die Kopplung des einzelnen Proteinmoleküls verwendeten Gruppe ist zufällig.

Der Begriff "gerichtete Immobilisierung" wie hier verwendet bedeutet, dass die Kopplung über Aminosäurereste oder andere Reste (z.B. Glykosyslierungen des Proteins) erfolgt, deren Position im Protein (z. B. N- oder C-terminal) bekannt ist. Die Auswahl dieser Gruppen für die Kopplung erfolgt durch die Auswahl geeigneter Reaktionspartner/Linker, die bevorzugt mit diesen Resten reagieren (z.B. Kopplung von Sulfhydrylresten an Iodoacetatreste; Iodoacetat reagiert tausendmal schneller mit Sulfhydrylresten als mit Aminoresten).

Der Begriff "Oberfläche" oder "Träger" wie hier verwendet umfasst alle Materialien, an die eine Kopplung oder Adhäsion eines Proteinmoleküls möglich ist, wie z.B. Glasoberflächen, Chromatographiematerialien, z.B. Agarose oder Sepharose, Plastikoberflächen, z.B. Polystyrol oder Polypropylen, Filtermaterialien, z.B. Cellulose.

Die vorliegende Erfindung betrifft Bakteriophagenschwanzproteine, die sowohl in Anwesenheit als auch in Abwesenheit vonzweiwertigen positiven Ionen, insbesondere in Abwesenheit von zweiwertigen positiven Ionen, insbesondere Ca²⁺ und/oder Mg²⁺, Endotoxine binden können.

Im Unterschied zu so genannten Calcium-abhängigen Bakteriophagenschwanzproteinen, also solchen die nur in Anwesenheit von zweiwertigen positiven Ionen, insbesondere Ca²⁺ und/oder Mg²⁺ Endotoxin binden können, ermöglichen die so genannten Calcium-unabhängigen Bakteriophagenschwanzproteine den Nachweis von Endotoxin in und das Entfernen von Endotoxin aus Proben, die keine zweiwertigen positiven Ionen, insbesondere Ca²⁺ und/oder Mg²⁺ enthalten.

Die erfindungsgemäßen Bakteriophagenschwanzproteine werden im Folgenden auch als "Calcium-unabhdngige Bakteriophagenschwanzproteine" bezeichnet. Bevorzugt sind kurze Schwanzproteine. Ferner bevorzugt sind Bakteriophagenschwanzproteine aus der Familie der Myoviridae, insbesondere aus der Gruppe der Pseudo-T-even, Schizo-T-Even oder der T-Even Phagen. Ferner bevorzugt sind Bakteriophagenschwanzproteine, die an die 2-Keto-3-desoxyoctonsäure (Kdo) der Endotoxine binden. Ferner bevorzugt sind Bakteriophagenschwanzproteine, die als Trimere vorliegen und resistent gegen Natriumdodecylsulfat (SDS) sind.

Die vorliegende Erfindung betrifft Bakteriophagenschwanzproteine gemäß SEQ ID NO:2 (Mirolp12), SEQ ID NO:4 (Miro2p12) und SEQ ID NO:6 (Effe04p12) sowie die DNA-Sequenzen gemäß SEQ ID NO: 1 (Miro1p12), SEQ ID NO:3 (Miro2p12) und SEQ ID NO:5 (Effe04p12) codierend für die erfindungsgemäßen Bakteriophagenschwanzproteine. Die vorliegende Erfindung betrifft ferner modifizierte Bakteriophagenschwanzproteine und die DNA-Sequenzen codierend für die modifizierten erfindungsgemäßen Bakteriophagenschwanzproteine. Modifizierte Bakteriophagenschwanzproteine weisen einen His-Tag oder Strep-Tag zur erleichterten Isolierung der Proteine nach rekombinanter Herstellung in Bakterien auf. Erfindungsgemäße Bakteriophagenschwanzproteine mit einem Strep-Tag sind die SEQ ID NO:8 (N-Strep Miro1p12), SEQ ID NO:10 (N-Strep Miro2p12), SEQ ID NO:12 (N-Strep Effe04p12) und SEQ ID NO:14 (N-Strep AehIp12) sowie die codierenden DNA-Sequenzen SEQ ID NO:7 (N-Strep Miro1p12), SEQ ID NO:9 (N-Strep Miro2p12), SEQ ID NO:11 (N-Strep Effe04p12) und SEQ ID NO:13 (N-Strep AehIp12). Ferner können Fragmente der Bakteriophagenschwanzproteine und die DNA-Sequenzen codierend für die Fragmente der Bakteriophagenschwanzproteine verwendet werden. Da der N-Terminus dieser Phagenproteine oft für die Bindung an den Phagen und der C-Terminus für die Bindung an den bakteriellen Rezeptor verantwortlich ist, wie für den Phagen T4 gezeigt wurde (Makhov AM, et al., Virology. 1993 May;194(1):117-127), werden insbesondere C-Terminale Fragmente bevorzugt. Derivate oder Fragmente können z.B. durch limitierte Proteolyse gewonnen werden (van Raaij MJ, et al., Biol Chem. 2001 Jul;382(7):1049-1055) oder willkürlich gentechnisch hergestellt werden. Die Fragmente können die Binderegion an den bakteriellen Rezeptor enthalten.

Die erfindungsgemäßen Bakteriophagenschwanzproteine können für die nachstehend beschriebenen Verfahren zur Entfernung und zum Nachweis von Endotoxinen verwendet werden. Die erfindungsgemäßen Bakteriophagenschwanzproteine binden Endotoxin und sind im Wesentlichen unabhängig von der Konzentration an zweiwertigen positiven Ionen, z.B. Ca²⁺ und/oder Mg²⁺. Die Endotoxine können somit in Lösungen oder Proben vorliegen, die zweiwertige positive Ionen enthalten oder nicht enthalten. Im Wesentlichen unabhängig bedeutet ferner, dass die Lösung oder Probe keine freien oder gebundenen zweiwertigen positiven Ionen aufweist. Die Lösung oder Probe kann einerseits völlig frei von zweiwertigen positiven Ionen sein. Die zweiwertigen positiven Ionen können aber andererseits durch zweiwertige positive Ionen bindende Substanzen, z.B. EDTA, HEDTA, EGTA, Citrat und ähnliche gebunden in der Lösung oder Probe vorliegen.

Bei den Bakteriophagenschwanzproteinen gibt es zwei Gruppen, die aufgrund der Abhängigkeit von zweiwertigen positiven Ionen, wie Calcium, in ihrer Bindung unterschieden werden können. P12 Bakteriophagenschwanzproteine z.B. der Myoviridae Phagen wie T4, T2, K3, Ox2, RB32-33, AR1, PP01 oder RB69 benötigen Calcium für die Bindung an Endotoxine, während die erfindungsgemäßen Bakteriophagenschwanzproteine der Phagen Miro1, Miro2 und Effe04, sowie zu diesen Bakteriophagenschwanzproteinen strukturell ähnliche Proteine, z.B. RB43p12, RB49p12, 44RR2p12, PHG31p12, AehIp12 und KVP40p12 auch ohne Calcium oder andere zweiwertige positive Ionen an Endotoxine binden können.

Der Bindungsmechanismus von Calcium-unabhängigen Bakteriophagenschwanzprotein Miro2p12 an Endotoxin unterscheidet sich von demjenigen des Calcium-abhängigen Bakteriophagenschwanzproteins T4p12. T4p12, ein Calcium-abhängiges Bakteriophagenschwanzprotein, benötigt für die Bindung an Endotoxine die Heptose-Kdo-Region in der inneren Herz-Region der Endotoxine. Miro2p12, ein Calcium-unabhängiges Bakteriophagenschwanzprotein, benötigt dagegen die Heptosen in der inneren Herz-Region nicht für die Bindung, vielmehr kann Miro2p12 auch Mutanten in der Herz-Region binden, die nur noch die 2-Keto-3-desoxyoctonsäure (Kdo) besitzen, siehe Tabelle 1. Dies belegen auch die in Figur 5 und Figur 6 beschriebenen Experimente, bei denen Miro2p12 und T4p12 gleichzeitig und damit an verschiedene Bindestellen von Endotoxin binden.

Die vorliegende Erfindung betrifft ferner ein Verfahren zur Entfernung von Endotoxin aus einer Probe, umfassend die Schritte:
a) Inkubation oder in Kontakt bringen einer Probe mit Bakteriophagenschwanzproteinen, die unspezifisch oder gerichtet, an einem festen Träger immobilisiert sind, wobei die Bakteriophagenschwanzproteine in der Lage sind, Endotoxin unabhängig von der Konzentration an zweiwertigen positiven Ionen zu binden, und anschließend
b) Trennen des Bakteriophagenschwanzprotein-Endotoxin-Komplexes von der Probe.

Für das erfindungsgemäße Abreicherungsverfahren sind die erfindungsgemäßen Bakteriophagenschwanzproteine an feste Träger gekoppelt. Die festen Träger können Füllmaterialien for Chromatographiesäulen (z.B. Sepharosematerialien), Cellulose, Filtrationsmedien, Glaspartikel, Magnetpartikel, Zentrifugations- oder Sedimentationsmaterialien (z.B. Agarosepartikel) sein.

Wichtig hierbei ist eine funktionelle Kopplung, d.h. Bakteriophagenschwanzproteine verfilgen trotz Bindung an das Trägermaterial über für Endotoxin zugängliche Strukturen. Die Kopplung der Bakteriophagenschwanzproteine kann unspezifisch, oder aber bevorzugt gerichtet, über z.B. eine selektive Biotinylierung, oder gekoppelt über einen Spacer oder Linker erfolgen.

Dazu können die erfindungsgemäßen Bakteriophagenschwanzproteine mit niedermolekularen Substanzen z.B. Biotin verknüpft sein, um über diese niedermolekularen Substanzen an Polypeptide z. B. Streptavidin zu binden, die ihrerseits auf dem Träger immobilisiert wurden. Statt Biotin kann ferner der sogenannte Strep-Tag (Skerra, A. & Schmidt, T. G. M., Biomolecular Engineering, 16 (1999), 79-86) verwendet werden, der eine kurze Aminosäuresequenz ist und an Streptavidin bindet. Ferner kann der His-Tag verwendet werden, der über zweiwertige Ionen (Zink oder Nickel) an Chelator-Materialien wie Nickel-Nitrilotriacetat (Ni-NTA, Qiagen, GmbH, Hilden; toleriert kein EDTA) oder Nickel-Sepharose (General Electric HealthcareBio-Sciences/Amersham Biosciences Europe GmbH, Freiburg; toleriert geringe EDTA-Konzentrationen) oder einen für ihn spezifischen Antikörper (Qiagen GmbH, Hilden) an ein Trägermaterial binden kann. Der Strep-Tag sowie der His-Tag wird vorzugsweise über DNA-Rekombinationstechnologie an die Bakteriophagenproteine gebunden. Diese Kopplung kann gerichtet, z.B. am N- oder C-Terminus oder ungerichtet erfolgen. Die gerichtete Kopplung erfolgt über eine geeignete reaktive, natürlicherweise bei Phagenproteinen nicht häufig oberflächenexponierte Aminosäure wie Cystein, das an geeigneter Stelle gezielt eingeführt wurde. Da Bakteriophagenschwanzproteine im Cytoplasma synthetisiert werden, ist nicht mit Disulfidbrücken zu rechnen. Vorzugsweise kann auch über andere Aminosäuren direkt, oder wie auch bei Cystein über einen "Spacer" oder "CrossLinker" (Monofunktionell oder bifunktionell) indirekt gekoppelt werden.

Bei der Cysteinkopplung sind alle bifunktionellen Crosslinker mit NH- und SH-reaktiven Gruppen, mit und ohne Spacer, z.B. 11-Maleimidouhdecanoic acid sulfo-NHS oder Succinimidyl-4-[N-maleimidomethyl]-cyclohexane-1-carboxy-[6-amido]caproate möglich. Sofern keine Spacer vorhanden sind, können 8-12 C-Atom-Spacer mit endständiger NH-Gruppe eingefügt werden. Vorzugsweise erfolgt die Cysteinkopplung über eine spezifische Biotinylierung des Cysteins durch z.B. EZ-Link-PEO-Maleimide activated Biotin (Pierce).

Ferner kann die Kopplung über bekannte Kopplungsreaktionen an Proteinreste, wie z.B. Carboxyl-, Amino-, Hydroxyl- oder Sulfhydrylreste erfolgen.

Die Konzentration an freien nicht gebundenen zweiwertigen positiven Ionen beträgt vorzugsweise weniger als etwa 0,1 µM.

Die Abtrennung von Endotoxinen kann in einem chromatographischen Verfahren oder dem so genannten "Batch"-Verfahren erfolgen. Beim "Batch"-Verfahren wird die mit Endotoxin belastete Probe mit Trägermaterial, an das die Bakteriophagenschwanzproteine der vorliegenden Erfindung kovalent gekoppelt sind, vermischt und gemeinsam inkubiert.

Die Abtrennung erfolgt nach Inkubation der Probe mit dem entsprechenden mit Bakteriophagenschwanzproteinen gekoppelten Trägermaterial für etwa 5-60 min oder etwa 30-180 min oder bei Bedarf auch über Nacht. Die Dauer der Inkubation muss an die jeweilige Probe angepasst werden und kann zwischen 1 min und 24 h variieren. Dazu wird die Probe z.B. aus der Chromatographiesäule eluiert oder filtriert oder die entsprechenden Partikel zentrifugiert oder sedimentiert bzw. durch Anlegen eines Magnetfeldes magnetisch separiert. Die Abtrennung in dem hier beschriebenen Batch-Verfahren, d.h. mit Vorinkubation von Probe und mit den entsprechenden Bakteriophagenschwanzproteinen gekoppelten Trägermaterialien, kann insbesondere bei sehr niedrigen Endotoxinkonzentrationen sinnvoll sein.

Die Abreicherung von Endotoxinen über Chromatographiesäulen kann aber auch im reinen Durchflussverfahren erfolgen. Dazu wird zunächst das mit dem erfindungsgemäßen Bakteriophagenschwanzprotein beladene Trägermaterial auf eine Chromatographiesäule gegossen. Die Probe kann dazu auf die Säule aufgetragen werden, die ein Trägermaterial mit daran gekoppelten Bakteriophagenschwanzproteinen enthält. Die mit Endotoxin belastete Probe wird auf diese Säule aufgetragen und fließt durch diese, wobei das Endotoxin an die Bakteriophagenschwanzproteine bindet und auf der Säule bleibt. Die Probe selbst zeigt idealerweise keine Wechselwirkung mit dem Chromatographiematerial und befindet sich im Durchlauf. Die Flussrate ist abhängig von Volumen und Geometrie der Säule. Die Flussrate ist ferner abhängig von Volumen und Endotoxingehalt der Probe, uin durch eine möglichst lange Kontaktzeit zwischen Säule und Endotoxin auch bei niedrigen Endotoxinkonzentrationen eine effiziente Abreicherung zu erzielen. Die Kontaktzeit ist dabei die Zeit, die die Probe vom Auftragen auf die Säule bis zum Herausfließen benötigt. Das auf der Säule gebundene Endotoxin kann durch Waschen mit geeigneten Puffern wieder von der Säule entfernt werden, so dass die Säulen mehrfach benutzt werden können.

Die für das erfindungsgemäße Verfahren zur Entfernung von Endotoxinen verwendeten Bakteriophagenschwanzproteine können alle vorstehend beschriebenen erfindungsgemäßen Bakteriophagenschwanzproteine gemäß SEQ ID NO:2, 4, 6, 8, 10, 12, 14 sein.

Ein weiterer Aspekt der vorliegenden Erfindung betrifft Verfahren zum Nachweis von Endotoxin. Die Detektion von Endotoxin erfolgt entweder über den direkten Nachweis von an das Endotoxin gebundenen Bakteriophagenschwanzproteinen oder aber indirekt über den Nachweis von überschüssigen, nicht gebundenen Bakteriophagenschwanzproteinen im Überstand oder Durchlauf.

Der direkte Endotoxin-Nachweis umfasst folgende Schritte:
a) In Kontakt bringen einer Probe enthaltend Endotoxine mit einer Oberfläche, anschließend gegebenenfalls
b) Entfernen der Probe von der Oberfläche, anschließend
c) Inkubieren von Bakteriophagenschwanzproteinen an die auf der Oberfläche immobilisierten Endotoxine, wobei die Bakteriophagenschwanzproteine in der Lage sind, Endotoxin unabhängig von der Konzentration an zweiwertigen positiven Ionen zu binden, und anschließend gegebenenfalls
d) Entfernen der ungebundenen Bakteriophagenschwanzproteine, und anschließend
e) Nachweis der an Endotoxin gebundenen Bakteriophagenschwanzproteine.

Im ersten Schritt erfolgt die Bindung von Endotoxin an eine Oberfläche. Die Oberfläche kann über Adsorption oder kovalente Kopplung mit einem Endotoxin-bindenden Liganden beschichtet sein. Als Oberflächen werden unterschiedliche Kunststoffe z.B. Polystyrol, Polypropylen, Polyethylen, Polycarbonat, PMMA, Cellulose z.B. Celluloseacetat, Nitrozellulose, Glas, Silizium oder Agarose bevorzugt. Die kovalente Immobilisierung von Endotoxin-bindenden Liganden, wie z.B. Polymyxin B, Histidin, Histamin, Poly-L-Lysin, DEAE, Polyethylenimin, Deoxycholsäure, Poly γ-aminomethyl-L-glutamin, Poly Vinylalkohol, Poly- N,N-dimethylaminopropylacrlyamid, Dextran, Chitosan oder Calcium-unabhängige-Bakteriophagenschwanzproteine erfolgt mittels bekannter Kopplungsreaktionen. Alternativ dazu können diese Liganden auch über Biotin-Streptavidin Kopplung an die Oberfläche gebunden werden.

Die Calcium-unabhängigen-Bakteriophagenschwanzproteine können dabei sowohl für die Bindung von Endotoxinen an die Oberfläche eingesetzt werden als auch für den Nachweis von gebundenen Endotoxinen verwendet werden. Da Endotoxine in der Regel nicht monomer, sondern als Aggregate in Lösung vorliegen (Mueller M, et al., J Biol Chem. 2004 Jun 18; 279(25):26307-26313), ist es auch möglich, die Bindung der Endotoxine über ein Calcium-unabhängiges Phagenschwanzprotein zu erreichen und dasselbe Calcium-unabhängige Phagenschwanzprotein auch für den Nachweis von Endotoxin zu benutzen.

Ebenso ist es möglich, eine Kombination von Calcium-abhängigen und Calcium-unabhängigen Bakteriophagenschwanzproteinen zu verwenden, wobei eines davon zur Immobilisierung des Endotoxins auf einer Oberfläche dient und das zweite zum Nachweis des gebundenen Endotoxins.

Ein weiteres Verfahren zum Nachweis von Endotoxin umfasst daher folgende Schritte:
a) In Kontakt bringen einer Probe enthaltend Endotoxine mit einer Oberfläche, an die erste Bakteriophagenschwanzproteine immobilisiert sind, die in der Lage sind, Endotoxin unabhängig von der Konzentration an zweiwertigen positiven Ionen zu binden, und anschließend gegebenenfalls
b) Entfernen der Probe von der Oberfläche, anschließend
c) Inkubieren von zweiten Bakteriophagenschwanzproteinen an die an die ersten Bakteriophagenschwanzproteine gebundenen Endotoxine, wobei die zweiten Bakteriophagenschwanzproteine Endotoxine nur in Gegenwart von zweiwertigen positiven Ionen binden, und anschließend gegebenenfalls
d) Entfernen der ungebundenen zweiten Bakteriophagenschwanzproteine, und anschließend Nachweis der an Endotoxin gebundenen zweiten Bakteriophagenschwanzproteine.

Das Verfahren kann ebenfalls durchgeführt werden, indem das erste Bakteriophagenschwanzprotein ein Calcium-abhängiges ist und das zweite ein Calcium-unabhängiges Bakteriophagenschwanzprotein ist.

Die Bakteriophagenschwanzproteine, die für die erfindungsgemäßen Nachweise eingesetzt werden sollen, müssen lediglich mit einem Marker versehen sein, über den die Nachweise des Proteins anschließend erfolgen.

Der Nachweis von Endotoxin in oder aus einer Probe erfolgt über die Bindung von Bakteriophagenschwanzproteinen an Endotoxin. Diese Bindung kann z.B. durch direkte Messung mittels spektroskopischer Verfahren, z.B. über Fluoreszenzemission; Fluoreszenzpolarisation, Absorption oder Circulardichroismus nachgewiesen werden. Darüber hinaus kann die Bindung durch elektrische Signale, z.B. eine Kapazitätsmessung sichtbar gemacht werden. Für den Fluorimetrischen Nachweis werden die Phagenschwanzproteine mit Fluoreszenzmarkern, wie z.B. Fluorescein, Alexa448 oder ähnliche, substituiert. Alternativ dazu erfolgt der Nachweis mit einem ELISA ähnlichem Verfahren, bei dem zunächst spezifische Antikörper an die Bakteriophagenschwanzproteine binden. Die Detektion dieser Antikörper geschieht mit Hilfe von Enzymen, die entweder direkt mit dem Antikörper fusioniert sind, oder aber mit einem sogenannten Zweit-Antikörper fusioniert sind, der an den Erst-Antikörper bindet. Als Enzyme kommen vor allem die Alkalische Phosphatase oder die Meerrettich Peroxidase, aber auch andere in Frage. Diese enzymatischen Markerproteine können auch direkt mit dem Phagenschwanzprotein gekoppelt werden. Dies kann einerseits durch Herstellung von Fusionsproteinen erfolgen oder andererseits durch chemische Kopplung der beiden Proteine. Alternativ können die Bakteriophagenschwanzproteine mit Biotin markiert werden, das über an Streptavidin gekoppelte Enzyme, wie alkalische Phosphatase oder Meerrettich Peroxidase, nachgewiesen wird.

Ein weiteres Verfahren zum direkten Nachweis von Endotoxin umfasst folgende Schritte:
a) In Kontakt bringen einer Probe enthaltend Endotoxine mit einer Oberfläche, an die Bakteriophagenschwanzproteine immobilisiert sind, die in der Lage sind, Endotoxin unabhängig von der Konzentration au zweiwertigen positiven Ionen zu binden, und anschließend gegebenenfalls
b) Entfernen der Probe von der Oberfläche, anschließend
c) Nachweis der in Schritt a) gebundenen Endotoxine

Der Nachweis von Endotoxin, das an ein Calcium-unabhängiges Bakteriophagenschwanzprotein gebunden hat, kann über einen endotoxinspezifischen ELISA erfolgen oder auch über chemische oder enzymatische Nachweisreaktionen von Endotoxinen oder abgespaltenen Endotoxinkomponenten.

Der indirekte Nachweis umfasst folgende Schritte:
a) In Kontakt bringen einer Probe enthaltend Endotoxine mit einer Oberfläche, anschließend gegebenenfalls
b) Entfernen der Probe von der Oberfläche, anschließend
c) Inkubieren von Bakteriophagenschwanzproteinen an die auf der Oberfläche immobilisierten Endotoxine, wobei die Bakteriophagenschwanzproteine in der Lage sind, Endotoxin unabhängig von der Konzentration an zweiwertigen positiven Ionen zu binden, und anschließend gegebenenfalls
d) Entfernen der ungebundenen Bakteriophagenschwanzproteine, und anschließend
e) Nachweis der in Schritt d) erhaltenen ungebundenen Bakteriophagenschwanzproteine. Werden die Endotoxine an Calcium-unabhängige Bakteriophagenschwanzproteine gebunden, die wiederum an der Oberfläche immobilisiert sind, umfasst das Verfahren folgende Schritte:
   a) In Kontakt bringen einer Probe enthaltend Endotoxine mit einer Oberfläche, an die erste Bakteriophagenschwanzproteine immobilisiert sind, die in der Lage sind, Endotoxin unabhängig von der Konzentration an zweiwertigen positiven Ionen zu binden, und anschließend gegebenenfalls
   b) Entfernen der Probe von der Oberfläche, anschließend
   c) Inkubieren von zweiten Bakteriophagenschwanzproteinen an die an die ersten Bakteriophagenschwanzproteine gebundenen Endotoxine, wobei die zweiten Bakteriophagenschwanzproteine Endotoxine nur in Gegenwart von zweiwertigen positiven Ionen binden, und anschließend gegebenenfalls
   d) Entfernen der ungebundenen zweiten Bakteriophagenschwanzproteine, und anschließend
   e) Nachweis der in Schritt d) erhaltenen ungebundenen zweiten Bakteriophagenschwanzproteine.

Die Bindung der Endotoxine erfolgt entweder über Calcium-unabhängige Bakteriophagenschwanzproteine, die wie vorstehend beschrieben an die Oberfläche immobilisiert sind, oder aber über andere Endotoxin-bindende Oberflächen. Der Nachweis der gebundenen Endotoxine erfolgt über Calcium-unabhängige oder Calcium-abhängige Bakteriophagenschwanzproteine, die nach Bindung der Endotoxine an die Oberfläche zugegeben werden und die zusätzlich mit einem Marker versehen sind, über den sie detektiert werden. Diese' werden in bekannter Menge auf die Oberfläche mit den Endotoxinen gegeben, inkubiert und anschließend werden die nicht gebundenen markierten Bakteriophagenschwanzproteine wieder abgenommen oder abgewaschen. Über die Abnahme der markierten Bakteriophagenschwanzproteine in dem Überstand oder Durchlauf wird die Endotoxinmenge, die an die Oberfläche gebunden hat, bestimmt.

Außerdem kann Endotoxin über einen kompetitiven Test nachgewiesen werden, bei dem markierte Endotoxine oder markierte Endotoxinbestandteile mit dem in einer Probe enthaltenen Endotoxin um die Bindestellen auf den Calcium-unabhängigen Phagenproteinen konkurrieren. Der Nachweis der Endotoxine erfolgt in diesem Test ebenfalls indirekt über die Bestimmung der an die Calcium-unabhängigen Bakteriophagenschwanzproteine gebundenen markierten Endotoxine oder über die markierten Endotoxine, die aufgrund der kompetitiven Hemmung nicht an die Calcium-unabhängigen Bakteriophagenschwanzproteine gebunden haben.

Der kompetitive Nachweis umfasst folgende Schritte:
a) Mischen der Probe mit Endotoxinen, die mit einem Marker gekoppelt sind, anschließend
b) Auftragen des Gemisches aus Schritt a) auf eine Oberfläche mit immobilisierten Bakteriophagenschwanzproteinen, wobei die Bakteriophagenschwanzproteine in der Lage sind, Endotoxin unabhängig von der Konzentration an zweiwertigen positiven Ionen zu binden, anschließend
c) Abnehmen des Gemisches von der Oberfläche, anschließend
d) Waschen der Oberfläche, und anschließend
e) Nachweis der markierten Endotoxine auf der Oberfläche und/oder des freien markierten Endotoxins der vereinigten Proben nach Schritt c) und d).

Die für diesen Nachweis notwendigen Endotoxine werden über bekannte Verfahren zur Reinigung von Endotoxinen gewonnen (Galanos C., et al., (1969), Eur. J. Biochem. 9, 245-249; Westphal O., Jann K. (1965) In R.L. Whisthler (ed.) Methods in carbohydrate chemistry, vol.5, 83-91) und mit Markern versehen. Es werden dieselben Marker wie für die Markierung der erfindungsgemäßen Bakteriophagenschwanzproteine wie Fluoreszenzmarker, Biotin, Digoxigenin, Antikörper, enzymatische Marker oder andere Marker verwendet und damit auch die entsprechenden Nachweismethoden. Lediglich die Kopplung, die bei den Endotoxinen über die Zuckerreste erfolgt ist unterschiedlich und erfolgt entsprechend bekannter Methoden zur Markierung von Zuckern (Toelstra A. et al. (1997) J. Leukoc. Biol. 61, 170-178; Triantafilou K. et al. (2000), Cytometry 41, 316-320). Die Quantifizierung erfolgt über eine Konzentrationsreihe mit einem Standardendotoxin.

Die in den erfindungsgemäßen Nachweisen verwendeten Calcium-unabhängigen Bakteriophagenschwanzproteine können die vorstehend beschriebenen erfindungsgemäßen Bakteriophagenschwanzproteine sein, die ebenfalls für die Entfernung von Endotoxin verwendet werden können.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

Beispiel 1: Einfluss von Calcium auf die Endotoxin Bindung von T4p12, N-Strep-Miro2p12 und N-Strep-Aeh1p12.

Die kurzen Schwanzfaserproteine T4p12, N-Strep-Miro2p12 und N-Strep-Aeh1p12 wurden auf NHS-aktivierter Sepharose 4 Fast Flow (Amersham Biosciences), entsprechend den Vorschriften des Herstellers, immobilisiert und anschließend die Bindung von Endotoxin an diese Sepharosen untersucht. Dazu wurden Säulen gegossen, auf diese Säulen eine Lösung mit Endotoxin aufgetragen und der Durchlauf aufgefangen. Der Endotoxin Gehalt im Auftrag und im Durchlauf wurde mittels LAL-Test (kinetisch-chromogener LAL-Test. Cambrex) bestimmt. Die Säulen hatten Volumina von 1 ml für T4p12 und N-Strep-Miro2p12 und 0.2 ml für N-Strep-Aeh1p12. Auf die T4p12 und N-Strep-Miro2p12 Säulen wurde jeweils 1 ml einer BSA Lösung (1 mg/ml)_{.} aufgetragen und auf die N-Strep-Aeh1p12 Säule 0.2 ml einer Pufferlösung. Die aufgetragenen Lösungen waren alle mit Endotoxin aus *E. coli* O55:B5 gespickt worden (etwa 1000 EU/ml). Um den Einfluß von Calcium auf die Endotoxin-Entfernung und damit die Endotoxin Bindung zu sehen, wurde der Versuch mit einem "Calcium-haltigen" Puffer und mit einem "Calcium-freien" Puffer durchgeführt. Der "Calcium-haltige" Puffer bestand aus 20 mM Hepes, 150 mM NaCl, 0.1 mM CaCl₂, pH 7.5 und der "Calcium-freie" Puffer aus 20 mM Hepes, 150 mM NaC1, 2 mM EDTA, pH 7.5. Die Konzentration freier Calcium Ionen beträgt im "Calcium-haltigen" Puffer 0.1 mM und im "Calcium-freien" Puffer mit dem Chelator EDTA unter 1 µM (Bers D. et al., in Methods in Cell Biology 40, A practical guide to the study of Ca2+ in living cells, chapter 1: A practical guide to the preparation of Ca2+ Buffers, Academic Press, 1/94).

Wie in Figur 1 gezeigt, konnte mit T4p12 nur im "Calcium-haltigen" Puffer Endotoxin aus der Lösung entfernt werden (99.9% Endotoxin Entfernung), während im "Calcium-freien" Puffer so gut wie kein Endotoxin aus der Lösung entfernt wurde (3.2% Endotoxin Entfernung). Die Endotoxin Entfernung über N-Strep-Miro2p12 Sepharose war dagegen unabhängig von der Calciumkonzentration im Puffer. Sowohl mit dem "Calcium-haltigen" (99.5% Endotoxin Entfernung) als auch mit dem "Calcium-freien" Puffer (99.9% Endotoxin Entfernung) konnten über 99% des Endotoxins aus der Lösung entfernt werden. Auch mit der N-Strep-Ach1p12 Sepharose konnte kein Einfluss von Calcium auf die Endotoxin Entfernung gefunden werden. Im "Calcium-haltigen" Puffer wurden 88.5% Endotoxin und im "Calcium-freien" Puffer 84.3% Endotoxin aus der Lösung entfernt.

Beispiel 2: 1. Konstruktion von Miro1, Miro2 und Effe04 mit N-terminalem Strep-Tag: Mittels PCR wurde an das 5' Ende des Miro2-Gens die Nukleotidsequenz für den Strep-Tag (US Patent 5,506,121) eingeführt. Hierfür wurde für das 5'-Ende des Micro2-Gens ein Primer konstruiert (5'-GAA GGA ACT AGT CAT ATG GCT AGC *TGG AGC CAC CCG CAG TTC GAA AAA* GGC GCC GCC CAG AAT AAC TAT AAT CAC-3'; SEQ.ID NO:15), der die Nucleotidsequenz des Strep-Tags an seinem 5'-Ende beinhaltet (kursiv in der Sequenz) und eine Restriktionsschnittstelle *(Nde*I*,* unterstrichen iri der Sequenz) derart besitzt, dass das Gen im richtigen Leseraster in das Expressionsplasmid eingesetzt werden kann. Für das 3'-Ende des Miro2p12-Gens wurde ein Primer konstruiert, der hinter dem Miro2p12-Gen eine *BamHI* Restriktionsschnittstelle (unterstrichen in der Sequenz) einführt (5'- CG GGA TCC TCC TTA CGG TCT ATT TGT ACA-3'; SEQ ID NO:16). Die PCR wurde mit 35 Zyklen (15s 94°C, 15s 51°C, 1 min 74°C) durchgeführt. Der PCR Ansatz wurde mit *Nde*I und *Bam*HI geschnitten und das gewünschte Fragment nach Größenfrationierung über Agarosegel und Elution aus dem Gel in die *Nde*I und *Bam*HI site des Expressionsplasmids pET21a eingesetzt. Die Sequenz des Miro2p12-Gens wurde über DNA-Sequenzierung auf seine Richtigkeit hin überprüft. Das Plasmid pNS-Miro2 wurde dann in den Expressionsstamm BL21 (DE3) transformiert. Die Klonierung von Miro1p12 erfolgte analog der oben beschriebenen Klonierung von Miro2p12. Es wurden dieselben Primer und Restriktionsenzyme verwendet. Die Konstruktion und Klonierung von N-Strep-Effe04p12 erfolgte analog der oben für N-Strep-Miro2p12 beschriebenen Prozedur. Für das 5'-Ende des N-Strep-Effe04p12 Gens wurde ein Primer mit der Sequenz 5'-GAA GGA ACT AGT GCT AGC GCT AGC *TGG AGC CAC CCG CAG* TTC *GAA AAA* GGC GCC AGT AAC AAT ACA ATC AAC CAC G-3' (SEQ ID NO: 17) verwendet, der eine Restriktionsschnittstelle für NdeI (unterstrichen in der Sequenz) und die Nucleotidsequenz des Strep-Tags (kursiv) enthält. Für das 3'-Ende wurde ein Primer mit der Sequenz 5'-CG GGA TCC CCT CTG TTA TAA TAC GCG-3' (SEQ ID NO: 18) verwendet, der eine Restriktionsschnittstelle für BamHI (unterstrichen in der Sequenz) enthält. Der PCR Ansatz wurde mit NdeI und BamHI geschnitten, in das Expressionsplasmid pET21a eingefügt und in den Expressionsstamm BL21(DE3) transformiert.

Beispiel 3. Reinigung von N-Strep-Miro2 Protein: Der *E*. *coli* Stamm BL21(DE3) mit dem Plasmid pNS-Miro2 wurde in 21 Schitttellculturen (LB-Medium mit Ampicillin, 100 µg(ml) bis zu einer OD600 von 0.5-0.7 bei 37°C gezogen und die Expression des N-Strep-Miro2-Proteins wurde durch Zugabe von 1mM IPTG (Isopropyl-β-thio-galactopyranoside) induziert. Nach Inkubation bei 37°C für 4h wurden die Zellen abgeerntet. Geerntete Zellen aus 10 1 Kultur wurden in 50 ml 10 mM Natriumphosphat, pH 8.0, 2 mM MgCl₂, 150 mM NaCl aufgenommen, durch dreimalige French-Press-Behandlung (20.000 psi) aufgebrochen und anschließend 30 min bei 15.000 rpm (SS34) zentrifugiert. Nach zweimaligem Waschen im gleichen Puffer wurde das N-Strep-Miro2 Protein aus dem Pellet durch Rühren für 30 min in 10 mM TrisHCl pH 8.0, 150 mM NaCl, 1 M Harnstoff extrahiert, der Ansatz für 30 min bei 15.000 rpm (SS34) zentrifugiert und das abgelöste N-Strep-Miro2 im Überstand bei 4°C gelagert. Die Extraktion wurde zweimal wiederholt. Die vereinigten Überstände wurden auf eine Streptactin-Affinitätssäule (15 ml), äquilibriert mit Puffer "W" (100 mM TrisHCl pH 8, 1 mM EDTA, 150 mM NaCl), aufgetragen (IBA GmbH, Göttingen). Nach Waschen mit 5 Säulenvolumina Puffer "W" wurde mit 3 Volumina Puffer "W" mit 2.5 mM Desthiobiotin in Puffer "W" eluiert. Nach mehrmaliger Dialyse gegen Puffer "W" und Aufkonzentration wurde über SDS-PAGE und UV-Spektroskopie die Konzentration und Reinheit von N-Strep-Miro2 ermittelt. Aus 10 Litern Kultur wurden so ca. 100 mg N-Strep-Miro2 gereinigt

Beispiel 4: Nachweis der Bindung verschieden langer Endotoxin Varianten an N-Strep-Miro2p12 mittels Oberflächen-Plasmon-Resonanz-Spektroskopie. Dieses Experiment dient dazu, Aufschluss über die von Miro2p12 erkannte Struktur in der Herzregion (Core) des Endotoxins zu gewinnen. N-Strep-Mirop12 wurde kovalent an die Oberfläche einer Zelle auf einem CM-5 Chip der Firma Biacore gekoppelt. Dazu wurde die Oberfläche zunächst mit EDC/NHS aktiviert anschließen N-Strep-Miro2p12 über primäre Aminoreste gebunden und danach unverbrauchte
Kopplungsgruppen mit Ethanolamin abgesättigt (siehe Biacore Handbuch). Endotoxin wurde aus E. coli Stämmen isoliert, die unterschiedlich lange Core Zucker besitzen (siehe Figur 9, Lit.: Boman H.G., Jonsson S., Monner D., Normark S., Bloom G.D., Cell-Surface alterations, in Escherichia coli K-12 with chromosomal mutations changing ampicillin resistance. Ann. N.Y. Acad. Sci. 1971; 182: 342-357; Prehm P. Stirm S., Jann B., Jann K., Bomann H.G., Cell-wall lipopolysaccharides of ampicillin-resistant mutants of Escherichia coli K-12. Eur. J. Biochem. 1976; 66(2): 369-377; Eriksson-Grennberg K.R., Nordstrom K., Englund P., Resistance of Escherichia coli to penicillins. IX. Genetics and physiology of class II ampicillin-resistant mutants that are galactose negative or sensitive to bacteriophage C21, or both. J. Bacteriol. 1971; 108(3): 1210-1223; Boman H.G., Monner D.A., Characterization of lipopolysaccharides from Escherichia coli K-12 mutants. J. Bacteriol. 1975; 121(2): 355-464). Dazu wurden die Bakterien über Nacht in LB-Medium bei 37°C angezogen, durch Zentrifugation geerntet, mit PBS gewaschen und anschließend das Pellet in 100 mM Tris, 50 mM EDTA, pH 8 aufgenommen und 30 min bei Raumtemperatur inkubiert. In diesem Puffer geht ein Teil des Endotoxins in Lösung und kann von den Zellen abgetrennt werden. Das lösliche Endotoxin wurde anschließend mit 4-fachem Volumen Aceton gefällt und getrocknet. Für die Messung wurde das Endotoxin im Laufpuffer aufgenommen (Laufpuffer: 20 mM Hepes, 150 mM NaCl, 0.005% Tween 20, pH 7.5) und über die mit N-Strep-Miro2p12 beladene Oberfläche gespült. Als Kontrolle diente eine zweite, unbeladene Zelle auf dem Biacore Chip. Die Endotoxinlösung wurde während der Messung über beide Zellen gegeben und aus der Signaldifferenz von Zellel und Zelle2 wurde das resultierende Signal errechnet. Die Zunahme des Signals in der resultierenden Kurve wurde als Bindung des Endotoxins an das N-Strep-Miro2p12 interpretiert. Wie in Tabelle 1 gezeigt, konnte auch mit dem Endotoxin aus dem E. coli Stamm D21f2 eine Bindung gefunden werden. Dieser Stamm enthält nur noch die Kdo-Core-Zucker und damit die kürzeste Endotoxin Form, die unbedingt notwendig für das Überleben der Zellen ist. Mit T4p12 konnte keine Bindung dieser Bakterien gefunden werden. Dabei bedeutet der Zusatz R, dass diese Bakterien zum sogenannten "rough"-Typ gehören, dessen Endotoxin kein O-Antigen besitzt und die kleinen Buchstaben kennzeichnen die von a zu e abnehmende Länge der Core Zucker im Endotoxin. Die entsprechenden Core-Zucker sind in Figur 9 skizziert.

| **Tabelle 1** | | |
|---|---|---|
| Bakterienstamm | Bindung an T4p12 | Bindung an N-Strep-Miro2p12 |
| *E. coli* D21, Ra | + | + |
| *E. coli* D21e7, Rb | + | n.b. |
| *E. coli* D21e8, Rc | + | n.b. |
| *E. coli* D21f1, Rd | + | + |
| *E. coli* D21f2, Re | - | + |
| | | n.b. = nicht bestimmt |

### Beispiel 5: Endotoxin Entfernung über das Bakteriophagenschwanzprotein Miro2.

5 ml NHS-aktivierte Sepharose 4 Fast Flow (Amersham Biosciences) wurde abzentrifugiert, der Isopropanol Überstand abgenommen und mit 870 ml Citrat Puffer (25 mM Citrat, 2 mM EDTA, pH 7.0) vermischt. Anschließend wurden 217 ml Bakteriophagenschwanzprotein Miro2 (0.46 mg/ml in 50 mM Formiat, pH 3.5) zugegeben und zum Koppeln des Bakteriophagenschwanzproteins an die Sepharose 2 Stunden bei 37°C geschüttelt. Der Überstand wurde abgenommen, die Sepharose 3-mal mit 10 mM Natriumphosphat, pH 10 gewaschen und 1 ml Aliquots von Überstand und Waschfraktionen gegen 10 mM Natriumphosphat, pH 10 dialysiert. Die Konzentration des Bakteriophagenschwanzproteins in diesen Aliquots wurde durch Absorptionsmessung bei 280 nm bestimmt und die Menge des Bakteriophagenschwanzproteins, das an die Sepharose gebunden hat, errechnet. Es wurden 12.2 mg Bakteriophagenschwanzproteins Miro2 pro 1 ml Sepharose gebunden.

Es wurden Säulen mit einem Volumen von 1.5 ml gegossen. Außerdem wurden Säulen mit dem gleichen Volumen aus T4p12 Material und Polymyxin B-Sepharose gegossen. Jeweils eine dieser Säulen wurde mit dem Laufpuffer (20 mM Hepes, 150 mM NaCl, 2 mM EDTA, pH 7.5) äquilibriert und anschließend die Endotoxin Entfernung aus einer BSA-Lösung (1.2 mg/ml), die gespickt war mit Lipopolysaccharid von E. coli O55:B5 (etwa 1000 EU/ml), untersucht. Dazu wurde vor dem Auftrag der Probe von jeder Säule (Miro2p12, T4p12, Polymyxin B) zunächst 1 ml Laufpuffer gesammelt, anschließend jeweils 1 ml BSA-Lösung aufgetragen und mit Laufpuffer gewaschen. Der Durchlauf wurde fraktioniert und die Fraktionen auf Protein (BSA) und Endotoxin untersucht. Die Ergebnisse sind in Figur 2 dargestellt. Mit T4p12, das für die Bindung von Endotoxin freies Calcium benötigt, konnte kein Endotoxin aus der Lösung entfernt werden. Mit Polymyxin B wurde der Endotoxingehalt um 96 % verringert, aber es wurden nur 67 % des aufgetragenen BSA wieder gefunden. Mit Miro2p12 konnten mehr als 99% des Endotoxins entfernt werden und die Wiederfindungsrate für BSA betrug 92% und war damit deutlich höher als für Polymyxin B.

Ähnliche Versuche wurden mit PBS (10 mM Na2HPO4, 1.8 mM KH2PO4, 137 mM NaCl, 2.7 mM KCl, pH 7.4) und Citrat (20 mM Citrat, 150 mM NaCl, pH 7.0) als Laufpuffer durchgeführt. Auch in diesen Puffern ist die Endotoxin Entfernung mit T4p 12 stark vermindert, da Calcium entweder präzipitiert (Phosphat-Puffer) oder komplexiert wird (Citrat). Die Ergebnisse sind in den Figuren 3 und 4 dargestellt und zeigen für T4p12, wie zu erwarten, eine geringe (PBS) bzw. keine Endotoxin Entfernung (Citrat). Mit Polymyxin B konnten jeweils mehr als 96 % des Endotoxins entfernt werden, aber die BSA Wiederfindung lag nur bei 60-80%. Mit Miro2p12 wurden mehr als 99% des Endotoxins entfernt und die BSA Wiederfindung war größer als 90%. Außerdem wurde die Eignung von N-Strep-Miro2p12 für die Endotoxin Entfernung aus Serum untersucht. Dazu wurde menschliches Serum mit Lipopolysaccharid aus E. coli 055:B5 gespickt und auf N-Strep-Miro2p12 Sepharose Säulen aufgetragen (siehe Figur 7). Es konnten etwa 90% des aufgetragenen Endotoxins aus menschlichem Serum entfernt werden.

### Beispiel 6: Nachweis von LPS über die Bindung von T4p12 an immobilisiertes LPS.

Es wurde Bakteriophagenprotein Miro2p12 auf NHS-Sepharose (Amersham Pharmacia) immobilisiert (3.4 mg Protein pro 1 ml Sepharose) und daraus 4 Säulen mit je 0.5 ml Volumen gegossen. Die Säulen wurden mit je 3 ml Natriumphosphat Puffer (20 mM Natriumphosphat, pH . 12.0) und je 3 ml Regenerationspuffer (20 mM Hepes, 150 mM NaCl, 2 mM EDTA, pH 7.5) gewaschen. Anschließend wurden auf zwei dieser Säulen je 1 ml LPS von E. coli 055:B5 aufgetragen (0.1 mg/ml in Hepes Puffer, 10⁶ EU/ml). Die zwei anderen Säulen wurden mit je 1 ml Regenerationspuffer gespült. Danach wurden alle Säulen mit je 3 ml Äquilibrationspuffer (20 mM Hepes, 150 mM NaCl, 0.1 mM CaCl₂, pH 7.5) gewaschen und anschließend nochmals 1 ml dieses Puffers aufgetragen und als Fraktion 1 aufgefangen. Anschließend wurden 0.5 ml einer Lösung mit dem Bakteriophagenschwanzprotein T4p12 (0.844 mg/ml in 20 mM Hepes, 150 mM NaCl, 0.1 mM CaCl₂) auf die Säulen aufgetragen und mit 2.5 ml Äquilibrationspuffer und 2 ml Regenerationspuffer gewaschen. Der Durchlauf wurde in Fraktionen von dreimal 1 ml und einmal 2 ml aufgefangen und die Konzentration des Bakteriophagenschwanzproteins T4p12 in diesen Fraktionen mittels Absorptionsmessung bei 280 nm bestimmt (Figur 5). Das Bakteriophagenschwanzprotein T4p12 wurde zum großen Teil an die Säulen gebunden, die vorher mit LPS behandelt worden waren, und konnte durch Zugabe von Regenerationspuffer von diesen Säulen gelöst werden. Im Gegensatz dazu lief es ohne Verzögerung durch die Säulen, die nicht mit LPS behandelt worden waren.

Beispiel 7: 1. Konstruktion von Aeh1 mit N-terminalem Strep-Tag (N-Strep-Aeh1p12): Mittels PCR wurde an das 5' Ende des Aeh1p12-Gens (NCBI Acc.Nr.AAQ17871) die Nukleotidsequenz für den Strep-Tag (US Patent 5,506,121) eingeführt. Hierfür wurde für das 5'-Ende des Aeh1-Gens ein Primer konstruiert (5'-GAA GGA ACT AGT CAT ATG GCT AGC *TGG AGC CAC CCG CAG TTC GAA AAA* GGC GCC AGA ACA AAT AAT ATC ACA CAG 3'; SEQ ID NO:19), der die Nukleotidsequenz des Strep-Tags an seinem 5'-Ende beinhaltet (kursiv in der Sequenz) und eine Restriktionsschnittstelle (*Nde*I*,* unterstrichen in der Sequenz) derart besitzt, dass das Gen im richtigen Leseraster in das Expressionsplasmid eingesetzt werden kann. Für das 3'-Ende des Miro2p12-Gens wurde ein Primer konstruiert, der hinter dem Aeh1p12-Gen eine *Bam*HI Restriktionsschnittstelle (unterstrichen in der Sequenz) einführt (5'-GAA GGA ACT AGT CAT ATG AGA ACA AAT AAT ATC ACA CAG 3'; SEQ ID NO:20). Die PCR wurde mit 35 Zyklen (15s 94°C, 15s 51°C, 1 min 74°C) durchgeführt. Der PCR Ansatz wurde mit *NdeI* und *Bam*HI geschnitten und das gewünschte Fragment nach Größenfraktionierung über Agarosegel und Elution aus dem Gel in die *Nde*I und *Bam*HI site des Expressionsplasmids pE121a eingesetzt. Die Sequenz des Aehlp12-Gens wurde über DNA-Sequenzierung auf seine Richtigkeit hin überprüft. Das Plasmid pNS-Aeh1 wurde dann in den Expressionsstamm HMS174 (DE 3) transformiert.

2. Reinigung von N-Strep-Aeh1 Protein: Der *E. coli* Stamm HMS174 (DE 3) mit dem Plasmid pNS-Aeh1p12 wurde in 21 Schüttelkulturen (LB-Medium mit Ampicillin 100 µg/ml, Kanamycin 25 µg/ml, Rifampicin 10 µg/ml) bis zu einer OD600 von 0.5-0.7 bei 37°C gezogen und die Expression des N-Strep-Aeh1p12-Proteins wurde durch Zugabe von 1mM IPTG (Isopropyl-βthio-galactopyranoside) induziert. Nach Inkubation bei 37°C für 4 h wurden die Zellen abgeerntet. Geerntete Zellen aus 10 1 Kultur wurden in 50 ml 10 mM Natriumphosphat, pH 8.0, 2 mM MgCl₂, 150 mM NaCl aufgenommen, in einem Microfluidizer (Microfluidics, M110L) aufgebrochen und anschließend 30 min bei 15.000 rpm (SS34) zentrifugiert. Nach zweimaligem Waschen im gleichen Puffer wurde das N-Strep-Aeh1p12 Protein aus dem Pellet durch Rühren für 30 min in 50 mM Natriumphosphat pH 12 extrahiert, und der Ansatz für 30 min bei 15.000 rpm (SS34) zentrifugiert. Die Extraktion wurde einmal wiederholt und die vereinigten Überstände mit dem abgelösten N-Strep-Aeh1p12 gegen 100 mM Tris, 150 mM NaCl, pH 8.0 dialysiert. Das Protein wurde anschließend über eine Streptactin-Affinitätssäule (5 ml, IBA GmbH, Göttingen) weiter gereinigt. Dazu wurde die Streptactin-Affinitätssäule mit Puffer "W" (100 mM TrisHCl pH 8, 1 mM EDTA, 150 mM NaCl) äquilibriert und Aeh1p12 aufgetragen. Nach Waschen mit 5 Säulenvolumina Puffer "W" wurde mit 3 Volumina Puffer "W" mit 2.5 mM Desthiobiotin in Puffer "W" eluiert. Nach mehrmaliger Dialyse gegen 100 mM Borat, 150 mM NaCl, pH 8 wurde über SDS-PAGE und UV-Spektroskopie die Konzentration und Reinheit von N-Strep-Aeh1p12 ermittelt. Aus 4 Liter Kultur wurden so ca. 20 mg N-Strep-Aeh1p12 gereinigt.

3. Kopplung von Aeh1p12 an NHS-aktivierte Sepharose. 22 ml N-Strep-Aeh1p12 (0.9 mg/ml in 100 mM Borat, 150 mM NaCl, pH 8) wurden mit 200 µl NHS-aktivierter Sepharose versetzt und 3 h bei Raumtemperatur auf einem Roller inkubiert. Danach wurde die Sepharose abzentrifugiert (15 min, 3000g) und 3x mit jeweils 20 ml 100 mM Tris, 150 mM NaCl, pH 8 gewaschen. Anschließend wurden 0.5 ml Aliqouts der Waschfraktionen gegen 100 mM Tris, 150 mM NaCl, pH 8 dialysiert, um das abgetrennte NHS zu entfernen, und in diesen Aliquots die Konzentration von N-Strep-Aeh1p12 durch Absorptionsmessung bei 280nm bestimmt. Aus der Ausgangsproteinmenge und den Proteinmengen in den Waschfraktionen wurde die Belegungsdichte errechnet. Es konnten 2.5 mg N-Strep-Aeh1p12 pro 1 ml Sepharose gekoppelt werden.

4. Endotoxin Entfernung mit Aeh1p12-Sepharose. Es wurde eine Säule mit Aeh1p12-Sepharose und eine Kontroll-Säule gegossen, beide mit jeweils einem Volumen von 200 µl. Die Kontrollsäule bestand aus NHS-Sepharose, die mit Ethanolamin abgesättigt worden war. Beide Säulen wurden anschließend mit 2 ml 0.5 % Natriumdeoxycholat, 15 ml pyrogenfreiem Wasser und 2 ml Laufpuffer gewaschen. Als Probe wurde jeweils 200 µl Lipopolysaccharid von *E. coli* O55:B5, das im jeweiligen Laufpuffer gelöst war, aufgetragen. Nach dem Aufbringen der Probe wurde diese mit der Sepharose vermischt und 30 min bei Raumtemperatur inkubiert, bevor die Lösung aus der Säule ablief. Die Säulen wurden zunächst mit 0.6 ml und danach noch 2-mal mit 1 ml Laufpuffer gewaschen. Der Durchlauf wurde fraktioniert und die Endotoxinkonzentrationen im Auftrag und den Fraktionen mittels LAL-Test (kinetisch-chromogener LAL-Test, Cambrex) bestimmt. Dieser Versuch wurde mit 2 unterschiedlichen Laufpuffern durchgeführt, um den Einfluß von Calcium auf die Endotoxin Entfernung zu untersuchen. Die Laufpuffer waren wie folgt zusammengesetzt: Puffer 1: 20 mM Hepes, 150 mM NaCl, 0.1 mM CaCl₂, pH 7.5. Puffer 2: 20 mM Hepes, 150 mM NaCl, 2 mM EDTA, pH 7.5. Wie in Figur 8 gezeigt, wird über die Aeh1p12-Sepharose Säule deutlich mehr Endotoxin entfernt (88% und 84%) als über die unbeladene Kontrollsäule (25% und 39%). Calcium spielt für die Bindung von Endotoxin an Ach1p12 keine Rolle, da die Endotoxin Entfernung in Gegenwart von Calcium (88%) ähnlich der in einem Puffer mit einem Calcium Chelator (84%) war.

### Beispiel 8:

1. Reinigung von N-Strep-Effe04p12. Der *E. coli* Stamm HMS174 (DE 3) mit dem Plasmid pNS-Effe04p12 wurde in 21 Schüttelkulturen (LB-Medium mit Ampicillin 100 µg/ml, Rifampicin 10 µg/ml) bis zu einer OD600 von 0.5-0.7 bei 37°C gezogen und die Expression des N-Strep-Effe04p12-Proteins wurde durch Zugabe von 1mM IPTG (Isopropyl-β-thiogalactopyranoside) induziert. Nach Inkubation bei 37°C für 4h wurden die Zellen abgeerntet. Geerntete Zellen aus 6 1 Kultur wurden in 50 ml 100 mM Tris, 25 mM EDTA, 100 mM NaCl, pH 8.0 aufgenommen, in einem Microfluidizer (Microfluidics, M110L) aufgebrochen und anschließend 30 min bei 15.000 rpm (SS34) zentrifugiert. Anschließend wurde das N-Strep-Effe04p12 Protein aus dem Pellet durch Rühren für 2 h bei 37°C in 100 mM Tris, 1.5 M Guanidinhydrochlorid, pH 8.0 extrahiert, und der Ansatz für 30 min bei 13.000 rpm (SS34) zentrifugiert. Die Extraktion wurde einmal wiederholt. Die vereinigten Überstände wurden mit dem gelösten N-Strep-Effe04p12 gegen 100 mM Tris, pH 8.0 dialysiert. Das Protein wurde anschließend über eine Streptactin-Affinitätssäule (5 ml, IBA GmbH, Göttingen) weiter gereinigt. Dazu wurde die Streptactin-Affinitätssäule mit Puffer "W" (100 mM TrisHCl pH 8, 1 mM EDTA, 150 mM NaCl) äquilibriert und N-Strep-Effe04p12 aufgetragen. Nach Waschen mit 5 Säulenvolumina Puffer "W" wurde mit 3 Volumina Puffer "W" mit 2.5 mM Desthiobiotin in Puffer "W" eluiert. Nach mehrmaliger Dialyse gegen 100 mM Borat, 150 mM NaCl, pH 8 wurde über SDS-PAGE und UV-Spektroskopie die Konzentration und Reinheit von N-Strep-Effe04p12 ermittelt. Aus 6 Liter Kultur wurden so ca. 2 mg N-Strep-Effe04p12 gereinigt
2. Kopplung von N-Strep-Effe04p12 an NHS-aktivierte Sepharose. 4 ml N-Strep-Effe04p12 (0.2 mg/ml in 100 mM Borat, 150 mM NaCl, pH 8) wurden mit 100 µl NHS-aktivierter Sepharose versetzt und 3 h bei Raumtemperatur auf einem Roller inkubiert. Danach wurde die Sepharose abzentrifugiert (15 min, 3000g) und 3x mit jeweils 20 ml 100 mM Tris, 150 mM NaCl, pH 8 gewaschen. Anschließend wurden 0.5 ml Aliqouts der Waschfraktionen gegen 100 mM Tris, 150 mM NaCl, pH 8 dialysiert, um das abgetrennte NHS zu entfernen, und in diesen Aliquots die Konzentration von N-Strep-Effe 04p12 durch Absorptionsmessung bei 280nm bestimmt. Aus der Ausgangsproteinmenge und den Proteinmengen in den Waschfraktionen wurde die

Belegungsdichte errechnet. Es konnten 3.6 mg N-Strep-Effe04p12 pro 1 ml Sepharose gekoppelt werden.

3. Endotoxin Entfernung mit N-Strep-Effe04p12 Sepharose. 100 µl N-Strep-Effe04p12-Sepharose wurden in eine Mini-Spin Säule (Handee Mini-Spin, Pierce) gegeben und zunächst 3-mal mit 1 ml endotoxinfreiem Laufpuffer (20 mM Hepes, 150 mM NaCl, 2 mM EDTA) gewaschen. Die Lösung wurde dabei durch kurze Zentrifugation (400g, 5s) von der Sepharose abgetrennt. Anschließend wurde 100µl Endotoxin Lösung (Lipopolysaccharid von *E. coli* O55:B5 in Laufpuffer) 30 min mit der Sepharose inkubiert bevor die Lösung zentrifugiert wurde und die Sepharose noch zweimal mit 200 µl Laufpuffer gewaschen wurde. Die Endotoxinmengen im Auftrag (F0) sowie den Fraktionen nach der Sepharose wurden mittels LAL-Test bestimmt (kinetisch-chromogener LAL-Test, Cambrex). Wie in Figur 9 zu sehen, wird der größte Teil des aufgetragenen Endotoxins aus der Lösung entfernt (Endotoxin Entfernung: 92%). Da im Laufpuffer EDTA enthalten war, spielt Calcium für die Bindung von Endotoxin an N-Strep-Effe04p12 keine Rolle.

Beispiel 9: Nachweis von Endotoxin über die Bindung von Miro2p12 an immobilisiertes LPS. Es wurde Bakteriophagenprotein Miro2p12 auf NHS-Sepharose (Amersham Pharmacia) immobilisiert (5 mg Protein pro 1 ml Sepharose) und daraus 4 Säulen mit je 1 ml Volumen gegossen. 2 davon wurden mit LPS von *E. coli* O55:B5 (10⁶ EU in 1ml PLS-Puffer, 10 mM Natriumphosphat, 70 mM NaCl, pH 7.4) beladen (+ ET, schwarze Bälken, Fig 12), 2 dienten als Kontrollen (- ET, weiße Balken, FIg. 12). Als Laufpuffer diente 10 mM Natriumphosphat, 80 mM NaCl, pH 7.4 Auf alle Säulen wurde Miro2p12 aufgetragen (je 600 µl einer Lösung mit 0.1 mg/ml Protein). Die Menge des aufgetragenen bzw. eluierten Miro2p12 wurde über Absorption bei 280 nm bestimmt. Die Menge des Bakteriophagenschwanzproteins Miro2p12 wurde gegen die Fraktionen des Chromatographielaufs aufgetragen, wie in Figur 12 abgebildet. Fraktion 3 (F3) zeigt den Durchlauf von Miro2p12 nach dem Aüftrag (F0), also alles Bakteriophagenschwanzprotein, das nicht auf der Säule zurückgehalten wurde, Fraktion 4 (F4) ist eine Waschfraktion. Nach dem Waschen wurde Regenerierungspuffer RB (10 mM Natriumphosphat, 500 mM NaCl, pH 7.4) zugegeben (siehe Pfeilmarkierung), der an Endotoxin gebundenes Miro2p12 wieder von der Säule löst. Im Anschluss wurden die Fraktionen 5 und 6 gesammelt. Fraktion F3 hat ein Volumen von 0.6 ml, alle weiteren Fraktionen haben ein Volumen von 1ml. Als Kontrolle ist der Auftrag auf die Säule (F0) mit der Gesamtmenge an Miro2p12 dargestellt. Man sieht, dass in den Säulen, auf denen zuvor Endotoxin immobilisiert wurde, Miro2p12 zurückgehalten wurde, während auf den Kantrollsäulen ohne Endotoxin nur eine geringe Menge Miro2p12 unspezifisch gebunden wurde.

Beispiel 10: Nachweis von Endotoxin adsorbiert an einer PolySorp Mikrotiterplatte über die Bindung von biotinmnarkiertem Miro2p12.

Herstellung von biotinmarkiertem Miro2p12. 2 ml einer Miro2p12 Lösung mit einer Konzentration von 2 mg/ml in einem Puffer mit 50 mM NaBorat, 1.75 M Guanidiniumchlorid, pH 8.0 werden mit 250 µl einer 10 mM Sulfo-NHS-LC-LC-Biotin Lösung in Wasser (Pierce) versetzt und 30 min bei Raumtemperatur inkubiert. Anschliessend wird die Reaktionslösung gegen Puffer mit 50 mM NaBorat, 1.75 M Guanidiniumchlorid, pH 8.0 dialysiert. Das so gewonnene Miro2p12-bio wurde für den folgenden Endotoxin Nachweis eingesetzt, der in Figur 13 abgebildet ist. LPS von *E. coli* O55:B5 (3 µg/ml) wurde an PolySorp-Platten (Nunc) über Adsorption immobilisiert (18h bei Raumtemperatur in PBS-Puffer, 10 mM Na₇HPO₄, 1.8 mM KH₂PO₄, 137 mM NaCl, 2.7 mM KCl, pH 7.4). Anschliessend wurden die Mikrotiterplatten mit Casein (0.05 % in PBS, 1.5 h bei RT) blockiert und einmal mit PBS-Puffer gewaschen. Kontrollplatten wurden nicht mit Endotoxin inkubiert, sondern nur mit Casein blockiert. Jeweils 200 µl biotinmarkiertes Miro2p12 (Miro2p12-bio) in 50mM Tris, pH 8, 0.05% Casein, 0.05% Tween20 wurde in steigender Konzentration zugegeben (weiße Balken: Platten ohne ET, schwarze Balken: Platten mit ET, Protein-Konzentrationen wie angegeben). Nach 5 h Inkubation bei Raumtemperatur wurde 3-mal mit je 200 µl PBST (10 mM Na₇HPO₄, 1.8 mM KH₂PO₄, 137 mM NaCl, 2.7 mM KCl, 0.05 % Tween, pH 7.4) gewaschen. Der Nachweis von Endotoxin gebundenem biotin-markiertem Miro2p12 erfolgte über einen biotinspezifischen ELISA. Ein Konjugat aus Streptavidin mit alkalischer Phosphatase (Amersham Biosciences) wird 1:5000 in PBST verdünnt und je 200 µl davon 1.5 h bei Raumtemperatur mit gebundenem miro2p12-bio inkubiert. Danach wird 3-mal mit je 200 µl Tris-T (50 mM Tris, 0.05 % Tween, pH 8.0) gewaschen. Der colorimetrische Nachweis erfolgt durch Absorptionsmessung bei 405 nm nach Zugabe von pNPP (para-Nitrophenylphosphat) in einer Konzentration von 0.8 mg/ml als chromogenem Substrat. Biotinmarkiertes Miro2p12 bindet in konzentrationsabhängiger Form an die Mikrotiterplatten, die vorher mit Endotoxin beschichtet wurden.

Beispiel 11: Nachweis von FITC-markiertem Endotoxin gebunden an immobilisiertes Miro2p12. Miro2p12 (je 200 µl mit 5 µg/ml Protein) wurde an eine MaxiSorp-Platte (Nunc) adsorbiert (16 h bei RT in PBS, 10 mM Na₂HPO₄, 1.8 mM KH₂PO₄, 137 mM NaCl, 2.7 mM KCl, pH 7.4). Anschliessend wurden die Mikrotiterplatten mit Casein (0.05 % Casein in PBS, 1.5h bei RT, je 200µl pro Vertiefung) blockiert und einmal mit PBS-Puffer gewaschen. Kontrollplatten wurden nicht mit Miro2p12 inkubiert, sondern nur mit Casein blockiert. Jeweils 100 µl FITC-markiertes LPS von *E. coli* O55:B5 (Sigma) in PBS wurde in steigender Konzentration zugegeben (weiße Balken: Platten ohne Miro2p12, schwarze Balken: Platten mit Miro2p12). Es wurde 3-mal mit je 200 µl PBST (10 mM Na₂HPO₄, 1.8 mM KH₂PO₄, 137 mM NaCl, 2.7 mM KCl, 0.05 % Tween, pH 7.4) gewaschen. Der Versuch ist in Figur 14 dargestellt. Der Nachweis von an Bakteriophagenschwanzprotein gebundenem Endotoxin erfolgt in einem FITC-spezifischen ELISA. Anti-FITC-Antikörper (0.5 µg/ml; Zymed) wird 1:500 in PBST verdünnt und anschliessend werden je 200 µl 1 h bei Raumtemperatur mit dem gebundenen FITC-markierten Endotoxin inkubiert. Anschliessend wird 3-mal mit je 200 µl PBST gewaschen. Als sekundärer Antikörper wird ein anti-rabbit-IgG-alkalische Phosphatase Konjugat verwendet (1 µg/ml, Pierce). Es wird 1:5000 verdünnt eingesetzt und für 1.5 h bei Raumtemperatur inkubiert Anschliessend wird 3-mal mit je 200 µl PBST gewaschen. Die Quantifizierung erfolgte über Fluoreszenzmessung der Reaktionsprodukte eines fluoreszierenden alkalische Phosphatase Substrates (Methylumbelliferylphosphat; Sigma) mit 0.1 mg/ml Methylumbelliferylphosphat in 50 mM Tris, pH 8.0. Fluoreszenzmarkiertes Endotoxin bindet in konzentrationsabhängiger Form an die Mikrotiterplatten, die vorher mit Miro2p12 beschichtet wurden.

## Patentansprüche

1. Verfahren zur Entfernung von Endotoxin aus einer Probe, umfassend die Schritte:
a) Inkubation oder in Kontakt bringen einer Probe mit Bakteriophagenschwanzproteinen, die unspezifisch oder gerichtet, an einem festen Träger immobilisiert sind, wobei die Bakteriophagenschwanzproteine in der Lage sind, Endotoxin sowohl in Anwesenheit als auch in Abwesenheit von zweiwertigen positiven Ionen und unabhängig von der Konzentration an zweiwertigen positiven Ionen zu binden, und anschließend
b) Trennen des Bakteriophagenschwanzprotein-Endotoxin-Komplexes von der Probe.

2. Verfahren nach Anspruch 1, wobei die Schritte a) und b) in einem Chromatographiesäulen-Durchflussverfahren durchgeführt werden.

3. Verfahren nach Anspruch 1, wobei der feste Träger Cellulose, Filtrationsmedien, Glaspartikel, Magnetpartikel, Zentrifugations-, Sedimentationsmaterialien oder Füllmaterialien für Chromatographiesäulen sind.

4. Verfahren nach einem der vorhergehenden Ansprüche, wobei die Bakteriophagenschwanzproteine über Kopplungsgruppen an dem festen Träger immobilisiert sind.

5. Verfahren nach einem der vorhergehenden Ansprüche, wobei die Bakteriophagenschwanzproteine kovalent über chemische Bindungen an dem festen Träger immobilisiert sind.

6. Verfahren nach einem der vorhergehenden Ansprüche, wobei das Bakteriophagenschwanzprotein einen Strep-Tag oder einen His-Tag aufweist.

7. Verfahren nach einem der vorhergehenden Ansprüche, wobei das Bakteriophagenschwanzprotein ein kurzes Bakteriophagenschwanzprotein ist.

8. Verfahren nach einem der vorhergehenden Ansprüche, wobei das Bakteriophagenschwanzprotein aus der Familie der Myoviridae stammt.

9. Verfahren nach einem der vorhergehenden Ansprüche, wobei das Bakteriophagenschwanzprotein aus der Gruppe der Pseudo-T-even, Schizo-T-Even oder der T-Even Phagen stammt.

10. Verfahren nach einem der vorhergehenden Ansprüche, wobei das Bakteriophagenschwanzprotein in der Lage ist, an die 2-Keto-3-desoxyoctonsäure in der Herz-Region der Endotoxine zu binden.

11. Verfahren nach einem der vorhergehenden Ansprüche, wobei das Bakteriophagenschwanzprotein ausgewählt ist aus SEQ ID NO: 2, 4, 6, 8, 10, 12 und 14.

12. Verfahren nach einem der vorhergehenden Ansprüche, wobei die Konzentration an freien nicht gebundenen zweiwertigen positiven Ionen kleiner als 0,1 µM ist.

13. Verfahren nach einem der vorhergehenden Ansprüche, wobei die zweiwertigen positiven Ionen Ca²⁺ und/oder Mg²⁺ sind.

14. Polypeptid gemäß SEQ ID NO: 2, 4, 6, 8, 10, 12 oder 14.

15. Nukleinsäuremolekül mit einer Sequenz gemäß SEQ ID NO: 1, 3, 5, 7, 9, 11 oder 13.

16. Verfahren zum Nachweis von Endotoxin, umfassend die Schritte:
a) In Kontakt bringen einer Probe enthaltend Endotoxine mit einer Oberfläche, anschließend
b) Inkubieren von Bakteriophagenschwanzproteinen an die auf der Oberfläche immobilisierten Endotoxine, wobei die Bakteriophagenschwanzproteine in der Lage sind, sowohl in Anwesenheit als auch in Abwesenheit von zweiwertigen positiven Ionen und unabhängig von der Konzentration an zweiwertigen positiven Ionen zu binden, und anschließend
c) Nachweis der an Endotoxin gebundenen Bakteriophagenschwanzproteine.

17. Verfahren nach Anspruch 16, ferner umfassend nach Schritt a) und vor Schritt b) den zusätzlichen Schritt
a') Entfernen der Probe.

18. Verfahren nach Anspruch 16 oder 17, ferner umfassend nach Schritt b) und vor Schritt c) den zusätzlichen Schritt
b') Entfernen der ungebundenen Bakteriophagenschwanzproteine.

19. Verfahren zum Nachweis von Endotoxin, umfassend die Schritte:
a) In Kontakt bringen einer Probe enthaltend Endotoxine mit einer Oberfläche, anschließend
b) Inkubieren von Bakteriophagenschwanzproteinen an die auf der Oberfläche immobilisierten Endotoxine, wobei die Bakteriophagenschwanzproteine in der Lage sind, Endotoxin sowohl in Anwesenheit als auch in Abwesenheit von zweiwertigen positiven Ionen und unabhängig von der Konzentration an zweiwertigen positiven Ionen zu binden, und anschließend
c) Entfernen der ungebundenen Bakteriophagenschwanzproteine
d) Nachweis der in Schritt c) erhaltenen ungebundenen Bakteriophagenschwanzproteine.

20. Verfahren nach Anspruch 19, ferner umfassend nach Schritt a) und vor Schritt b) den zusätzlichen Schritt
a') Entfernen der Probe von der Oberfläche.

21. Verfahren nach einem der Ansprüche 16 bis 20, wobei die Oberfläche mit einem Endotoxin-bindenden Liganden mittels Adsorption oder kovalenter Kopplung beschichtet ist.

22. Verfahren nach Anspruch 21, wobei der Endotoxin-bindenden Ligand ein Bakteriophagenschwanzprotein ist, das in der Lage ist, Endotoxin sowohl in Anwesenheit als auch in Abwesenheit von zweiwertigen positiven Ionen zu binden.

23. Verfahren zum Nachweis von Endotoxin, umfassend folgende Schritte:
a) In Kontakt bringen einer Probe enthaltend Endotoxine mit einer Oberfläche, an die erste Bakteriophagenschwanzproteine immobilisiert sind, die in der Lage sind, Endotoxin sowohl in Anwesenheit als auch in Abwesenheit von zweiwertigen positiven Ionen und unabhängig von der Konzentration an zweiwertigen positiven Ionen zu binden, und anschließend gegebenenfalls
b) Entfernen der Probe von der Oberfläche, anschließend
c) Inkubieren von zweiten Bakteriophagenschwanzproteinen an die an die ersten Bakteriophagenschwanzproteine gebundenen Endotoxine, wobei die zweiten Bakteriophagenschwanzproteine Endotoxine nur in Gegenwart von zweiwertigen positiven Ionen binden, und anschließend gegebenenfalls
d) Entfernen der ungebundenen zweiten Bakteriophagenschwanzproteine, und anschließend
e) Nachweis der an Endotoxin gebundenen zweiten Bakteriophagenschwanzproteine.

24. Verfahren zum Nachweis von Endotoxin, umfassend folgende Schritte:
a) In Kontakt bringen einer Probe enthaltend Endotoxine mit einer Oberfläche, an die Bakteriophagenschwanzproteine immobilisiert sind, die in der Lage sind, Endotoxin sowohl in Anwesenheit als auch in Abwesenheit von zweiwertigen positiven Ionen und unabhängig von der Konzentration an zweiwertigen positiven Ionen zu binden, und anschließend gegebenenfalls a. Entfernen der Probe von der Oberfläche, anschließend
f) Nachweis der in Schritt a) gebundenen Endotoxine.

25. Verfahren nach einem der Ansprüche 16 bis 24, wobei der Nachweis mittels spektroskopischer Verfahren, ELISA, chemischer oder enzymatischer Nachweisreaktion von Endotoxinen oder abgespaltenen Endotoxinkomponenten oder mittels Kapazitätsmessung durchgeführt wird.

26. Verfahren zum Nachweis von Endotoxin, umfassend die Schritte:
a) Mischen der Probe mit Endotoxinen, die mit einem Marker gekoppelt sind, anschließend
b) Auftragen des Gemisches aus Schritt a) auf eine Oberfläche mit immobilisierten Bakteriophagenschwanzproteinen, wobei die Bakteriophagenschwanzproteine in der Lage sind, Endotoxin sowohl in Anwesenheit als auch in Abwesenheit von zweiwertigen positiven Ionen und unabhängig von der Konzentration an zweiwertigen positiven Ionen zu binden, anschließend
c) Abnehmen des Gemisches von der Oberfläche, anschließend
d) Waschen der Oberfläche, und anschließend
e) Nachweis der markierten Endotoxine auf der Oberfläche und/oder des freien markierten Endotoxins der vereinigten Proben nach Schritt c) und d).

27. Verfahren nach einem der Ansprüche 16 bis 26, wobei die Bakteriophagenschwanzproteine über Kopplungsgruppen an dem festen Träger immobilisiert sind.

28. Verfahren nach einem der Ansprüche 16 bis 27, wobei die Bakteriophagenschwanzproteine kovalent über chemische Bindungen an dem festen Träger immobilisiert sind.

29. Verfahren nach einem der Ansprüche 16 bis 28, wobei das Bakteriophagenschwanzprotein einen Strep-Tag oder einen His-Tag aufweist.

30. Verfahren nach einem der Ansprüche 16 bis 29, wobei das Bakteriophagenschwanzprotein ein kurzes Bakteriophagenschwanzprotein ist.

31. Verfahren nach einem der Ansprüche 16 bis 30, wobei das Bakteriophagenschwanzprotein aus der Familie der Myoviridae stammt.

32. Verfahren nach einem der Ansprüche 16 bis 31, wobei das Bakteriophagenschwanzprotein aus der Gruppe der Pseudo-T-even, Schizo-T-Even oder der T-Even Phagen stammt.

33. Verfahren nach einem der Ansprüche 16 bis 32, wobei das Bakteriophagenschwanzprotein in der Lage ist, an die 2-Keto-3-desoxyoctonsäure in der Herz-Region der Endotoxine zu binden.

34. Verfahren nach einem der Ansprüche 16 bis 33, wobei das Bakteriophagenschwanzprotein ausgewählt ist aus SEQ ID NO: 2, 4, 6, 8, 10, 12 und 14.

## Claims

1. Method for removing endotoxin from a sample, comprising the steps:
a) incubating or contacting bacteriophage tail proteins to a sample unspecifically or directedly immobilized to a solid carrier, wherein the bacteriophage tail proteins are able to bind endotoxin both in presence and in absence of bivalent positive ions and independently of the bivalent positive ion concentrations, and subsequently
b) separating the bacteriophage tail proteins-endotoxin complex from the sample.

2. Method according to claim 1, wherein the steps a) and b) are carried out in a column chromatography flow-through method.

3. Method according to claim 1, wherein the solid carrier is cellulose, filtration media, glass particles, magnetic particles, centrifugation or sedimentation substances or packing material for chromatography columns.

4. Method according to anyone of the preceding claims, wherein the bacteriophage tail proteins are immobilized to the solid carrier by coupling groups.

5. Method according to anyone of the preceding claims, wherein the bacteriophage tail proteins are covalent immobilized to the solid carrier by chemical bonds.

6. Method according to anyone of the preceding claims, wherein the bacteriophage tail protein comprises a Strep-tag or a His-tag.

7. Method according to anyone of the preceding claims, wherein the bacteriophage tail protein is a short bacteriophage tail protein.

8. Method according to anyone of the preceding claims, wherein the bacteriophage tail protein derives from the myoviridae family.

9. Method according to anyone of the preceding claims, wherein the bacteriophage tail protein is selected from the group consisting of pseudo-T-even, schizo-T-even or T-even phage.

10. Method according to anyone of the preceding claims, wherein the bacteriophage tail protein is able to bind the 2-keto-3-deoxyoctonic acid in the core region of the endotoxins.

11. Method according to anyone of the preceding claims, wherein the bacteriophage tail protein is selected from SEQ ID NO: 2,4, 6, 8, 10, 12 and 14.

12. Method according to anyone of the preceding claims, wherein the concentration of free unbound bivalent positive ions is less than 0.1 µM,

13. Method according to anyone of the preceding claims, wherein the bivalent positive ions are Ca²⁺ and/or Mg²⁺.

14. Polypeptide according to SEQ ID NO: 2, 4, 6, 8, 10, 12 or 14.

15. Nucleic acid molecule having a sequence according SEQ ID NO: 1, 3, 5, 7, 9, 11 or 13.

16. Method for the detection of endotoxin, comprising the steps:
a) contacting an endotoxin containing sample to a surface, subsequently
b) incubating of bacteriophage tail proteins to endotoxins immobilized to the surface, wherein the bacteriophage tail proteins are able to bind endotoxin both in presence and in absence of bivalent positive ions and independently of the bivalent positive ion concentration, and subsequently
c) detection of the bacteriophage tail proteins bound to endotoxin.

17. Method according to claim 16, further comprising the additional step after step a) and before step b):
a') removing the sample.

18. Method according to claim 16 or 17, further comprising the additional step after step b) and before step c):
b') removing the unbound bacteriophage tail proteins.

19. Method for the detection of endotoxin, comprising the steps:
a) contacting an endotoxin containing sample to a surface, subsequently
b) incubating of bacteriophage tail proteins to endotoxins immobilized to the surface, wherein the bacteriophage tail proteins are able to bind endotoxin both in presence and in absence of bivalent positive ions and independently of the bivalent positive ion concentration, and subsequently
c) removing the unbound bacteriophage tail proteins
d) detection of the unbound bacteriophage tail proteins obtained in step c).

20. Method according to claim 19, further comprising the additional step after step a) und before step b)
a') removing the sample from the surface.

21. Method according to anyone of the claims 16 to 20, wherein the surface is coated with an endotoxin binding ligand by adsorption or covalent coupling.

22. Method according to claim 21, wherein the endotoxin binding ligand is a bacteriophage tail protein, which is able to bind endotoxin both in presence and in absence of bivalent positive ions.

23. Method for the detection of endotoxin, comprising the steps:
a) contacting an endotoxins containing sample to a surface immobilized with first bacteriophage tail proteins, which are able to bind endotoxin both in presence and in absence of bivalent positive ions and independently of the bivalent positive ion concentration, and subsequently optionally
b) removing the sample from the surface, subsequently
c) incubating of second bacteriophage tail proteins to endotoxins bound to the first bacteriophage tail proteins, wherein the second bacteriophage tail proteins bind endotoxins only in the presence of bivalent positive ions, and subsequently optionally
d) removing the unbound second bacteriophage tail proteins, and subsequently
e) detection of the second bacteriophage tail proteins bound to endotoxin.

24. Method for the detection of endotoxin, comprising the steps:
a) contacting an endotoxins containing sample to a surface immobilized with bacteriophage tail proteins, which are able to bind endotoxin both in presence and in absence of bivalent positive ions and independently of the bivalent positive ion concentration, and subsequently optionally
b) removing the sample from the surface, and subsequently
c) detection of the endotoxins bound in step a).

25. Method according to anyone of the claims 16 to 24, wherein the detection is carried out by spectroscopic methods, ELISA, chemical or enzymatic detection reaction of endotoxins or separated endotoxin components or by capacity measurement.

26. Method for the detection of endotoxin, comprising the steps:
a) mixing the sample with endotoxins coupled to a marker, subsequently
b) applying the mixture of step a) onto a surface with immobilized bacteriophage tail proteins, wherein the bacteriophage tail proteins are able to bind endotoxin both in presence and in absence of bivalent positive ions and independently of the bivalent positive ion concentration, subsequently
c) removing the mixture from the surface, subsequently
d) washing the surface, and subsequently
e) detection of the labeled endotoxins on the surface and/or the free labeled endotoxins of the pooled samples after step c) and d).

27. Method according to anyone of claims 16 to 26, wherein the bacteriophage tail proteins are immobilized to the solid carrier by coupling groups.

28. Method according to anyone of claims 16 to 27, wherein the bacteriophage tail proteins are covalent immobilized to the solid carrier by chemical bonds.

29. Method according to anyone of claims 16 to 28, wherein the bacteriophage tail protein comprises a Strep-tag or a His-tag.

30. Method according to anyone of claims 16 to 29, wherein the bacteriophage tail protein is a short bacteriophage tail protein.

31. Method according to anyone of claims 16 to 30, wherein the bacteriophage tail protein derives from the myoviridae family.

32. Method according to anyone of claims 16 to 31, wherein the bacteriophage tail protein is selected from the group consisting of pscudo-T-even, schizo-T-even or T-even phages.

33. Method according to anyone of claims 16 to 32, wherein the bacteriophage tail protein is able to bind the 2-keto-3-deoxyoctonic acid in the core region of endotoxins.

34. Method according to anyone of claims 16 to 33, wherein the bacteriophage tail protein is selected from SEQ ID NO: 2, 4, 6, 8, 10, 12 and 14.

## Revendications

1. Procédé pour l'élimination d'endotoxine d'un échantillon, comprenant les étapes de:
a) incuber ou mettre en contact un échantillon avec des protéines de queue de bactériophage qui sont immobilisées de manière non spécifique ou dirigée sur un support solide, les protéines de queue de bactériophage étant capables de lier l'endotoxine en présence comme en l'absence d'ions positifs bivalents et indépendamment de la concentration en ions positifs bivalents, puis
b) séparer les complexes protéine de queue de bactériophage - endotoxine de l'échantillon.

2. Procédé selon la revendication 1, dans lequel les étapes a) et b) sont réalisées dans un procédé de passage sur colonne de chromatographie.

3. Procédé selon la revendication 1, dans lequel le support solide est de la cellulose, des milieux de filtration, des particules de verre, des particules magnétiques, des matériaux de centrifugation, de sédimentation ou des matériaux de remplissage pour colonne de chromatographie.

4. Procédé selon l'une des revendications précédentes, dans lequel les protéines de queue de bactériophage sont immobilisées sur le support solide par des groupes de couplage.

5. Procédé selon l'une des revendications précédentes, dans lequel les protéines de queue de bactériophage sont immobilisées sur le support solide par covalence via des liaisons chimiques.

6. Procédé selon l'une des revendications précédentes, dans lequel la protéine de queue de bactériophage présente une étiquette Strep ou une étiquette His.

7. Procédé selon l'une des revendications précédentes, dans lequel la protéine de queue de bactériophage est une protéine courte de queue de bactériophage.

8. Procédé selon l'une des revendications précédentes, dans lequel la protéine de queue de bactériophage vient de la famille des myoviridae.

9. Procédé selon l'une des revendications précédentes, dans lequel la protéine de queue de bactériophage vient du groupe des phages Pseudo-T-pairs, Schizo-T-pairs ou T-pairs.

10. Procédé selon l'une des revendications précédentes, dans lequel la protéine de queue de bactériophage est capable de se lier à l'acide 2-céto-3-désoxyoctanoïque dans la région centrale des endotoxines.

11. Procédé selon l'une des revendications précédentes, dans lequel la protéine de queue de bactériophage est choisie parmi SEQ ID NO 2, 4, 6, 8, 10, 12 et 14.

12. Procédé selon l'une des revendications précédentes, dans lequel la concentration en ions positifs bivalents libres non liés est inférieure à 0,1 µM.

13. Procédé selon l'une des revendications précédentes, dans lequel les ions positifs bivalents sont Ca²⁺ et/ou Mg²⁺.

14. Polypeptide selon SEQ ID NO 2, 4, 6, 8, 10, 12 ou 14.

15. Molécule d'acide nucléique ayant une séquence selon SEQ ID NO 1, 3, 5, 7, 9, 11 ou 13.

16. Procédé pour la détection d'endotoxine, comprenant les étapes de :
a) mettre en contact un échantillon contenant des endotoxines avec une surface, ensuite
b) incuber des protéines de queue de bactériophage avec les endotoxines immobilisées sur la surface, les protéines de queue de bactériophage étant capables de lier l'endotoxine en présence comme en l'absence d'ions positifs bivalents et indépendamment de la concentration en ions positifs bivalents, et ensuite
c) détecter les protéines de queue de bactériophage liées à l'endotoxine.

17. Procédé selon la revendication 16, comprenant en outre après l'étape a) et avant l'étape b), l'étape supplémentaire de :
a') éliminer l'échantillon.

18. Procédé selon la revendication 16 ou 17, comprenant en outre après l'étape b) et avant l'étape c) l'étape supplémentaire de
b') éliminer les protéines de queue de bactériophage non liées.

19. Procédé de détection d'endotoxine, comprenant les étapes de :
a) mettre en contact un échantillon contenant des endotoxines avec une surface, ensuite
b) incuber les protéines de queue de bactériophage avec les endotoxines immobilisées sur la surface, les protéines de queue de bactériophage étant capables de lier l'endotoxine en présence comme en l'absence d'ions positifs bivalents et indépendamment de la concentration en ions positifs bivalents, et ensuite
c) éliminer les protéines de queue de bactériophage non liées
d) détecter les protéines de queue de bactériophage non liées conservées à l'étape c).

20. Procédé selon la revendication 19, comprenant en outre après l'étape a) et avant l'étape b) l'étape supplémentaire de
a') éliminer l'échantillon de la surface.

21. Procédé selon l'une des revendications 16 à 20, dans lequel la surface est couverte d'un ligand se liant à l'endotoxine par adsorption ou couplage covalent.

22. Procédé selon la revendication 21, dans lequel le ligand se liant à l'endotoxine est une protéine de queue de bactériophage qui est capable de lier l'endotoxine en présence comme en l'absence d'ions positifs bivalents.

23. Procédé pour la détection d'endotoxine, comprenant les étapes suivantes :
a) mettre en contact un échantillon contenant des endotoxines avec une surface sur laquelle des premières protéines de queue de bactériophage sont immobilisées, qui sont capables de lier l'endotoxine en présence comme en l'absence d'ions positifs bivalents et indépendamment de la concentration en ions positifs bivalents, et ensuite le cas échéant
b) éliminer l'échantillon de la surface, ensuite
c) incuber des deuxièmes protéines de queue de bactériophage avec les endotoxines liées aux premières protéines de queue de bactériophage, les deuxièmes protéines de queue de bactériophage ne liant les endotoxines qu'en présence d'ions positifs bivalents et ensuite le cas échéant
d) éliminer les deuxièmes protéines de queue de bactériophage non liées et ensuite
e) détecter les deuxièmes protéines de queue de bactériophage liées à l'endotoxine.

24. Procédé de détection d'endotoxine, comprenant les étapes suivantes :
a) mettre en contact un échantillon contenant des endotoxines avec une surface sur laquelle sont immobilisées des protéines de queue de bactériophage qui sont capables de lier l'endotoxine en présence comme en l'absence d'ions positifs bivalents et indépendamment de la concentration en ions positifs bivalents, et ensuite le cas échéant
a. éliminer l'échantillon de la surface, et ensuite
f) détecter les endotoxines liées à l'étape a).

25. Procédé selon l'une des revendications 16 à 24, dans lequel la détection est réalisée par des procédés spectroscopiques, par ELISA, par une réaction de détection chimique ou enzymatique des endotoxines ou des composants d'endotoxine séparés ou par mesure de capacité.

26. Procédé de détection d'endotoxine, comprenant les étapes de :
a) mélanger l'échantillon avec des endotoxines qui sont couplées à un marqueur, ensuite
b) appliquer le mélange de l'étape a) sur une surface avec des protéines de queue de bactériophage immobilisées, les protéines de queue de bactériophage étant capables de lier l'endotoxine en présence comme en l'absence d'ions positifs bivalents et indépendamment de la concentration en ions positifs bivalents, ensuite
c) retirer le mélange de la surface, ensuite
d) laver la surface et ensuite
e) détecter les endotoxines marquées sur la surface et/ou l'endotoxine marquée libre de l'échantillon réuni après l'étape c) et l'étape d).

27. Procédé selon l'une des revendications 16 à 26, dans lequel les protéines de queue de bactériophage sont immobilisées par des groupes de couplage sur le support solide.

28. Procédé selon l'une des revendications 16 à 27, dans lequel les protéines de queue de bactériophage sont immobilisées par covalence via des liaisons chimiques sur le support solide.

29. Procédé selon l'une des revendications 16 à 28, dans lequel la protéine de queue de bactériophage présente une étiquette Strep ou une étiquette His.

30. Procédé selon l'une des revendications 16 à 29, dans lequel la protéine de queue de bactériophage est une protéine courte de queue de bactériophage.

31. Procédé selon l'une des revendications 16 à 30, dans lequel la protéine de queue de bactériophage vient de la famille des myoviridae.

32. Procédé selon l'une des revendications 16 à 31, dans lequel la protéine de queue de bactériophage vient du groupe des phages Pseudo-T-pairs, Schizo-T-pairs ou T-pairs.

33. Procédé selon l'une des revendications 16 à 32, dans lequel la protéine de queue de bactériophage est capable de se lier à l'acide 2-céto3-désoxyoctanoïque dans la région centrale des endotoxines.

34. Procédé selon l'une des revendications 16 à 33, dans lequel la protéine de queue de bactériophage est choisie parmi SEQ ID NO 2, 4, 6, 8, 10, 12 et 14.


### SEQUENCE LISTING

<110> PROFOS AG
<120> Verfahren zum Nachweis und zur Entfernung von Endotoxin
<130>- PRO-017 PCT
<140> unknown
   <141> 2006-01-23
<150> DE 10 2005 002 969.8
   <151> 2005-01-21
<160> 20
<170> PatentIn version 3.3
<210> 1
   <211> 54
   <212> DNA
   <213> bacteriophage Mirol
<400> 1
   acgcgcaaag cttgtcgacg gatcctatca ttcttttacc ttaattatgt agtt 54
<210> 2
   <211> 457
   <212> PRT
   <213> Bacteriophage Mirol
<400> 2
   Met Leu Glu Pro Pro Ala Val Glu Lys Gly Ala Ala Gln Asn Asn Tyr
   1               5                   10                  15
   Asn His Tyr Ser Asp Leu Ala Lys Tyr Thr Ile Phe Asp Pro Thr Asn
               20                  25                  30
   Thr Gln Trp Pro Val Ala Ile Lys Asp Val Gln Ser Ala Leu Glu Leu
           35                  40                  45
   Ile Gly Ser Trp Ala Arg Thr Asp Thr Gly Leu Pro Val Ala Ser Pro
       50                  55                  60
   Thr Val Ala Gly Val Ile Arg Thr Ala Thr Gln Ala Glu Val Asp Ala
   65                  70                  75                  80
   Gly Thr Ile Gly Asn Ala Ala Val Thr Pro Ala Thr Leu Lys Ser Thr
                   85                  90                  95
   Val Thr Arg Pro Glu Ala Thr Thr Ala Val Leu Gly Leu Thr Arg Tyr
               100                 105                 110
   Ala Thr Asn Thr Glu Ala Ala Ala Leu Thr Ala Gly Asn Arg Thr Ile
           115                 120                 125
   Thr Ala Ala Ala Leu Gly His Val Phe Lys Thr Val Lys Ala Gln Glu
       130                 135                 140
   Asn Val Asp Gly Thr Val Arg Leu Thr Thr Ala Ala Gln Ala Gln Ala
   145                 150                 155                 160
   Gly Thr Asp Glu Thr Ala Val Thr Pro Lys Arg Val Val Glu Met Ile
                   165                 170                 175
   Gly Lys Phe Ser Val Ser Pro Pro Ser Tyr Thr Ser Ala Thr Glu Ser
               180                 185                 190
   Asn Leu Gly Leu Val Arg Val Ala Thr Gln Ala Gln Val Ala Ala Gly
           195                 200                 205
   Ala Val His Asp Gly Tyr Ala Val Thr Pro Lys Thr Phe Met Ala Ser
       210                 215                 220
   Lys Ala Ser Asp Ser Val Phe Gly Ile Val Lys Phe Ala Lys Asp Ser
   225                 230                 235                 240
   Asp Val Ala Ser Ala Thr Ser Asn Asn Leu Ala Val Thr Pro Lys Ser
                   245                 250                 255
   Leu Gln Ala Leu Lys Ser Thr Lys Asp Lys Tyr Gly Leu Thr Arg Leu
               260                 265                 270
   Ser Gly Ser Pro Thr Thr Asp Ala Ser Leu Ala Ala Ala Ala Thr Asp
           275                 280                 285
   Ala Val Phe Lys Thr Arg Lys Ile Asn Gly Lys Thr Leu Asp Asn Asp
       290                 295                 300
   Ile Thr Ile Thr Asn Asn Asp Ile Asn Cys Tyr Thr Arg Gln Glu Ser
   305                 310                 315                 320
   Asp Gly Arg Tyr Met Pro Ala Gly Thr Arg Val Gly Asn Val Thr Trp
                   325                 330                 335
   Val Glu Gly Gln Ser Trp Ile Ser Arg Gly Ala Thr Phe Thr Cys Asn
               340                 345                 350
   Ala Pro Trp Glu Ala Ser Ser Arg Leu Ala Leu Asn Val Asn Val Lys
           355                 360                 365
   Phe Glu Arg Asn Asn Asp Gly Tyr Asp Asn Arg Ile Phe Arg Phe Val
       370                 375                 380
   Val Ile Val Asn Gly Ser Gln Trp Gly Gly Glu Leu Thr Leu Asn Ile
   385                 390                 395                 400
   Glu Asn Thr Lys Gly Gly Arg Asn Gly His Ser Trp Arg Phe Glu Ala
                   405                 410                 415
   Tyr Ala Ser Ser Asn Phe Phe Phe Asn Asn Ile Pro Pro Asn Ala Thr
               420                 425                 430
   Val Gln Ile Arg Pro Thr Glu Asp Ser Arg Ile Ile Phe Tyr Asp Cys
           435                 440                 445
   Met Leu Thr Phe Cys Thr Asn Arg Pro
       450                 455
<210> 3
   <211> 1347
   <212> DNA
   <213> Bacteriophage Miro2
<400> 3
   atggcccaga ataactataa tcactacagt gatttggcca aatatacgat atttgaccca      60
   acaaatacac aatggcctgt tgctataaaa gacgtccaat cagcgttaga gctgattggt     120
   agctgggcaa gaactgatac cggattacct gtagcatctc ctacagtagc cggtgtaatt     180
   cgcacagcaa cacaggctga agttgatgct ggaactattg gtaatgctgc ggtaactcct     240
   gctacattaa aatccacagt tacccgtcct gaagcaacta cagcagttct tggcttaaca     300
   cggtatgcta ctaatactga agccgcagca ttaaccgcag gaaatagaac tattaccgcg     360
   gcggctctcg gtcatgtgtt taaaactgtg aaagcccaag aaaacgtaga tggaactgtt     420
   aggttaacta ctgcggctca agcacaagca ggaactgacg aaactaccgc agtaactcct     480
   aagcgtgttg tagaaatgat tggaaagttc agtgtcagtc ctcctagtta tacctctgcg     540
   acagaaagca acttgggatt agttcgtgtc gcaacccaag cccaggtagc agcaggtgct     600
   gttcacgatg gatacgcagt aactccaaaa accttcatgg catcaaaagc gtctgacagc     660
   gtatttggta tagtaaaatt tgctaaagac tcagatgtgg cttcagctac ttctaacaat     720
   ttggctgtta ctccaaaaag tcttcaagcg ctaaaatcca ccaaggataa atatggatta     780
   accagattat caggttctcc aactactgat gcttcattgg cagcggctgc aacagatgca     840
   gtctttaaaa cccgtagaat aaacggaaaa actcttgata atgacataac aattactaat     900
   aatgatatta attgttatac aagacaagaa tctgacgggc gttacatgcc agctggaacc     960
   agagtaggta atgttacttg ggttgaagga caatcttgga ttagtcgagg tgcaacgttt    1020
   acatgtaatg caccatggga agcttctagt agattagctc taaacgttaa tgtaaaattt    1080
   gagcgtaaca acgacggata tgacaatcgt attttcagat ttgttgtaat agttaacggt    1140
   tcccaatggg gaggtgaact tactcttaac atcgaaaata ctaaaggcgg acgaaatggt    1200
   cattcatgga gatttgaagc ttacgcatct agcaactttt tcttcaataa cattcctcca    1260
   aatgccactg ttcaaataag accaacagaa gacagtcgta ttatatttta tgactgcatg    1320
   cttacattct gtacaaatag accgtaa                                        1347
<210> 4
   <211> 448
   <212> PRT
   <213> Bacteriophage Miro2
<400> 4
   Met Ala Gln Asn Asn Tyr Asn His Tyr Ser Asp Leu Ala Lys Tyr Thr
   1 5 10 15
   Ile Phe Asp Pro Thr Asn Thr Gln Trp Pro Val Ala Ile Lys Asp Val
   20 25 30
   Gln Ser Ala Leu Glu Leu Ile Gly Ser Trp Ala Arg Thr Asp Thr Gly
   35 40 45
   Leu Pro Val Ala Ser Pro Thr Val Ala Gly Val Ile Arg Thr Ala Thr
   50 55 60
   Gln Ala Glu Val Asp Ala Gly Thr Ile Gly Asn Ala Ala Val Thr Pro
   65 70 75 80
   Ala Thr Leu Lys Ser Thr Val Thr Arg Pro Glu Ala Thr Thr Ala Val
   85 90 95
   Leu Gly Leu Thr Arg Tyr Ala Thr Asn Thr Glu Ala Ala Ala Leu Thr
   100 105 110
   Ala Gly Asn Arg Thr Ile Thr Ala Ala Ala Leu Gly His Val Phe Lys
   115 120 125
   Thr Val Lys Ala Gln Glu Asn Val Asp Gly Thr Val Arg Leu Thr Thr
   130 135 140
   Ala Ala Gln Ala Gln Ala Gly Thr Asp Glu Thr Thr Ala Val Thr Pro
   145 150 155 160
   Lys Arg Val Val Glu Met Ile Gly Lys Phe Ser Val Ser Pro Pro Ser
   165 170 175
   Tyr Thr Ser Ala Thr Glu Ser Asn Leu Gly Leu Val Arg Val Ala Thr
   180 185 190
   Gln Ala Gln Val Ala Ala Gly Ala Val His Asp Gly Tyr Ala Val Thr
   195 200 205
   Pro Lys Thr Phe Met Ala Ser Lys Ala Ser Asp Ser Val Phe Gly Ile
   210 215 220
   Val Lys Phe Ala Lys Asp Ser Asp Val Ala Ser Ala Thr Ser Asn Asn
   225 230 235 240
   Leu Ala Val Thr Pro Lys Ser Leu Gln Ala Leu Lys Ser Thr Lys Asp
   245 250 255
   Lys Tyr Gly Leu Thr Arg Leu Ser Gly Ser Pro Thr Thr Asp Ala Ser
   260 265 270
   Leu Ala Ala Ala Ala Thr Asp Ala Val Phe Lys Thr Arg Arg Ile Asn
   275 280 285
   Gly Lys Thr Leu Asp Asn Asp Ile Thr Ile Thr Asn Asn Asp Ile Asn
   290 295 300
   Cys Tyr Thr Arg Gln Glu Ser Asp Gly Arg Tyr Met Pro Ala Gly Thr
   305 310 315 320
   Arg Val Gly Asn Val Thr Trp Val Glu Gly Gln Ser Trp Ile Ser Arg
   325 330 335
   Gly Ala Thr Phe Thr Cys Asn Ala Pro Trp Glu Ala Ser Ser Arg Leu
   340 345 350
   Ala Leu Asn Val Asn Val Lys Phe Glu Arg Asn Asn Asp Gly Tyr Asp
   355 360 365
   Asn Arg Ile Phe Arg Phe Val Val Ile Val Asn Gly Ser Gln Trp Gly
   370 375 380
   Gly Glu Leu Thr Leu Asn Ile Glu Asn Thr Lys Gly Gly Arg Asn Gly
   385 390 395 400
   His Ser Trp Arg Phe Glu Ala Tyr Ala Ser Ser Asn Phe Phe Phe Asn
   405 410 415
   Asn Ile Pro Pro Asn Ala Thr Val Gln Ile Arg Pro Thr Glu Asp Ser
   420 425 430
   Arg Ile Ile Phe Tyr Asp Cys Met Leu Thr Phe Cys Thr Asn Arg Pro
   435 440 445
<210> 5
   <211> 1386
   <212> DNA
   <213> Bacteriophage Effe04
<400> 5
   atgagtaaca atacaatcaa ccacgtaagt gataaatcca tttacgtgac atttgaccca     60
   acaggtactg attggcctga taccataacc aacgtacaag atgcgttgga aaaaataggt    120
   agttgggcgc gtactgatac tgggcttcct atcgcaacaa cttctgttcg tggtattgct    180
   cagatcgcaa ccgaagctga tattaacgct ggcacggata acactaagat tgttactccg    240
   aaactgttag cataccgtat gcagaaccct aaagcatcac aaaccgtatg gggttatacg     300
   aagtattcga ctgatgcgga atctacaacc gtaactaacg atgcatcgtc tattactccg    360
   cgatcgctga actatgtgtt caataaccgc aaaggtacag aatcggtttg gggttcttct     420
   aaaatcgcta ccactgcaca ggcggttgct ggtacagata acactgtaac tatgactccg    480
   cttaaagtca agcaagcgat cgcgtctctg gttcctgttc agtcaagtgc gaccgaaagc    540
   tcgcaaggtc tggtacaact ggcaacagtt gcacaggttc aggctggtac gatccgtgaa    600
   gggtatgcaa tttcacctta tacgtttatt cgtttaactg caactgaaag caacctgggc    660
   gttattcgta tcgcatcaca ggcagaagca aacgcaggta ctgatgacac caaagcgatt    720
   actgcgaaaa aattaatcaa tacccgtgca actggttccc agttcggtgt tgtcaaatta    780
   gcaacaactg ttggttatgt ggcaaacacc gcactttctt ctaatgctta tgtattgcct    840
   agcgatcgta gtgcggtaat taatggttct ctttatgaga atagcgcaat acataacaac    900
   aaatatcaga cgtggacaga tcttgattgg catttcccag taggtgctat tgtcatgact    960
   ggtttccaga ctgaccacgg tagtttgtat atttgtgatg gacgttcact gaataaaaat   1020
   aattacccgt tactgtttga gcgtataggt tatacatttg gtggtggcgg tgattggttt   1080
   aacattccag actgtcgagg cgttgcagta cgtggtcatg accgtgggcg tggactaaac   1140
   cctaatcgtg ggtatggtac atatgaagga gatatgttgg gatggcacga acacccatta   1200
   caacttatct accagaacgg cggtaacatt ccgaaatggc aagcagttta cgaactgaaa   1260
   agcgccgaga agaatgacca aagcgctcgc gtatttgatg cttctataac taaagctact   1320
   ggtgtgggcg gtgaagaaac ccgcatgaaa aatatcgcat taaactacgt aattcgcgta   1380
   ttataa                                                              1386
<210> 6
   <211> 475
   <212> PRT
   <213> Bacteriophage Effe04
<400> 6
   Met Ala Ser Ala Ser Trp Ser His Pro Gln Phe Glu Lys Gly Ala Ser
   1 5 10 15
   Asn Asn Thr Ile Asn His Val Ser Asp Lys Ser Ile Tyr Val Thr Phe
   20 25 30
   Asp Pro Thr Gly Thr Asp Trp Pro Asp Thr Ile Thr Asn Val Gln Asp
   35 40 45
   Ala Leu Glu Lys Ile Gly Ser Trp Ala Arg Thr Asp Thr Gly Leu Pro
   50 55 60
   Ile Ala Thr Thr Ser Val Arg Gly Ile Ala Gln Ile Ala Thr Glu Ala
   65 70 75 80
   Asp Ile Asn Ala Gly Thr Asp Asn Thr Lys Ile Val Thr Pro Lys Leu
   85 90 95
   Leu Ala Tyr Arg Met Gln Asn Pro Lys Ala Ser Gln Thr Val Trp Gly
   100 105 110
   Tyr Thr Lys Tyr Ser Thr Asp Ala Glu Ser Thr Thr Val Thr Asn Asp
   115 120 125
   Ala Ser Ser Ile Thr Pro Arg Ser Leu Asn Tyr Val Phe Asn Asn Arg
       130                 135                 140
   Lys Gly Thr Glu Ser Val Trp Gly Ser Ser Lys Ile Ala Thr Thr Ala
   145                 150                 155                 160
   Gln Ala Val Ala Gly Thr Asp Asn Thr Val Thr Met Thr Pro Leu Lys
                   165                 170                 175
   Val Lys Gln Ala Ile Ala Ser Leu Val Pro Val Gln Ser Ser Ala Thr
               180                 185                 190
   Glu Ser Ser Gln Gly Leu Val Gln Leu Ala Thr Val Ala Gln Val Gln
           195                 200                 205
   Ala Gly Thr Ile Arg Glu Gly Tyr Ala Ile Ser Pro Tyr Thr Phe Ile
       210                 215                 220
   Arg Leu Thr Ala Thr Glu Ser Asn Leu Gly Val Ile Arg Ile Ala Ser
   225                 230                 235                 240
   Gln Thr Glu Ala Asn Ala Gly Thr Asp Asp Thr Lys Ala Ile Thr Ala
                   245                 250                 255
   Lys Lys Leu Ile Asn Thr Arg Ala Thr Gly Ser Gln Phe Gly Val Val
               260                 265                 270
   Lys Leu Ala Thr Thr Val Gly Tyr Val Ala Asn Thr Ala Leu Ser Ser
           275                 280                 285
   Asn Ala Tyr Val Leu Pro Ser Asp Arg Ser Ala Val Ile Asn Gly Ser
       290                 295                 300
   Leu Tyr Glu Tyr Ser Ala Ile His Asn Asn Lys Tyr Gln Thr Trp Thr
   305                 310                 315                 320
   Asp Leu Asp Trp His Phe Pro Val Gly Ala Ile Val Met Thr Gly Phe
                   325                 330                 335
   Gln Thr Asp His Gly Ser Leu Tyr Ile Cys Asp Gly Arg Ser Leu Asn
               340                 345                 350
   Lys Asn Asn Tyr Pro Leu Leu Phe Glu Arg Ile Gly Tyr Thr Phe Gly
           355                 360                 365
   Gly Gly Gly Asp Trp Phe Asn Ile Pro Asp Cys Arg Gly Val Ala Val
       370                 375                 380
   Arg Gly His Asp Arg Gly Arg Gly Leu Asn Pro Asn Arg Gly Tyr Gly
   385                 390                 395                 400
   Thr Tyr Glu Gly Asp Met Leu Gly Trp His Glu His Pro Leu Gln Leu
                   405                 410                 415
   Ile Tyr Gln Asn Gly Gly Asn Ile Pro Lys Trp Gln Ala Val Tyr Glu
               420                 425                 430
   Leu Lys Ser Ala Glu Lys Asn Asp Gln Ser Ala Arg Val Phe Asp Ala
           435                 440                 445
   Ser Ile Thr Lys Ala Thr Gly Val Gly Gly Glu Glu Thr Arg Met Lys
       450                 455                 460
   Asn Ile Ala Leu Asn Tyr Val Ile Arg Val Leu
   465                 470                 475
<210> 7
   <211> 1383
   <212> DNA
   <213> Artificial Sequence
<220>
   <223> N-Strep Miro1p12
<400> 7
   atggctagct ggagccaccc gcagttcgaa aaaggcgccg cccagaataa ctataatcac      60
   tacagtgatt tggccaaata tacgatattt gacccaacaa atacacaatg gcctgttgct     120
   ataaaagacg tccaatcagc gttagagctg attggcagct gggcaagaac tgataccgga     180
   ttacccgtag catctcctac agtagccggt gtaattcgca cagcaacaca ggctgaagtt     240
   gatgctggaa ctattggtaa tgctgcggta actcctgcta cattaaaatc cacagttacc     300
   cgtcctgaag caactacagc agttcttggc ttaacacggt atgctactaa tactgaagct     360
   gcagcattaa ccgcaggaaa tagaactatt accgcggcgg ctctcggtca tgtgtttaaa     420
   actgtgaaag cccaagaaaa cgtagatgga actgttaggt taactactgc ggctcaagca     480
   caagcaggaa ctgacgaaac taccgcagta actcctaagc gtgttgtaga aatgattgga     540
   aagttcagcg ttagtcctcc tagttatacc tctgcaacag aaagcaactt gggattagtt     600
   cgtgtcgcaa cccaagccca ggtagcagca ggtgccgttc acgacggata cgcagtaact     660
   ccaaaaacct tcatggcatc aaaagcgtct gacagtgtat ttggtatagt aaaatttgct     720
   aaagactcag atgtggcttc agctacttct aacaatttgg ctgttactcc aaaaagtctt     780
   caagcgctaa aatccaccaa ggataaatat ggattaacca gattatcagg ttctccaact     840
   actgatgctt cactggcagc tgcggcaaca gatgctgttt ttaaaacccg taaaataaac     900
   ggaaaaactc ttgacaatga cataacgatt actaacaatg atattaattg ttatacaagg     960
   caagaatctg acgggcgtta catgccagct ggaactagag taggtaatgt tacttgggtt    1020
   gaaggacaat cttggattag tcgaggtgca acgtttacat gtaatgcacc atgggaagct    1080
   tctagtagat tagctctaaa tgttaatgta aaatttgagc gtaacaacga cggatatgac    1140
   aatcgtattt tcagatttgt tgtaatagtt aacggttccc aatggggcgg tgaacttact    1200
   cttaacatcg aaaatactaa aggcggacga aatggtcatt catggagatt tgaagcttac    1260
   gcatctagca actttttctt caataacatt cctccaaatg ccactgttca aataagacca    1320
   acagaagaca gtcgtattat attttatgac tgcatgctta cattctgtac aaatagaccg    1380
   taa                                                                  1383
<210> 8
   <211> 460
   <212> PRT
   <213> Artificial Sequence
<220>
   <223> N-Strep Miro1p12
<400> 8
   Met Ala Ser Trp Ser His Pro Gln Phe Glu Lys Gly Ala Ala Gln Asn
   1 5 10 15
   Asn Tyr Asn His Tyr Ser Asp Leu Ala Lys Tyr Thr Ile Phe Asp Pro
   20 25 30
   Thr Asn Thr Gln Trp Pro Val Ala Ile Lys Asp Val Gln Ser Ala Leu
   35 40 45
   Glu Leu Ile Gly Ser Trp Ala Arg Thr Asp Thr Gly Leu Pro Val Ala
   50 55 60
   Ser Pro Thr Val Ala Gly Val Ile Arg Thr Ala Thr Gln Ala Glu Val
   65 70 75 80
   Asp Ala Gly Thr Ile Gly Asn Ala Ala Val Thr Pro Ala Thr Leu Lys
   85 90 95
   Ser Thr Val Thr Arg Pro Glu Ala Thr Thr Ala Val Leu Gly Leu Thr
   100 105 110
   Arg Tyr Ala Thr Asn Thr Glu Ala Ala Ala Leu Thr Ala Gly Asn Arg
   115 120 125
   Thr Ile Thr Ala Ala Ala Leu Gly His Val Phe Lys Thr Val Lys Ala
   130 135 140
   Gln Glu Asn Val Asp Gly Thr Val Arg Leu Thr Thr Ala Ala Gln Ala
   145 150 155 160
   Gln Ala Gly Thr Asp Glu Thr Thr Ala Val Thr Pro Lys Arg Val Val
   165 170 175
   Glu Met Ile Gly Lys Phe Ser Val Ser Pro Pro Ser Tyr Thr Ser Ala
   180 185 190
   Thr Glu Ser Asn Leu Gly Leu Val Arg Val Ala Thr Gln Ala Gln Val
   195 200 205
   Ala Ala Gly Ala Val His Asp Gly Tyr Ala Val Thr Pro Lys Thr Phe
   210 215 220
   Met Ala Ser Lys Ala Ser Asp Ser Val Phe Gly Ile Val Lys Phe Ala
   225 230 235 240
   Lys Asp Ser Asp Val Ala Ser Ala Thr Ser Asn Asn Leu Ala Val Thr
   245 250 255
   Pro Lys Ser Leu Gln Ala Leu Lys Ser Thr Lys Asp Lys Tyr Gly Leu
   260 265 270
   Thr Arg Leu Ser Gly Ser Pro Thr Thr Asp Ala Ser Leu Ala Ala Ala
   275 280 285
   Ala Thr Asp Ala Val Phe Lys Thr Arg Lys Ile Asn Gly Lys Thr Leu
   290 295 300
   Asp Asn Asp Ile Thr Ile Thr Asn Asn Asp Ile Asn Cys Tyr Thr Arg
   305 310 315 320
   Gln Glu Ser Asp Gly Arg Tyr Met Pro Ala Gly Thr Arg Val Gly Asn
   325 330 335
   Val Thr Trp Val Glu Gly Gln Ser Trp Ile Ser Arg Gly Ala Thr Phe
   340 345 350
   Thr Cys Asn Ala Pro Trp Glu Ala Ser Ser Arg Leu Ala Leu Asn Val
   355 360 365
   Asn Val Lys Phe Glu Arg Asn Asn Asp Gly Tyr Asp Asn Arg Ile Phe
   370 375 380
   Arg Phe Val Val Ile Val Asn Gly Ser Gln Trp Gly Gly Glu Leu Thr
   385 390 395 400
   Leu Asn Ile Glu Asn Thr Lys Gly Gly Arg Asn Gly His Ser Trp Arg
   405 410 415
   Phe Glu Ala Tyr Ala Ser Ser Asn Phe Phe Phe Asn Asn Ile Pro Pro
   420 425 430
   Asn Ala Thr Val Gln Ile Arg Pro Thr Glu Asp Ser Arg Ile Ile Phe
   435 440 445
   Tyr Asp Cys Met Leu Thr Phe Cys Thr Asn Arg Pro
   450 455 460
<210> 9
   <211> 1383
   DNA
   <213> Artificial Sequence
<220>
   <223> N-Strep Miro2p12
<400> 9
   atggctagct ggagccaccc gcagttcgaa aaaggcgccg cccagaataa ctataatcac     60
   tacagtgatt tggccaaata tacgatattt gacccaacaa atacacaatg gcctgttgct    120
   ataaaagacg tccaatcagc gttagagctg attggtagct gggcaagaac tgataccgga    180
   ttacctgtag catctcctac agtagccggt gtaattcgca cagcaacaca ggctgaagtt    240
   gatgctggaa ctattggtaa tgctgcggta actcctgcta cattaaaatc cacagttacc    300
   cgtcctgaag caactacagc agttcttggc ttaacacggt atgctactaa tactgaagcc    360
   gcagcattaa ccgcaggaaa tagaactatt accgcggcgg ctctcggtca tgtgtttaaa    420
   actgtgaaag cccaagaaaa cgtagatgga actgttaggt taactactgc ggctcaagca    480
   caagcaggaa ctgacgaaac taccgcagta actcctaagc gtgttgtaga aatgattgga    540
   aagttcagtg tcagtcctcc tagttatacc tctgcgacag aaagcaactt gggattagtt    600
   cgtgtcgcaa cccaagccca ggtagcagca ggtgctgttc acgatggata cgcagtaact    660
   ccaaaaacct tcatggcatc aaaagcgtct gacagcgtat ttggtatagt aaaatttgct    720
   aaagactcag atgtggcttc agctacttct aacaatttgg ctgttactcc aaaaagtctt    780
   caagcgctaa aatccaccaa ggataaatat ggattaacca gattatcagg ttctccaact    840
   actgatgctt cattggcagc ggctgcaaca gatgcagtct ttaaaacccg tagaataaac    900
   ggaaaaactc ttgataatga cataacaatt actaataatg atattaattg ttatacaaga    960
   caagaatctg acgggcgtta catgccagct ggaaccagag taggtaatgt tacttgggtt   1020
   gaaggacaat cttggattag tcgaggtgca acgtttacat gtaatgcacc atgggaagct   1080
   tctagtagat tagctctaaa cgttaatgta aaatttgagc gtaacaacga cggatatgac   1140
   aatcgtattt tcagatttgt tgtaatagtt aacggttccc aatggggagg tgaacttact   1200
   cttaacatcg aaaatactaa aggcggacga aatggtcatt catggagatt tgaagcttac   1260
   gcatctagca actttttctt caataacatt cctccaaatg ccactgttca aataagacca   1320
   acagaagaca gtcgtattat attttatgac tgcatgctta cattctgtac aaatagaccg   1380
   taa                                                                 1383
<210> 10
   <211> 460
   <212> PRT
   <213> Artificial Sequence
<220>
   <223> N-Strep Miro2p12
<400> 10
   Met Ala Ser Trp Ser His Pro Gln Phe Glu Lys Gly Ala Ala Gln Asn
   1 5 10 15
   Asn Tyr Asn His Tyr Ser Asp Leu Ala Lys Tyr Thr Ile Phe Asp Pro
   20 25 30
   Thr Asn Thr Gln Trp Pro Val Ala Ile Lys Asp Val Gln Ser Ala Leu
           35                  40                  45
   Glu Leu Ile Gly Ser Trp Ala Arg Thr Asp Thr Gly Leu Pro Val Ala
       50                  55                  60
   Ser Pro Thr Val Ala Gly Val Ile Arg Thr Ala Thr Gln Ala Glu Val
   65                  70                  75                  80
   Asp Ala Gly Thr Ile Gly Asn Ala Ala Val Thr Pro Ala Thr Leu Lys
                   85                  90                  95
   Ser Thr Val Thr Arg Pro Glu Ala Thr Thr Ala Val Leu Gly Leu Thr
               100                 105                 110
   Arg Tyr Ala Thr Asn Thr Glu Ala Ala Ala Leu Thr Ala Gly Asn Arg
           115                 120                 125
   Thr Ile Thr Ala Ala Ala Leu Gly His Val Phe Lys Thr Val Lys Ala
       130                 135                 140
   Gln Glu Asn Val Asp Gly Thr Val Arg Leu Thr Thr Ala Ala Gln Ala
   145                 150                 155                 160
   Gln Ala Gly Thr Asp Glu Thr Thr Ala Val Thr Pro Lys Arg Val Val
                   165                 170                 175
   Glu Met Ile Gly Lys Phe Ser Val Ser Pro Pro Ser Tyr Thr Ser Ala
               180                 185                 190
   Thr Glu Ser Asn Leu Gly Leu Val Arg Val Ala Thr Gln Ala Gln Val
           195                 200                 205
   Ala Ala Gly Ala Val His Asp Gly Tyr Ala Val Thr Pro Lys Thr Phe
       210                 215                 220
   Met Ala Ser Lys Ala Ser Asp Ser Val Phe Gly Ile Val Lys Phe Ala
   225                 230                 235                 240
   Lys Asp Ser Asp Val Ala Ser Ala Thr Ser Asn Asn Leu Ala Val Thr
                   245                 250                 255
   Pro Lys Ser Leu Gln Ala Leu Lys Ser Thr Lys Asp Lys Tyr Gly Leu
               260                 265                 270
   Thr Arg Leu Ser Gly Ser Pro Thr Thr Asp Ala Ser Leu Ala Ala Ala
           275                 280                 285
   Ala Thr Asp Ala Val Phe Lys Thr Arg Arg Ile Asn Gly Lys Thr Leu
       290                 295                 300
   Asp Asn Asp Ile Thr Ile Thr Asn Asn Asp Ile Asn Cys Tyr Thr Arg
   305                 310                 315                 320
   Gln Glu Ser Asp Gly Arg Tyr Met Pro Ala Gly Thr Arg Val Gly Asn
                   325                 330                 335
   Val Thr Trp Val Glu Gly Gln Ser Trp Ile Ser Arg Gly Ala Thr Phe
               340                 345                 350
   Thr Cys Asn Ala Pro Trp Glu Ala Ser Ser Arg Leu Ala Leu Asn Val
           355                 360                 365
   Asn Val Lys Phe Glu Arg Asn Asn Asp Gly Tyr Asp Asn Arg Ile Phe
       370                 375                 380
   Arg Phe Val Val Ile Val Asn Gly Ser Gln Trp Gly Gly Glu Leu Thr
   385                 390                 395                 400
   Leu Asn Ile Glu Asn Thr Lys Gly Gly Arg Asn Gly His Ser Trp Arg
                   405                 410                 415
   Phe Glu Ala Tyr Ala Ser Ser Asn Phe Phe Phe Asn Asn Ile Pro Pro
               420                 425                 430
   Asn Ala Thr Val Gln Ile Arg Pro Thr Glu Asp Ser Arg Ile Ile Phe
           435                 440                 445
   Tyr Asp Cys Met Leu Thr Phe Cys Thr Asn Arg Pro
       450                 455                 460
<210> 11
   <211> 1428
   <212> DNA
   <213> Artificial Sequence
<220>
   <223> N-Strep Effe04p12
<400> 11
   atggctagcg ctagttggag ccacccgcag ttcgaaaaag gcgccagtaa caatacaatc     60
   aaccacgtaa gtgataaatc catttacgtg acatttgacc caacaggtac tgattggcct    120
   gataccataa ccaacgtaca agatgcgttg gaaaaaatag gtagttgggc gcgtactgat    180
   actgggcttc ctatcgcaac aacttctgtt cgtggtattg ctcagatcgc aaccgaagct    240
   gatattaacg ctggcacgga taacactaag attgttactc cgaaactgtt agcataccgt    300
   atgcagaacc ctaaagcatc acaaaccgta tggggttata cgaagtattc gactgatgcg    360
   gaatctacaa ccgtaactaa cgatgcatcg tctattactc cgcgatcgct gaactatgtg    420
   ttcaataacc gcaaaggtac agaatcggtt tggggttctt ctaaaatcgc taccactgca    480
   caggcggttg ctggtacaga taacactgta actatgactc cgcttaaagt caagcaagcg    540
   atcgcgtctc tggttcctgt tcagtcaagt gcgaccgaaa gctcgcaagg tctggtacaa    600
   ctggcaacag ttgcacaggt tcaggctggt acgatccgtg aagggtatgc aatttcacct    660
   tatacgttta ttcgtttaac tgcaactgaa agcaacctgg gcgttattcg tatcgcatca    720
   cagacagaag caaacgcagg tactgatgac accaaagcga ttactgcgaa aaaattaatc    780
   aatacccgtg caactggttc ccagttcggt gttgtcaaat tagcaacaac tgttggttat    840
   gtggcaaaca ccgcactttc ttctaatgct tatgtattgc ctagcgatcg tagtgcggta    900
   attaatggtt ctctttatga gtatagcgca atacataaca acaaatatca gacgtggaca    960
   gatcttgatt ggcatttccc agtaggtgct attgtcatga ctggtttcca gactgaccac   1020
   ggtagtttgt atatttgcga tggacgttca ctgaataaaa ataattaccc gttactgttt   1080
   gagcgtatag gttatacatt tggtggtggc ggtgattggt ttaacattcc agactgtcga   1140
   ggcgttgcag tacgtggtca tgaccgtggg cgtggactaa accctaatcg tgggtatggt   1200
   acatatgaag gagatatgtt gggatggcac gaacacccat tacaacttat ctaccagaac   1260
   ggcggtaaca ttccgaaatg gcaagcagtt tacgaactga aaagcgccga gaagaatgac   1320
   caaagcgctc gcgtatttga tgcttctata actaaagcta ctggtgtggg cggtgaagaa   1380
   acccgcatga aaaatatcgc attaaactac gtaattcgcg tattataa                1428
<210> 12
   <211> 475
   <212> PRT
   <213> Artificial Sequence
<220>
   <223> N-Strep Effe04p12
<400> 12
   Met Ala Ser Ala Ser Trp Ser His Pro Gln Phe Glu Lys Gly Ala Ser
   1               5                   10                  15
   Asn Asn Thr Ile Asn His Val Ser Asp Lys Ser Ile Tyr Val Thr Phe
               20                  25                  30
   Asp Pro Thr Gly Thr Asp Trp Pro Asp Thr Ile Thr Asn Val Gln Asp
           35                  40                  45
   Ala Leu Glu Lys Ile Gly Ser Trp Ala Arg Thr Asp Thr Gly Leu Pro
       50                  55                  60
   Ile Ala Thr Thr Ser Val Arg Gly Ile Ala Gln Ile Ala Thr Glu Ala
   65                  70                  75                  80
   Asp Ile Asn Ala Gly Thr Asp Asn Thr Lys Ile Val Thr Pro Lys Leu
                   85                  90                  95
   Leu Ala Tyr Arg Met Gln Asn Pro Lys Ala Ser Gln Thr Val Trp Gly
               100                 105                 110
   Tyr Thr Lys Tyr Ser Thr Asp Ala Glu Ser Thr Thr Val Thr Asn Asp
           115                 120                 125
   Ala Ser Ser Ile Thr Pro Arg Ser Leu Asn Tyr Val Phe Asn Asn Arg
       130                 135                 140
   Lys Gly Thr Glu Ser Val Trp Gly Ser Ser Lys Ile Ala Thr Thr Ala
   145                 150                 155                 160
   Gln Ala Val Ala Gly Thr Asp Asn Thr Val Thr Met Thr Pro Leu Lys
                   165                 170                 175
   Val Lys Gln Ala Ile Ala Ser Leu Val Pro Val Gln Ser Ser Ala Thr
               180                 185                 190
   Glu Ser Ser Gln Gly Leu Val Gln Leu Ala Thr Val Ala Gln Val Gln
           195                 200                 205
   Ala Gly Thr Ile Arg Glu Gly Tyr Ala Ile Ser Pro Tyr Thr Phe Ile
       210                 215                 220
   Arg Leu Thr Ala Thr Glu Ser Asn Leu Gly Val Ile Arg Ile Ala Ser
   225                 230                 235                 240
   Gln Thr Glu Ala Asn Ala Gly Thr Asp Asp Thr Lys Ala Ile Thr Ala
                   245                 250                 255
   Lys Lys Leu Ile Asn Thr Arg Ala Thr Gly Ser Gln Phe Gly Val Val
               260                 265                 270
   Lys Leu Ala Thr Thr Val Gly Tyr Val Ala Asn Thr Ala Leu Ser Ser
           275                 280                 285
   Asn Ala Tyr Val Leu Pro Ser Asp Arg Ser Ala Val Ile Asn Gly Ser
       290                 295                 300
   Leu Tyr Glu Tyr Ser Ala Ile His Asn Asn Lys Tyr Gln Thr Trp Thr
   305                 310                 315                 320
   Asp Leu Asp Trp His Phe Pro Val Gly Ala Ile Val Met Thr Gly Phe
   325 330 335
   Gln Thr Asp His Gly Ser Leu Tyr Ile Cys Asp Gly Arg Ser Leu Asn
   340 345 350
   Lys Asn Asn Tyr Pro Leu Leu Phe Glu Arg Ile Gly Tyr Thr Phe Gly
   355 360 365
   Gly Gly Gly Asp Trp Phe Asn Ile Pro Asp Cys Arg Gly Val Ala Val
   370 375 380
   Arg Gly His Asp Arg Gly Arg Gly Leu Asn Pro Asn Arg Gly Tyr Gly
   385 390 395 400
   Thr Tyr Glu Gly Asp Met Leu Gly Trp His Glu His Pro Leu Gln Leu
   405 410 415
   Ile Tyr Gln Asn Gly Gly Asn Ile Pro Lys Trp Gln Ala Val Tyr Glu
   420 425 430
   Leu Lys Ser Ala Glu Lys Asn Asp Gln Ser Ala Arg Val Phe Asp Ala
   435 440 445
   Ser Ile Thr Lys Ala Thr Gly Val Gly Gly Glu Glu Thr Arg Met Lys
   450 455 460
   Asn Ile Ala Leu Asn Tyr Val Ile Arg Val Leu
   465 470 475
<210> 13
   <211> 1347
   <212> DNA
   <213> Artificial Sequence
<220>
   <223> N-Strep AehIp12
<400> 13
   atggctagct ggagccaccc gcagttcgaa aaaggcgcca gaacaaataa tatcacacag      60
   catattagct ctaaagcggg gtcggttgta ttcgaccccg catctgctcc tgcttttgat     120
   acgactatca cagatctaca aaagctggga aataaaatcg atgctcatgc aactaaacca     180
   ctaccggtcg catctgaaac tgtatcgggt attgctgaat tggctaccgt tgatgaagtt     240
   ctattgggaa atgataaagt tagattagtc actccttata cacttcaaca aaaatgggcg     300
   cgtcctaatg cgagcgacac cgtttatggt ctagttagat ataatacagt agcagaacga     360
   gaagaagcgg cagcgaaagt tgacgttact gtgaataccg catctttgtg ggacgttgtt     420
   cgtaataaat cgattgcaac tgagtccaag cgcggatcag tgagtatttc aactctggta     480
   gctgccaaag ctggcgtaga tgatacaaca gcaatgacac cagcgaaagt taaagcggca     540
   atcgacacgt tcgcggtaac ttctgtatct ggtgcaactg aaaccgttac gggcacagtt     600
   aagaatagtc cggcattaat cactaacgcc gcgcttcata ccggttatgc agttacacct     660
   aaaggtttca ttgaaaccag agccgcacag gctcgcgttg gaacagttcg tatggcaact     720
   caagcagaag ctaacgctag aactctggga gacgtggcaa tcagtccagc gacgcttcca     780
   atcgcatctg atacacaata tggcatcact gctcttttgc ataacgcgca atccggcgta     840
   acaaacaaag cattgagcgc acacggagcg actctgttta tcaacagaaa cggcgactcc     900
   atgacgggtg atcttactgt tcataatatc tttactgcta acgggcaaaa cggacgtggc     960
   gattcgctga ctagaaagga ttacgttgac ggacttttca atcagaaagc gaatatttcg    1020
   catacgcacg gaactccaca agaatcatgg acactaattt ggcagggacc gttagatcgc    1080
   ggtaatttcg tgactaatca gccatggtgg aacttcgatg cactcgttat tgaaagttcg    1140
   cgtgatggtg gtagctggtt taataccatg gagattagtc gttggcagat cgaacagatg    1200
   caagcaaaat atccaaactt caacttggta tcggcacaag aatattattg gtttggtaag    1260
   tttagagccg atggtatgta tttcgacacc catacagaga actgctatct gtggagaata    1320
   tacggagtta acaaaacttg gagctaa                                        1347
<210> 14
   <211> 448
   <212> PRT
   <213> Artificial Sequence
<220>
   <223> N-Strep AehIp12
<400> 14
   Met Ala Ser Trp Ser His Pro Gln Phe Glu Lys Gly Ala Arg Thr Asn
   1               5                   10                  15
   Asn Ile Thr Gln His Ile Ser Ser Lys Ala Gly Ser Val Val Phe Asp
               20                  25                  30
   Pro Ala Ser Ala Pro Ala Phe Asp Thr Thr Ile Thr Asp Leu Gln Lys
           35                  40                  45
   Leu Gly Asn Lys Ile Asp Ala His Ala Thr Lys Pro Leu Pro Val Ala
       50                  55                  60
   Ser Glu Thr Val Ser Gly Ile Ala Glu Leu Ala Thr Val Asp Glu Val
   65                  70                  75                  80
   Leu Leu Gly Asn Asp Lys Val Arg Leu Val Thr Pro Tyr Thr Leu Gln
                   85                  90                  95
   Gln Lys Trp Ala Arg Pro Asn Ala Ser Asp Thr Val Tyr Gly Leu Val
               100                 105                 110
   Arg Tyr Asn Thr Val Ala Glu Arg Glu Glu Ala Ala Ala Lys Val Asp
           115                 120                 125
   Val Thr Val Asn Thr Ala Ser Leu Trp Asp Val Val Arg Asn Lys Ser
       130                 135                 140
   Ile Ala Thr Glu Ser Lys Arg Gly Ser Val Ser Ile Ser Thr Leu Val
   145                 150                 155                 160
   Ala Ala Lys Ala Gly Val Asp Asp Thr Thr Ala Met Thr Pro Ala Lys
                   165                 170                 175
   Val Lys Ala Ala Ile Asp Thr Phe Ala Val Thr Ser Val Ser Gly Ala
               180                 185                 190
   Thr Glu Thr Val Thr Gly Thr Val Lys Asn Ser Pro Ala Leu Ile Thr
           195                 200                 205
   Asn Ala Ala Leu His Thr Gly Tyr Ala Val Thr Pro Lys Gly Phe Ile
       210                 215                 220
   Glu Thr Arg Ala Ala Gln Ala Arg Val Gly Thr Val Arg Met Ala Thr
   225                 230                 235                 240
   Gln Ala Glu Ala Asn Ala Arg Thr Leu Gly Asp Val Ala Ile Ser Pro
                   245                 250                 255
   Ala Thr Leu Pro Ile Ala Ser Asp Thr Gln Tyr Gly Ile Thr Ala Leu
               260                 265                 270
   Leu His Asn Ala Gln Ser Gly Val Thr Asn Lys Ala Leu Ser Ala His
           275                 280                 285
   Gly Ala Thr Leu Phe Ile Asn Arg Asn Gly Asp Ser Met Thr Gly Asp
       290                 295                 300
   Leu Thr Val His Asn Ile Phe Thr Ala Asn Gly Gln Asn Gly Arg Gly
   305                 310                 315                 320
   Asp Ser Leu Thr Arg Lys Asp Tyr Val Asp Gly Leu Phe Asn Gln Lys
                   325                 330                 335
   Ala Asn Ile Ser His Thr His Gly Thr Pro Gln Glu Ser Trp Thr Leu
               340                 345                 350
   Ile Trp Gln Gly Pro Leu Asp Arg Gly Asn Phe Val Thr Asn Gln Pro
           355                 360                 365
   Trp Trp Asn Phe Asp Ala Leu Val Ile Glu Ser Ser Arg Asp Gly Gly
       370                 375                 380
   Ser Trp Phe Asn Thr Met Glu Ile Ser Arg Trp Gln Ile Glu Gln Met
   385                 390                 395                 400
   Gln Ala Lys Tyr Pro Asn Phe Asn Leu Val Ser Ala Gln Glu Tyr Tyr
                   405                 410                 415
   Trp Phe Gly Lys Phe Arg Ala Asp Gly Met Tyr Phe Asp Thr His Thr
               420                 425                 430
   Glu Asn Cys Tyr Leu Trp Arg Ile Tyr Gly Val Asn Lys Thr Trp Ser
           435                 440                 445
<210> 15
   <211> 75
   <212> DNA
   <213> Artificial Sequence
<220>
   <223> Primer
<400> 15
   gaaggaacta gtcatatggc tagctggagc cacccgcagt tcgaaaaagg cgccgcccag      60
   aataactata atcac                                                       75
<210> 16
   <211> 29
   <212> DNA
   <213> Artificial Sequence
<220>
   <223> Primer
<400> 16
   cgggatcctc cttacggtct atttgtaca 29
<210> 17
   <211> 76
   <212> DNA
   <213> Artificial Sequence
<220>
   <223> Primer
<400> 17
   gaaggaacta gtgctagcgc tagctggagc cacccgcagt tcgaaaaagg cgccagtaac 60
   aatacaatca accacg 76
<210> 18
   <211> 26
   <212> DNA
   <213> Artificial Sequence
<220>
   <223> Primer
<400> 18
   cgggatcccc tctgttataa tacgcg 26
<210> 19
   <211> 75
   <212> DNA
   <213> Artificial Sequence
<220>
   <223> Primer
<400> 19
   gaaggaacta gtcatatggc tagctggagc cacccgcagt tcgaaaaagg cgccagaaca 60
   aataatatca cacag 75
<210> 20
   <211> 39
   <212> DNA
   <213> Artificial Sequence
<220>
   <223> Primer
<400> 20
   gaaggaacta gtcatatgag aacaaataat atcacacag 39